(19) Europäisches Patentamt
European Patent Office
Office européen des brevets

(11) EP 0 612 741 B1

(12) **EUROPÄISCHE PATENTSCHRIFT**

(45) Veröffentlichungstag und Bekanntmachung des Hinweises auf die Patenterteilung:
**10.06.1998 Patentblatt 1998/24**

(21) Anmeldenummer: **94102557.9**

(22) Anmeldetag: **21.02.1994**

(51) Int Cl.[6]: **C07D 401/04**, C07D 403/04, C07D 417/04, C07D 471/04, C07D 487/04, A61K 31/41, A61K 31/47, A61K 31/55

(54) **Cyclische Derivate, diese Verbindungen enthaltende Arzneimittel und Verfahren zu ihrer Herstellung**

Cyclic derivatives, medicaments containing them and process for their preparation

Dérivés cycliques, médicaments les contenant et procédé pour leur préparation

(84) Benannte Vertragsstaaten:
**AT BE CH DE DK ES FR GB GR IE IT LI LU NL PT SE**

(30) Priorität: **22.02.1993 DE 4305388**
**22.09.1993 DE 4332168**

(43) Veröffentlichungstag der Anmeldung:
**31.08.1994 Patentblatt 1994/35**

(73) Patentinhaber: **Dr. Karl Thomae GmbH**
**D-88397 Biberach (DE)**

(72) Erfinder:
- **Himmelsbach, Frank, Dr. Dipl.-Chem.**
  **D-88441 Mittelbiberach (DE)**
- **Pieper, Helmut, Dr. Dipl.-Chem.**
  **D-88400 Biberach (DE)**
- **Austel, Volkhard, Prof. Dr. Dipl.-Chem.**
  **D-88440 Biberach (DE)**
- **Linz, Günter, Dr. Dipl.-Chem.**
  **D-88441 Mittelbiberach (DE)**
- **Guth, Brian, Dr.**
  **D-88447 Warthausen (DE)**
- **Müller, Thomas, Dr. Arzt und Dipl.-Chem.**
  **D-88400 Biberach (DE)**
- **Weisenberger, Johannes, Dr. Dipl.-Chem.**
  **D-88400 Biberach (DE)**

(74) Vertreter: **Laudien, Dieter, Dr.**
**c/o Boehringer Ingelheim GmbH**
**55216 Ingelheim (DE)**

(56) Entgegenhaltungen:
**EP-A- 0 503 548** **EP-A- 0 528 369**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 612 741 B1

Printed by Jouve, 75001 PARIS (FR)

## Beschreibung

In der EP-A-0,503,548 werden bereits Fibinogen-Rezeptor-Antagonisten beschrieben, die u.a. einen monocyclischen Alkyleniminoring enthalten können.

Es wurde nun gefunden, daß die cyclischen Derivate der allgemeinen Formel

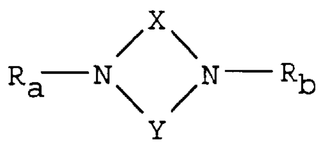

, (I)

welche einen bicyclischen Alkyleniminoring enthalten, wertvolle pharmakologische Eigenschaften aufweisen, vorzugsweise aggregationshemende Wirkungen.

Gegenstand der vorliegenden Anmeldung sind somit die neuen Verbindungen der obigen allgemeinen Formel I, deren Tautomere, deren Stereoisomere einschließlich deren Gemische und deren Salze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, diese Verbindungen enthaltende Arzneimittel, deren Verwendung und Verfahren zu ihrer Herstellung.

In der obigen allgemeinen Formel I bedeutet

X eine am Stickstoffatom durch eine Cyanogruppe substituierte Carbiminogruppe, eine Carbonyl- oder Sulfonylgruppe,

Y eine gegebenenfalls durch $R_c$ oder $R_c$ und $R_d$ substituierte $-CH_2CH_2-$, $-CH_2CH_2CH_2-$, -CH=CH-, $-CH_2CO-$ oder $-COCH_2$-Gruppe,

eine gegebenenfalls durch $R_c$ oder $R_d$ substituierte -CO-NH-, -NH-CO-, -CH=N- oder -N=CH-Gruppe,

der erste der Reste $R_a$ bis $R_d$ eine A-B-Gruppe, in der

A eine Gruppe der Formeln

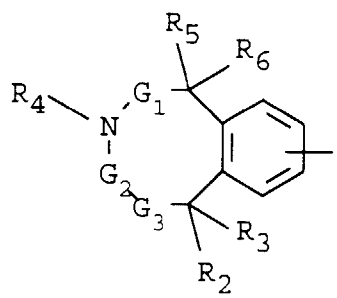

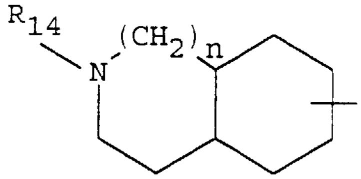

oder

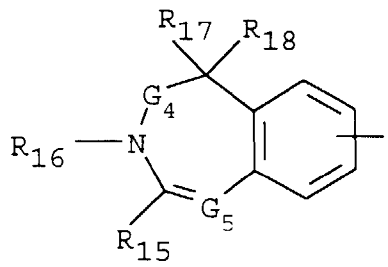

darstellt, wobei
jeweils der Benzoteil der vorstehend erwähnten Gruppen durch ein Fluor-, Chlor- oder Bromatom, durch eine Alkyl-, Cyano-, Trifluormethyl-, Hydroxy- oder Alkoxygruppe substituiert sein kann oder eine bis drei Methingruppen jeweils durch ein Stickstoffatom ersetzt sein können,

$G_1$ eine Bindung oder eine Methylengruppe, die durch eine Alkylgruppe mono- oder disubstituiert sein kann, wobei die Substituenten gleich oder verschieden sein können,

$G_2$ eine Bindung oder eine durch $R_7$ und $R_8$ substituierte Methylengruppe,

$G_3$ eine durch $R_9$ und $R_{10}$ substituierte Methylengruppe,

$G_4$ eine Bindung,

$G_5$ ein Stickstoffatom oder eine gegebenenfalls durch eine Alkylgruppe substituierte Methingruppe,

$R_2$ ein Wasserstoffatom oder eine Alkylgruppe,

$R_3$ ein Wasserstoffatom oder eine Alkylgruppe,

$R_4$ ein Wasserstoffatom, eine Cycloalkyl- oder Cycloalkylalkylgruppe mit jeweils 3 bis 7 Kohlenstoffatomen im Cycloalkylteil, eine Alkylgruppe mit 1 bis 6 Kohlenstoffatomen, eine Alkenylgruppe mit 3 bis 6 Kohlenstoffatomen, wobei die Alkenylgruppe nicht über den Vinylteil mit dem Stickstoffatom verbunden sein kann, eine Hydroxyalkyl-, Alkoxyalkyl-, Carboxyalkyl-, Alkoxycarbonylalkyl-, Aminocarbonylalkyl-, N-Alkylaminocarbonylalkyl-, N,N-Dialkylaminocarbonylalkyl-, Arylalkyl-, Alkoxycarbonyl-, Arylmethyloxycarbonyl-, Formyl-, Acetyl-, Trifluoracetyl-oder $R_{11}CO-O-(R_{12}CR_{13})$ -O-CO-Gruppe, in welcher

$R_{11}$ eine Alkylgruppe,

$R_{12}$ ein Wasserstoffatom oder eine Alkylgruppe und

$R_{13}$ ein Wasserstoffatom darstellen,

oder $R_4$ zusammen mit $R_3$ eine geradkettige Alkylengruppe mit 2 oder 3 Kohlenstoffatomen,

$R_5$ ein Wasserstoffatom oder eine Alkylgruppe oder auch, falls $G_1$ eine Bindung darstellt, $R_4$ zusammen mit $R_5$ eine weitere Bindung,

$R_6$ ein Wasserstoffatom oder eine Alkylgruppe oder, falls $G_1$ eine Bindung und $R_4$ zusammen mit $R_5$ eine weitere Bindung darstellen, eine Aminogruppe,

$R_7$ ein Wasserstoffatom oder eine Alkylgruppe,

$R_8$ ein Wasserstoffatom oder eine Alkylgruppe oder $R_8$ zusammen mit $R_4$ eine geradkettige Alkylengruppe mit 3 oder 4 Kohlenstoffatomen,

$R_9$ ein Wasserstoffatom oder eine Alkylgruppe,

$R_{10}$ ein Wasserstoffatom oder eine Alkylgruppe oder $R_{10}$ zusammen mit $R_4$ eine geradkettige Alkylengruppe mit 3 oder 4 Kohlenstoffatomen,

$R_{14}$ ein Wasserstoffatom oder eine Alkylgruppe,

$R_{15}$ ein Wasserstoffatom oder eine Alkylgruppe,

$R_{16}$ ein Wasserstoffatom oder eine Alkylgruppe,

$R_{17}$ ein Wasserstoffatom oder eine Alkylgruppe oder $R_{16}$ zusammen mit $R_{17}$ eine weitere Bindung,

$R_{18}$ ein Wasserstoffatom oder eine Alkylgruppe oder auch, wenn $R_{16}$ und $R_{17}$ zusammen eine weitere Bindung darstellen, ein Chloratom oder eine Aminogruppe,

n die Zahl 1 oder 2 darstellen, und

B eine Bindung,

eine Alkylengruppe,

eine Arylengruppe,

eine gegebenenfalls durch eine oder zwei Alkylgruppen substituierte Pyridinylen-, Pyrimidinylen-, Pyrazinylen- oder Pyridazinylengruppe,

eine gegebenenfalls durch eine oder zwei Alkylgruppen substituierte Cyclohexylengruppe,

eine gegebenenfalls durch eine oder zwei Alkylgruppen substituierte Piperidinylengruppe, in der eine zu einem Stickstoffatom benachbarte Methylengruppe durch eine Carbonylgruppe ersetzt sein kann,

der zweite der Reste $R_a$ bis $R_d$ eine Gruppe der Formel

F - E - D -,

in der

D eine Alkylengruppe,

eine Arylengruppe,

eine gegebenenfalls durch eine oder zwei Alkylgruppen substituierte Pyridinylen-, Pyrimidinylen-, Pyrazinylen- oder Pyridazinylengruppe,

eine Indanylen-, Naphthylen-, 1,2,3,4-Tetrahydronaphthylenoder Benzosuberanylengruppe, in denen jeweils einer der Ringe an den Rest E und der andere der Ringe an den cyclischen Rest der allgemeinen Formel I gebunden ist,

eine gegebenenfalls durch eine oder zwei Alkylgruppen substituierte Cycloalkylengruppe mit 4 bis 7 Kohlenstoffatomen,

eine gegebenenfalls durch eine oder zwei Alkylgruppen substituierte Cycloalkylengruppe mit 5 bis 7 Kohlenstoffatomen, in der eine >CH-Einheit durch ein Stickstoffatom ersetzt ist, wobei außerdem in den vorstehend erwähnten 5- bis 7-gliedrigen Ringen jeweils eine zu einem Stickstoffatom benachbarte Methylengruppe durch eine Carbonylgruppe ersetzt sein kann,

oder, wenn E eine cyclische Iminogruppe darstellt, auch eine Alkylencarbonylgruppe, wobei die Carbonylgruppe jeweils an das Stickstoffatom der cyclischen Iminogruppe der Gruppe E gebunden ist,

oder auch, falls E keine Bindung darstellt, eine Bindung,

E eine Bindung,

eine Alkylengruppe, die durch eine Alkylgruppe mit 1 bis 6 Kohlenstoffatomen, durch eine Amino-, Aryl-, Alkylamino-, Dialkylamino-, $HNR_{21}$- oder N-Alkyl-$NR_{21}$-Gruppe substituiert sein kann, wobei

$R_{21}$ eine Alkylcarbonyl- oder Alkylsulfonylgruppe mit jeweils 1 bis 6 Kohlenstoffatomen im Alkylteil, eine Alkyloxycarbonylgruppe mit insgesamt 2 bis 5 Kohlenstoffatomen, eine Cycloalkylcarbonyl- oder Cycloalkylsulfonylgruppe mit jeweils 5 bis 7 Kohlenstoffatomen im Cycloalkylteil, eine Arylalkylcarbonyl-, Arylalkylsulfonyl-, Arylalkoxycarbonyl-, Arylcarbonyl- oder Arylsulfonylgruppe darstellt,

eine Alkenylengruppe mit 2 bis 4 Kohlenstoffatomen,

eine Arylengruppe,

eine gegebenenfalls durch eine oder zwei Alkylgruppen substituierte Pyridinylen-, Pyrimidinylen-, Pyrazinylen- oder Pyridazinylengruppe,

eine gegebenenfalls durch eine oder zwei Alkylgruppen substituierte Cycloalkylengruppe mit 5 bis 7 Kohlenstoffatomen, in der eine >CH-Einheit durch ein Stickstoffatom, welches mit einem Kohlenstoffatom des Restes D verknüpft ist, ersetzt ist,

eine gegebenenfalls durch eine oder zwei Alkylgruppen substituierte Cycloalkylengruppe mit 4 bis 7 Kohlenstoffatomen im Cycloalkylenteil,

oder auch, falls D keine Bindung darstellt, eine über den Rest W mit dem Rest D verknüpfte Alkylengruppe, in der W ein Sauerstoff- oder Schwefelatom, eine Sulfinyl-, Sulfonyl-, -$NR_{20}$-CO-oder -CO-$NR_{20}$-Gruppe darstellt, wobei $R_{20}$ ein Wasserstoffatom oder eine Alkylgruppe darstellt und die Alkylengruppe zusätzlich durch eine Alkylgruppe mit 1 bis 6 Kohlenstoffatomen, durch eine Amino-, Aryl-, Alkylamino-, Dialkylamino-, -$HNR_{21}$-oder N-Alkyl-$NR_{21}$-Gruppe substituiert sein kann, wobei das Heteroatom des zusätzlichen Substituenten durch mindestens 2 Kohlenstoffatome von einem Heteroatom des Restes W getrennt ist und $R_{21}$ wie vorstehend definiert ist, und

F eine Carbonylgruppe, die durch eine Hydroxy-, Alkoxy-, Arylalkoxy- oder $R_{22}$O-Gruppe substituiert ist, wobei

$R_{22}$ eine Cycloalkylgruppe mit 5 bis 7 Kohlenstoffatomen oder eine Cycloalkylalkylgruppe mit 5 bis 7 Kohlenstoffatomen im Cycloalkylteil darstellt,

eine $R_{23}$CO-O-$CHR_{24}$-O-CO-, Phosphono- oder O-Alkyl-phosphonogruppe darstellen, wobei

$R_{23}$ eine Alkyl-, Alkoxy-, Cycloalkyl- oder Cycloalkoxygruppe mit jeweils 5 bis 7 Kohlenstoffatomen im Cycloalkylteil und

$R_{24}$ ein Wasserstoffatom oder eine Alkylgruppe darstellen,

und der kürzeste Abstand zwischen dem Rest F und dem von dem Rest F am weitesten entfernten Stickstoffatom der Gruppe A-B-mindestens 11 Bindungen beträgt,

der dritte der Reste $R_a$ bis $R_d$ ein Wasserstoffatom, eine Alkoxygruppe, wobei die Alkoxygruppe nicht an ein Stickstoffatom gebunden sein kann, eine Alkyl-, Trifluormethyl- oder Arylgruppe und

der vierte der Reste $R_a$ bis $R_d$ ein Wasserstoffatom oder eine Alkylgruppe,

wobei, soweit nichts anderes erwähnt wurde,

unter den bei der Definition der vorstehenden Reste erwähnten Arylteilen eine Phenylgruppe, die jeweils durch $R_{25}$ monosubstituiert, durch $R_{26}$ mono-, di- oder trisubstituiert oder durch $R_{25}$ monosubstituiert und zusätzlich durch $R_{26}$ mono- oder disubstituiert sein kann, wobei die Substituenten gleich oder verschieden sein können und

$R_{25}$ eine Cyano-, Aminocarbonyl-, Alkylaminocarbonyl-, Dialkylaminocarbonyl-, Alkylcarbonyl-, Alkylsulfenyl-, Alkylsulfinyl-, Alkylsulfonyl- oder Trifluormethylgruppe und

$R_{26}$ eine Alkyl-, Hydroxy- oder Alkoxygruppe, ein Fluor-, Chloroder Bromatom darstellen, wobei zwei Reste $R_{26}$ sofern diese an benachbarte Kohlenstoffatome gebunden sind, auch eine geradkettige Alkylengruppe mit 3 oder 4 Kohlenstoffatomen, eine 1,3-Butadien-1,4-diylengruppe oder eine Methylendioxygruppe darstellen können,

unter den bei der Definition der vorstehenden Reste erwähnten Arylenteilen eine Phenylengruppe die jeweils durch $R_{25}$ monosubstituiert, durch $R_{26}$ mono- oder disubstituiert oder durch $R_{25}$ monosubstituiert und zusätzlich durch $R_{26}$ monosubstituiert sein kann, wobei die Substituenten gleich oder verschieden sein können und wie vorstehend erwähnt definiert sind,

zu verstehen ist, sowie, soweit nichts anderes erwähnt wurde, die vorstehend erwähnten Alkyl-, Alkylen- oder Alkoxyteile jeweils 1 bis 4 Kohlenstoffatome enthalten können, und jedes Kohlenstoffatom in den vorstehend erwähnten Alkylen- und Cycloalkylenteilen höchstens mit einem Heteroatom verknüpft ist.

Bevorzugte Verbindungen der obigen allgemeinen Formel I sind jedoch diejenigen, in denen

X eine Carbonyl- oder Sulfonylgruppe,

Y eine gegebenenfalls durch eine oder zwei Methylgruppen substituierte $-CH_2CH_2-$, $-CH_2CH_2CH_2-$, -CH=CH-, $-CH_2CO-$ oder $-COCH_2-$Gruppe,

eine gegebenenfalls durch $R_c$ substituierte -CO-NH-, -CH=N- oder -N=CH-Gruppe,

der erste der Reste $R_a$ bis $R_c$ eine A-B-Gruppe, in der

A eine Gruppe der Formeln

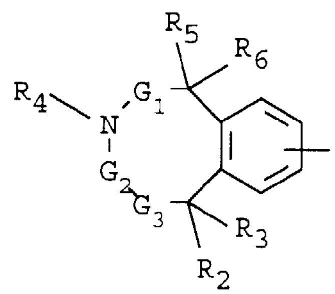

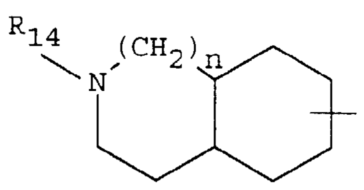

oder

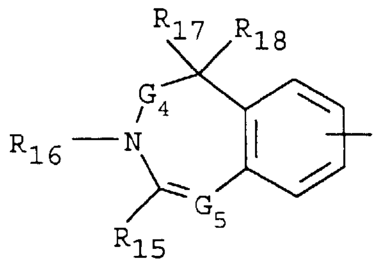

darstellt, wobei jeweils im Benzoteil der vorstehend erwähnten Gruppen eine oder zwei Methingruppen jeweils durch ein Stickstoffatom ersetzt sein können,

$G_1$ eine Bindung oder eine Methylengruppe,

$G_2$ eine Bindung,

$G_3$ eine Methylengruppe,

$G_4$ eine Bindung,

$G_5$ ein Stickstoffatom oder eine Methingruppe,

$R_2$ ein Wasserstoffatom,

$R_3$ ein Wasserstoffatom,

$R_4$ ein Wasserstoffatom, eine Cyclopropyl- oder Cyclopropylmethylgruppe, eine Alkylgruppe mit 1 bis 6 Kohlenstoffatomen, eine Allyl-, Hydroxyalkyl-, Carboxyalkyl-, Alkoxycarbonylalkyloder Benzylgruppe,

$R_5$ ein Wasserstoffatom,

$R_6$ ein Wasserstoffatom,

$R_{14}$ ein Wasserstoffatom oder eine Alkylgruppe,

$R_{15}$ ein Wasserstoffatom oder eine Alkylgruppe,

$R_{16}$ ein Wasserstoffatom oder eine Alkylgruppe,

$R_{17}$ ein Wasserstoffatom oder $R_{16}$ zusammen mit $R_{17}$ eine weitere Bindung,

$R_{18}$ ein Wasserstoffatom oder auch, wenn $R_{16}$ und $R_{17}$ zusammen eine weitere Bindung darstellen, eine Aminogruppe,

n die Zahl 1 oder 2 darstellen, und

B eine Bindung oder eine Phenylengruppe,

der zweite der Reste $R_a$ bis $R_c$ eine Gruppe der Formel

F - E - D -,

in der

D eine Alkylengruppe,

eine Phenylengruppe,

eine Cyclohexylengruppe oder

eine Piperidinylengruppe, wobei das Ringstickstoffatom mit der gegebenenfalls substituierten geradkettigen Alkylengruppe des Restes E verknüpft ist, oder

eine Bindung,

E eine geradkettige Alkylengruppe, die durch eine Alkyl- oder Phenylgruppe substituiert sein kann,

eine Alkenylengruppe mit 2 bis 4 Kohlenstoffatomen,

eine Phenylengruppe,

oder auch, falls D keine Bindung darstellt, eine über das Sauerstoffatom mit dem Rest D verknüpfte geradkettige O-Alkylengruppe und

F eine Carbonylgruppe, die durch eine Hydroxy- oder Alkoxygruppe substituiert ist, darstellen

und der kürzeste Abstand zwischen dem Rest F und dem von dem Rest F am weitesten entfernten Stickstoffatom der Gruppe A-B-mindestens 11 Bindungen beträgt, und

der dritte der Reste $R_a$ bis $R_c$ ein Wasserstoffatom, eine Alkoxygruppe, wobei die Alkoxygruppe nicht an ein Stickstoffatom gebunden sein kann, eine Alkyl- oder Phenylgruppe bedeuten,

wobei, soweit nichts anderes erwähnt wurde, die vorstehend erwähnten Alkyl-, Alkylen- oder Alkoxyteile jeweils 1 bis 4 Kohlenstoffatome enthalten können, und jedes Kohlenstoffatom in den vorstehend erwähnten Alkylen- und Cycloalkylenteilen höchstens mit einem Heteroatom verknüpft ist,

deren Tautomere, deren Stereoisomere und Salze.

Besonders bevorzugte Verbindungen der allgemeinen Formel I sind diejenigen, in denen

X eine Carbonylgruppe,

Y eine $-CH_2CH_2-$, $-CH_2CH_2CH_2-$, -CH=CH-, $-CH_2CO-$ oder $-COCH_2$-Gruppe oder

eine gegebenenfalls durch eine Methylgruppe substituierte -N=CH-Gruppe,

der Rest $R_a$ eine A-B-Gruppe, in der

A eine Gruppe der Formeln

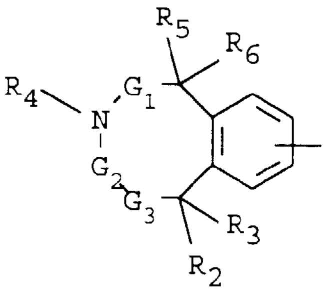

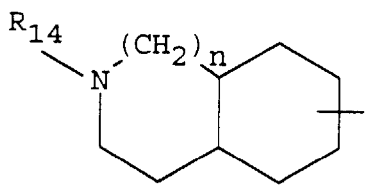

oder

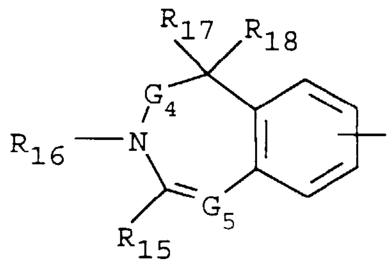

darstellt, wobei jeweils im Benzoteil der vorstehend erwähnten Gruppen eine oder zwei Methingruppen jeweils durch ein Stickstoffatom ersetzt sein können,

$G_1$ eine Bindung oder eine Methylengruppe,

$G_2$ eine Bindung,

$G_3$ eine Methylengruppe,

$G_4$ eine Bindung,

$G_5$ eine Methingruppe,

$R_2$ ein Wasserstoffatom,

$R_3$ ein Wasserstoffatom,

$R_4$ ein Wasserstoffatom, eine Alkylgruppe mit 1 bis 4 Kohlenstoffatomen, eine Allyl- oder Benzylgruppe,

$R_5$ ein Wasserstoffatom,

$R_6$ ein Wasserstoffatom,

$R_{14}$ ein Wasserstoffatom oder eine Methylgruppe,

$R_{15}$ ein Wasserstoffatom,

$R_{16}$ zusammen mit $R_{17}$ eine Bindung,

$R_{18}$ ein Wasserstoffatom oder eine Aminogruppe und

n die Zahl 1 darstellen,

B eine Bindung,

der Rest $R_b$ eine Gruppe der Formel

F - E - D -,

in der

D eine $-CH_2CH_2$-Gruppe,

eine 1,4-Phenylengruppe oder

eine 1,4-Cyclohexylengruppe,

E eine gegebenenfalls durch eine Methylgruppe substituierte $-CH_2CH_2$-Gruppe, eine -CH=CH-, 1,4-Phenylen- oder $-O-CH_2$-Gruppe, wobei das Sauerstoffatom der $-O-CH_2$-Gruppe mit dem Rest D verknüpft ist, und

F eine Carbonylgruppe, die durch eine Hydroxy- oder Alkoxygruppe mit 1 bis 4 Kohlenstoffatomen substituiert ist, bedeuten

und der kürzeste Abstand zwischen dem Rest F und dem von dem Rest F am weitesten entfernten Stickstoffatom der Gruppe A-B-mindestens 11 Bindungen beträgt,

deren Tautomere, deren Stereoisomere und Salze.

Als besonders bevorzugte Verbindungen seien beispielsweise folgende erwähnt:

(a) 1-[4-[2-(Methoxycarbonyl)ethyl]phenyl]-3-(1,2,3,4-tetrahydroisochinolin-6-yl)-imidazolidin-2-on,

(b) 1-[4-(2-Carboxyethyl)phenyl]-3-(1,2,3,4-chinolin-6-yl)-imidazolidin-2-on,

(c) 1-[4-(2-Carboxyethyl)phenyl]-3-(2-methyl-1,2,3,4-tetrahydroisochinolin-6-yl)-imidazolidin-2-on,

(d) 1-[4-[2-(Isobutyloxycarbonyl)ethyl]phenyl]-3-(1,2,3,4-tetrahydroisochinolin-6-yl)-imidazolidin-2-on,

(e) 1-[4-(2-Carboxyethyl)phenyl]-3-(2,3,4,5-tetrahydro-1H-3-benzazepin-7-yl)-imidazolidin-2-on,

(f) 1-[4-[2-(Methoxycarbonyl)ethyl]phenyl]-3-(2,3,4,5-tetrahydro-1H-3-benzazepin-7-yl)-imidazolidin-2-on,

(g) 1-[4-[2-(Isopropyloxycarbonyl)ethyl]phenyl]-3-(2,3,4,5-tetrahydro-1H-3-benzazepin-7-yl)-imidazolidin-2-on,

(h) 1-[4-(2-Carboxyethyl)phenyl]-3-(3-methyl-2,3,4,5-tetrahydro-1H-3-benzazepin-7-yl)-imidazolidin-2-on,

(i) 4-[4-[2-(Carboxyethyl)phenyl]-5-methyl-2-(2,3,4,5-tetrahydro-1H-3-benzazepin-7-yl)-4H-1,2,4-triazol-3-on,

(j) 1-[trans-4-(2-Carboxyethyl)cyclohexyl]-3-(2,3,4,5-tetrahydro-1H-3-benzazepin-7-yl)-imidazolidin-2-on,

(k) 1-[4-[2-(Methoxycarbonyl)ethyl]phenyl]-3-13-methyl-2,3,4,5-tetrahydro-1H-3-benzazepin-7-yl)-imidazolidin-2-on,

(l) 1-[4-[2-(Ethoxycarbonyl)ethyl]phenyl]-3-(3-methyl-2,3,4,5-tetrahydro-1H-3-benzazepin-7-yl)-imidazolidin-2-on,

(m) 1-[4- [2- (Isopropyloxycarbonyl)ethyl]phenyl]-3- (3-methyl-2,3,4,5-tetrahydro-1H-3-benzazepin-7-yl)-imidazolidin-2-on,

(n) 1-[trans-4-(2-Carboxyethyl)cyclohexyl]-3-13-methyl-2,3,4,5-tetrahydro-1H-3-benzazepin-7-yl)-imidazolidin-2-on,

(o) 1-[trans-4-[2-(Isopropyloxycarbonyl)ethyl]cyclohexyl]-3-(3-methyl-2,3,4,5-tetrahydro-1H-3-benzazepin-7-yl)-imidazolidin-2-on,

(p) 1-[trans-4-[2-(Ethoxycarbonyl)ethyl]cyclohexyl]-3-(3-methyl-2,3,4,5-tetrahydro-1H-3-benzazepin-7-yl)-imidazolidin-2-on,

(q) 1-[trans-4-[2-(Methoxycarbonyl)ethyl]cyclohexyl]-3-(3-methyl-2,3,4,5-tetrahydro-1H-3-benzazepin-7-yl)-imidazolidin-2-on,

(r) 1-[trans-4- [(Carboxymethyl)oxy]cyclohexyl]-3-(3-methyl-2,3,4,5-tetrahydro-1H-3-benzazepin-7-yl)-imidazolidin-2-on,

(s) 3-[trans-4-(2-Carboxyethyl)cyclohexyl]-1-(3-methyl-2,3,4,5-tetrahydro-1H-3-benzazepin-7-yl)-hydantoin und

(t) 1-[trans-4-(2-Carboxyethyl)cyclohexyl]-3-(3-ethyl-2,3,4,5-tetrahydro-1H-3-benzazepin-7-yl)-imidazolidin-2-on,

deren Tautomere und deren Salze.

Erfindungsgemäß erhält man die neuen Verbindungen der allgemeinen Formel I beispielsweise nach folgenden Verfahren:

a) Zur Herstellung von Verbindungen der allgemeinen Formel I, in der F eine Carboxylgruppe darstellt: Überführung einer Verbindung der allgemeinen Formel

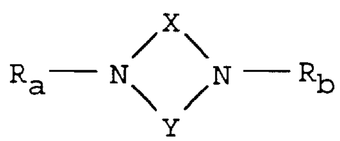

, (I)

in der

$R_a$, $R_b$, X und Y mit der Maßgabe wie eingangs definiert sind, daß einer der Reste $R_a$ bis $R_d$ eine F'-E-D-Gruppe darstellt, in der

E und D wie eingangs definiert sind und

F' eine mittels Hydrolyse, Behandeln mit Säuren, Thermolyse oder Hydrogenolyse in eine Carboxyl gruppe überführbare Gruppe bedeutet,

in eine Verbindung der allgemeinen Formel I, in der F eine Carboxylgruppe darstellt.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe,

Ester mit tertiären Alkoholen, z.B. der tert. Butylester, mittels Behandlung mit einer Säure oder Thermolyse in eine Carboxylgruppe und

Ester mit Aralkanolen, z.B. der Benzylester, mittels Hydrogenolyse in eine Carboxylgruppe übergeführt werden

Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemische oder in Gegenwart einer Base wie Lithiumhydroxid, Natriumhydroxid oder Kaliumhydroxid in einem geeigneten Lösungsmittel wie Wasser, Wasser/Methanol, Wasser/Äthanol, Wasser/Isopropanol, Methanol, Äthanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Unter den vorstehend erwähnten Reaktionsbedingungen können gegebenenfalls vorhandene N-Acylamino- oder N-Acyliminogruppen wie eine N-Trifluoracetyliminogruppe in die entsprechenden Amino- oder Iminogruppen übergeführt werden. Außerdem können gegebenenfalls vorhandene alkoholische Hydroxygruppen bei der Behandlung mit einer organischen Säure wie Trichloressigsäure oder Trifluoressigsäure gleichzeitig in eine entsprechende Acyloxygruppe wie die Trifluoracetoxygruppe übergeführt werden.

Bedeutet F' in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C in die Carboxylgruppe übergeführt werden.

Bedeutet F' in einer Verbindung der Formel II beispielsweise die tert. Butyloxycarbonylgruppe, so kann die tert. Butylgruppe auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden. Bei den vorstehend erwähnten Reaktionsbedigungen können gegebenenfalls vorhandene N-tert.Butyloxycarbonylamino- oder N-tert.-Butyloxycarbonyliminogruppen in die entsprechenden Amino- oder Iminogruppen übergeführt werden.

Bedeutet F' in einer Verbindung der Formel II beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe in eine Aminogruppe, eine Benzyloxygruppe in eine Hydroxygruppe und eine N-Benzylamino-, N-Benzylimino-, N-Benzyloxycarbonylaminooder N-Benzyloxycarbonyliminogruppe in eine entsprechende Amino- oder Iminogruppe übergeführt werden.

b) Zur Herstellung von Verbindungen der allgemeinen Formel I, in der $R_{16}$ und $R_{17}$ zusammen eine weitere Bindung, $G_4$ eine Bindung und $R_{18}$ eine Aminogruppe darstellen:
Umsetzung einer Verbindung der allgemeinen Formel

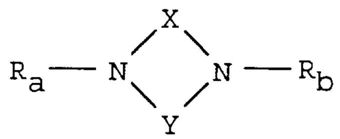

, (III)

in der
$R_a$, $R_b$, X und Y mit der Maßgabe wie eingangs definiert sind, daß einer der Reste $R_a$ bis $R_d$ eine A-B-Gruppe darstellt, in der

B wie eingangs definiert ist und

A eine Gruppe der Formel

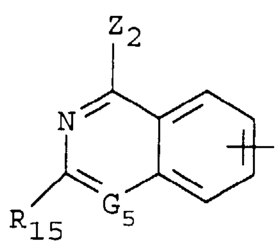

darstellt, wobei der Benzoteil, $R_{15}$ und $G_5$ wie eingangs definiert sind und
$Z_2$ eine nukleophile Austrittsgruppe wie ein Halogenatom, z.B. ein Chlor- oder Bromatom, darstellt,
mit einer Verbindung der allgemeinen Formel

$$H - R_{27} , \qquad (IV)$$

in der

$R_{27}$ eine Aminogruppe darstellt.

Die Umsetzung wird zweckmäßigerweise in einem Lösungsmittel wie Wasser, Aceton, Ethanol, Tetrahydrofuran, Dioxan, Dimethylformamid oder Dimethylsulfoxid, gegebenenfalls jedoch in einem Überschuß der eingesetzten Verbindung der allgemeinen Formel IV als Lösungsmittel und gegebenenfalls in Gegenwart einer Base wie Natriumhydroxid, Kaliumhydroxid, Kaliumcarbonat, Natriumamid oder Natriumhydrid bei Temperaturen zwischen 0 und 250°C, vorzugsweise jedoch bei Temperaturen zwischen 50 und 225°C, durchgeführt.

c) Zur Herstellung von Verbindungen der allgemeinen Formel I, in der $R_{16}$ und $R_{17}$ zusammen eine weitere Bindung, $G_4$ eine Bindung und $R_{18}$ ein Chloratom darstellen:
Umsetzung einer Verbindung der allgemeinen Formel

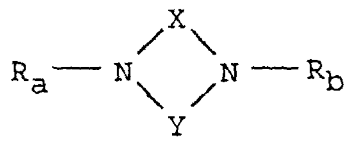

, (V)

in der
$R_a$, $R_b$, X und Y mit der Maßgabe wie eingangs definiert sind, daß einer der Reste $R_a$ bis $R_d$ eine A-B-Gruppe darstellt, in der

B wie eingangs definiert ist und

A eine Gruppe der Formel

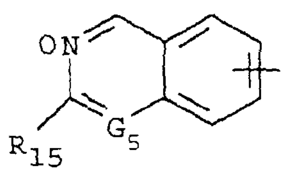

darstellt, wobei der Benzoteil, $R_{15}$ und $G_5$ wie eingangs definiert sind, in Gegenwart eines Säurehalogenids.

Die Umsetzung wird mit einem Säurehalogenid wie Phosphoroxychlorid oder Phosphoroxybromid gegebenenfalls in Gegenwart eines Lösungsmittels wie Benzol, Dichlorbenzol, Nitrobenzol, Tetrachlorkohlenstoff und gegebenenfalls in Gegenwart eines Salzes einer entsprechenden Halogenwasserstoffsäure wie Natriumchlorid oder Natriumbromid bei erhöhten Temperaturen, z.B. bei Temperaturen zwischen 50 und 250°C, vorzugsweise jedoch bei der Siedetemperatur des Reaktionsgemisches, durchgeführt.

d) Zur Herstellung von Verbindungen der allgemeinen Formel I, in der X eine durch eine Cyanogruppe substituierte Carbiminogruppe, eine Carbonyl- oder Sulfonylgruppe und Y eine gegebenenfalls durch $R_c$ oder $R_d$ oder $R_c$ und $R_d$ substituierte geradkettige Alkylengruppe mit 2 bis 4 Kohlenstoffatomen darstellt:
Cyclisierung einer Verbindung der allgemeinen Formel

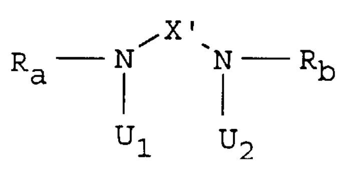

, (VI)

in der
$R_a$ und $R_b$ wie eingangs definiert sind,
X' eine durch eine Cyanogruppe substituierte Carbiminogruppe, eine Carbonyl- oder Sulfonylgruppe, einer der Reste $U_1$ oder $U_2$ ein Wasserstoffatom und der andere der Reste $U_1$ oder $U_2$ eine gegebenenfalls durch $R_c$ oder $R_d$ oder $R_c$ und $R_d$ substituierte geradkettige Alkylengruppe mit 2 bis 4 Kohlenstoffatomen, an die zusätzlich endständig eine nukleophile Austrittsgruppe wie ein Halogenatom, eine Hydroxy- oder Sulfonsäureestergruppe, z. B.

ein Chlor-, Brom- oder Jodatom, eine Hydroxy-, Methansulfonyloxy- oder p-Toluolsulfonyloxygruppe, gebunden ist.

Die Umsetzung wird vorzugsweise in einem Lösungsmittel wie Methylenchlorid, Acetonitril, Tetrahydrofuran, Toluol, Dimethylformamid oder Dimethylsulfoxid gegebenenfalls in Gegenwart einer Base wie Natriumhydrid, Kaliumkarbonat, Kalium-tert.butylat oder N-Ethyl-diisopropylamin oder gegebenenfalls in Gegenwart eines wasserentziehenden Mittels wie Triphenylphospin/Azodicarbonsäure-diethylester bei Temperaturen zwischen -20 und 100°C, vorzugsweise bei Temperaturen zwischen 0 und 60°C, durchgeführt.

e) Zur Herstellung von Verbindungen der allgemeinen Formel I, in der X eine Carbonylgruppe und Y eine der eingangs erwähnten Alkylen- oder Alkenylengruppen darstellen:
Umsetzung einer Verbindung der allgemeinen Formel

$$R_{28} - NH - T , \qquad (VII)$$

mit einem Isocyanat der allgemeinen Formel

$$O = C = N - R_{29} , \qquad (VIII)$$

in denen
einer der Reste $R_{28}$ oder $R_{29}$ die für $R_a$ eingangs erwähnten Bedeutungen besitzt und
der andere der Reste $R_{28}$ oder $R_{29}$ die für $R_b$ eingangs erwähnten Bedeutungen besitzt und
T eine im Alkylidenteil gegebenenfalls durch $R_c$ oder $R_d$ oder durch $R_c$ und $R_d$ substituierte Gruppe der Formel

$$-(CH_2)_m - HC(OR_{30})_2$$

darstellt, wobei

wobei m die Zahl 1, 2 oder 3 und

$R_{30}$ jeweils eine Alkylgruppe mit 1 bis 4 Kohlen stoffatomen darstellen,

und gegebenenfalls anschließende Hydrierung.

Die Umsetzung wird gegebenenfalls in einem inerten Lösungsmittel wie Dioxan oder Toluol bei Temperaturen zwischen 20 und 200°C, vorzugsweise bei Temperaturen zwischen 20 und 160°C, durchgeführt. Die Umsetzung kann jedoch auch ohne Lösungsmittel durchgeführt werden.

Ein gegebenenfalls bei der Umsetzung einer Verbindung der allgemeinen Formel VII und mit einem Isocyanat der allgemeinen Formel VIII als Zwischenprodukt erhaltener offenkettiger Harnstoff wird anschließend gegebenenfalls in Gegenwart einer Säure wie Essigsäure, Trifluoressigsäure, p-Toluolsulfonsäure oder Salzsäure gegebenenfalls in einem Lösungsmittel wie Methanol, Ethanol, Tetrahydrofuran oder Methylenchlorid bei Temperaturen zwischen 0°C und der Siedetemperatur des Reaktionsgemisches in die gewünschte Verbindung übergeführt.

Die gegebenenfalls anschließende Hydrierung erfolgt vorzugsweise mit Wasserstoff in Gegenwart eines Katalysators wie Palladium/Kohle oder Platin in einem Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen Raumtemperatur und 50°C, und bei einem Wasserstoffdruck von 1 bis 7 bar, vorzugsweise jedoch von 3 bis 5 bar.

f) Zur Herstellung von Verbindungen der allgemeinen Formel I, in der $G_1$ und $G_2$ jeweils eine Bindung, $G_3$ eine gegebenenfalls durch eine Alkylgruppe mit 1 bis 4 Kohlenstoffatomen substituierte Methylengruppe, $R_2$, $R_4$ und $R_5$ jeweils ein Wasserstoffatom, $R_3$ und $R_6$, die gleich oder verschieden sein können, jeweils ein Wasserstoffatom oder eine Alkylgruppe mit 1 bis 4 Kohlenstoffatomen darstellen:
Hydrierung einer Verbindung der allgemeinen Formel

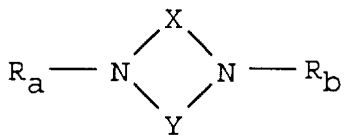

, (IX)

in der
$R_a$, $R_b$, X und Y mit der Maßgabe wie eingangs definiert sind, daß einer der Reste $R_a$ bis $R_d$ eine A-B-Gruppe darstellt, in der

B wie eingangs definiert ist und

A eine Gruppe der Formel

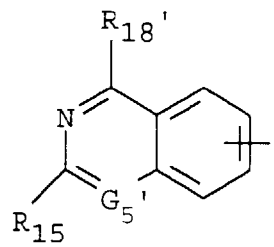

darstellt, wobei der Benzoteil und $R_{15}$ wie eingangs definiert ist,

$G_5$' eine gegebenenfalls durch eine Alkylgruppe mit 1 bis 4 Kohlenstoffatomen substituierte Methingruppe und

$R_{18}$' ein Wasserstoffatom oder eine Alkylgruppe mit 1 bis 4 Kohlenstoffatomen darstellen.

Die Hydrierung wird vorzugsweise in einem geeigneten Lösungsmittel wie Methanol, Methanol/Wasser, Essigsäure, Essigester, Ethanol, Ether, Tetrahydrofuran, Dioxan oder Dimethylformamid mit Wasserstoff in Gegenwart eines Hydrierungskatalysators wie Raney-Nickel, Platin, Platindioxid, Rhodium oder Palladium/Kohle gegebenenfalls unter Zusatz einer Säure wie Salzsäure bei Temperaturen zwischen 0 und 100°C, vorzugsweise bei Temperaturen zwischen 20 und 80°C, durchgeführt. Hierbei können gegebenenfalls in einer Verbindung der allgemeinen Formel IX vorhandene gegebenenfalls substituierte Alkenylengruppen in gegebenenfalls substituierte Alkylengruppen übergeführt werden.

g) Zur Herstellung von Verbindungen der allgemeinen Formel I, in der F eine durch eine Alkoxygruppe mit 1 bis 4 Kohlenstoffatomen, durch eine Arylalkoxygruppe mit 1 bis 4 Kohlenstoffatomen im Alkoxyteil, wobei der Arylteil wie eingangs definiert ist, oder durch eine $R_{22}$O-Gruppe substituierte Carbonylgruppe darstellt:
Umsetzung einer Verbindung der allgemeinen Formel

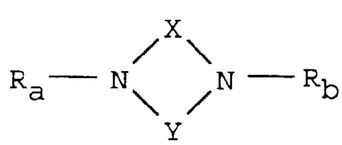

, (X)

in der
$R_a$, $R_b$, X und Y mit der Maßgabe wie eingangs definiert sind, daß einer der Reste $R_a$ bis $R_d$ eine F"-E-D-Gruppe darstellt, in der

E und D wie eingangs definiert sind und
F" eine Carboxy- oder Alkoxycarbonylgruppe darstellt,

mit einem Alkohol der allgemeinen Formel

$$HO - R_{31} , \qquad (XI)$$

in der

$R_{31}$ die für $R_{22}$ eingangs erwähnten Bedeutungen besitzt sowie eine Alkylgruppe mit 1 bis 4 Kohlenstoffatomen oder eine Arylalkylgruppe, in welcher der Arylteil wie eingangs erwähnt definiert ist und der Alkylteil 1 bis 4 Kohlenstoffatome enthalten kann, darstellt.

Die Umsetzung einer Carboxyverbindung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder besonders vorteilhaft in einem entsprechenden Alkohol der allgemeinen Formel XI gegebenenfalls in Gegenwart einer Säure wie Salzsäure oder in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Schwefelsäure, Methansulfonsäure, p-Toluolsulfonsäure, Phosphortrichlorid, Phosphorpentoxid, N,N'-Di-cyclohexylcarbodiimid, N,N'-Dicyclohexylcarbodiimid/N-Hydroxysuccinimid, N,N'-Dicyclohexylcarbodiimid/1-Hydroxybenztriazol, N,N'-Carbonyldiimidazol oder Triphenylphosphin/Tetrachlorkohlenstoff, und gegebenenfalls unter Zusatz eine Base wie Pyridin, 4-Dimethylaminopyridin oder Triethylamin zweckmäßigerweise bei Temperaturen zwischen 0 und 150°C, vorzugsweise bei Temperaturen zwischen 0 und 100°C, durchgeführt.

Die Umsetzung einer entsprechende Alkoxycarbonylverbindung mit einem Alkohol der allgemeinen Formel XI wird vorzugsweise in einem entsprechenden Alkohol als Lösungsmittel gegebenenfalls in Gegenwart eines weiteren Lösungsmittels wie Methylenchlorid oder Ether vorzugsweise in Gegenwart einer Säure wie Salzsäure bei Temperaturen zwischen 0 und 150°C, vorzugsweise bei Temperaturen zwischen 50 und 100°C, durchgeführt.

h) Zur Herstellung von Verbindungen der allgemeinen Formel I, in der $R_4$ einen der bei der Definition von $R_4$ eingangs erwähnten gegebenenfalls substituierten Alkylreste, Alkenyl-, Cycloalkyl-, Cycloalkylalkyl- oder Aralkylreste darstellt:

Umsetzung einer Verbindung der allgemeinen Formel

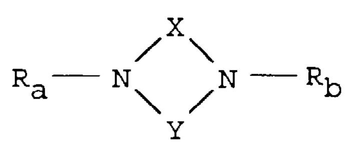

, (XI)

in der

$R_a$, $R_b$, X und Y mit der Maßgabe wie eingangs definiert sind, daß einer der Reste $R_a$ bis $R_d$ eine A-B-Gruppe darstellt, in der

A und B mit der Maßgabe wie eingangs definiert sind, daß $R_4$ ein Wasserstoffatom darstellt,

mit einer Verbindung der allgemeinen Formel

$$Z_3 - R_{32} , \qquad (XIII)$$

in der

$R_{32}$ eine Alkylgruppe mit 1 bis 6 Kohlenstoffatomen, eine Cycloalkyl- oder Cycloalkylalkylgruppe, in denen der Cycloalkylteil jeweils 3 bis 7 Kohlenstoffatome und der Alkylteil 1 bis 4 Kohlenstoffatome enthalten kann, eine Alkenylgruppe mit 3 bis 6 Kohlenstoffatomen, eine Arylalkyl-, Hydroxyalkyl-, Alkoxyalkyl-, Carboxyalkyl-, Alkoxycarbonylalkyl-, Aminocarbonylalkyl-, N-Alkylaminocarbonylalkyl- oder N,N-Dialkylaminocarbonylgruppe, in denen der Arylteil und die Alkylteile wie eingangs definiert sind, und

$Z_3$ eine nukleophile Austrittsgruppe wie ein Halogenatom, z.B. ein Chlor-, Brom- oder Jodatom, oder eine Sulfonsäureestergruppe, z.B. eine Methansulfonyloxy- oder p-Toluolsulfonyloxygruppe oder

$Z_3$ zusammen mit einem benachbarten Wasserstoffatom des Restes $R_{32}$ ein Sauerstoffatom bedeuten.

Die Alkylierung mit einer Verbindung der Formel XIII, in der $Z_3$ eine nukleophile Austrittsgruppe darstellt, wird zweckmäßigerweise in einem Lösungsmittel wie Methylenchlorid, Tetrahydrofuran, Dioxan, Dimethylsulfoxid oder Dimethylformamid gegebenenfalls in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig als Lösungsmittel dienen können, bei Temperaturen zwischen -30 und 150°C, vorzugsweise

jedoch bei Temperaturen zwischen 20 und 120°C, durchgeführt.

Die reduktive Alkylierung mit einer Carbonylverbindung der allgemeinen Formel XIII wird in Gegenwart eines komplexen Metallhydrids wie Natriumborhydrid, Lithiumborhydrid oder Natriumcyanborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck 1 bis 5 bar durchgeführt. Die Methylierung wird jedoch vorzugsweise in Gegenwart von Ameisensäure als Reduktionsmittel bei erhöhten Temperaturen, z. B. bei Temperaturen zwischen 60 und 120°C, durchgeführt.

i) Zur Herstellung von Verbindungen der allgemeinen Formel I, in der X eine am Stickstoffatom durch eine Cyanogruppe substituierte Carbiminogruppe, eine Carbonyl- oder Sulfonylgruppe darstellt:
Umsetzung einer Verbindung der allgemeinen Formel

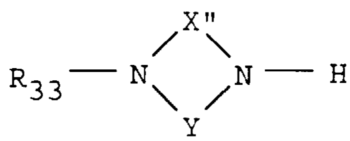
, (XIV)

mit einer Verbindung der allgemeinen Formel

$$Z_4 - R_{34} , \quad (XV)$$

in denen
Y wie eingangs definiert ist,
X" eine am Stickstoffatom durch eine Cyanogruppe substituierte Carbiminogruppe, eine Carbonyl- oder Sulfonylgruppe, einer der Reste $R_{33}$ oder $R_{34}$ die für $R_a$ eingangs erwähnten Bedeutungen besitzt und
der andere der Reste $R_{33}$ oder $R_{34}$ die für $R_b$ eingangs erwähnten Bedeutungen besitzt und
$Z_4$ eine nukleophile Austrittsgruppe wie ein Halogenatom, eine Hydroxy- oder Sulfonsäureestergruppe, z. B. ein Fluor-, Chlor-, Brom- oder Jodatom, eine Methansulfonyloxy- oder p-Toluolsulfonyloxygruppe, bedeuten.

Die Umsetzung wird vorzugsweise in einem Lösungsmittel wie Methylenchlorid, Acetonitril, Tetrahydrofuran, Toluol, Pyridin, Dimethylformamid, Dimethylsulfoxid oder N-Methyl-pyrrolidon gegebenenfalls in Gegenwart einer oder mehrerer Basen wie Natriumhydrid, Kaliumkarbonat, Kalium-tert.butylat, N-Ethyl-diisopropylamin, Tris-[2-(2-methoxyethoxy)ethyl] amin oder N,N,N',N'-Tetramethylethylendiamin und gegebenenfalls in Gegenwart eines wasserentziehenden Mittels wie Triphenylphosphin/Azodicarbonsäure-diethylester und gegebenenfalls in Gegenwart von Kupferpulver oder eines oder mehrerer Kupfersalzen wie Kupfer(I)jodid als Reaktionsbeschleuniger bei Temperaturen zwischen -20 und 250°C, vorzugsweise jedoch bei Temperaturen zwischen 0 und 60°C, falls $Z_4$ an ein aliphatisches Kohlenstoffatom gebunden ist, oder bei Temperaturen zwischen 60 und 180°C, falls $Z_4$ an ein aromatisches Kohlenstoffatom gebunden ist, wobei in diesem Falle $Z_4$ nur ein Halogenatom darstellen kann.

j) Zur Herstellung von Verbindungen der allgemeinen Formel I, in der $R_4$ eine Alkoxycarbonyl-, Arylmethyloxycarbonyl-, Formyl-, Acetyl-, Trifluoracetyl- oder $R_{11}$CO-O-($R_{12}$C$R_{13}$)-O-CO-Gruppe, in denen $R_{11}$ bis $R_{13}$ und der Arylteil wie eingangs definiert sind und der Alkoxyteil 1 bis 4 Kohlenstoffatome enthalten kann, darstellt:
Umsetzung einer Verbindung der allgemeinen Formel

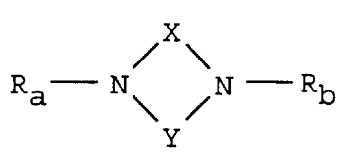
, (XVI)

in der
$R_a$, $R_b$, X und Y mit der Maßgabe wie eingangs definiert sind, daß $R_4$ ein Wasserstoffatom darstellt, mit einer Verbindung der allgemeinen Formel

$$Z_5 - R_{35} , \quad (XVII)$$

in der

$R_{35}$ eine Alkoxycarbonylgruppe mit insgesamt 2 bis 5 Kohlenstoffatomen, eine Arylmethyloxycarbonylgruppe, in der der Arylteil wie eingangs definiert ist, eine $R_{11}CO\text{-}O\text{-}(R_{12}CR_{13})\text{-}O\text{-}CO\text{-}$, Formyl-, Acetyl- oder Trifluoracetylgruppe, wobei $R_{11}$ bis $R_{13}$ wie eingangs definiert sind, und

$Z_5$ eine nukleophile Austrittsgruppe wie ein Halogenatom, eine Aryloxy-, Arylthio-, Alkoxy-, Alkoxycarbonyloxy-, Aralkoxycarbonyloxy- oder N-Imidazolylgruppe, z. B. ein Chlor- oder Bromatom oder eine 4-Nitro-phenoxygruppe, darstellen.

Die Acylierung wird zweckmäßigerweise in einem Lösungsmittel wie Tetrahydrofuran, Methylenchlorid, Chloroform, Dimethylformamid, Wasser oder Gemischen aus diesen Lösungsmitteln gegebenenfalls in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 60°C, durchgeführt.

k) Zur Herstellung von Verbindungen der allgemeinen Formel I, in der F eine Carbonylgruppe darstellt, die durch eine Alkoxygruppe mit 1 bis 4 Kohlenstoffatomen, durch eine Arylalkoxygruppe, in der der Arylteil wie eingangs erwähnt definiert ist und der Alkoxyteil 1 bis 4 Kohlenstoffatome enthalten kann, durch eine $R_{22}O$- oder $R_{23}CO\text{-}O\text{-}CHR_{24}\text{-}O$-Gruppe substituiert ist, wobei $R_{22}$ bis $R_{24}$ wie eingangs erwähnt definiert sind:

Umsetzung einer Verbindung der allgemeinen Formel

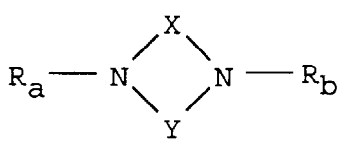

, (XVIII)

in der

$R_a$, $R_b$, X und Y mit der Maßgabe wie eingangs definiert sind, daß einer der Reste $R_a$ bis $R_d$ eine F'''-E-D-Gruppe darstellt, in der

E und D wie eingangs definiert sind und

F''' eine Carboxylgruppe darstellt, mit einer Verbindung der allgemeinen Formel

$$Z_6 - R_{36} , \quad (XIX)$$

in der

$R_{36}$ eine Alkylgruppe mit 1 bis 4 Kohlenstoffatomen, eine Arylalkylgruppe, in der der Arylteil wie eingangs erwähnt definiert ist und der Alkylteil 1 bis 4 Kohlenstoffatome enthalten kann, eine $R_{22}$- oder $R_{23}CO\text{-}O\text{-}CHR_{24}$-Gruppe, wobei $R_{22}$ bis $R_{24}$ wie eingangs erwähnt definiert sind, und

$Z_6$ eine nukleophile Austrittsgruppe wie ein Halogenatom oder eine Sulfonsäureestergruppe, z. B. ein Chlor- oder Bromatom, eine Methansulfonyloxy- oder p-Toluolsulfonyloxygruppe, bedeuten.

Die Umsetzung wird vorzugsweise in einem Lösungsmittel wie Methylenchlorid, Tetrahydrofuran, Dioxan, Dimethylsulfoxid oder Dimethylformamid gegebenenfalls in Gegenwart eines Reaktionsbeschleunigers wie Natrium- oder Kaliumjodid und vorzugsweise in Gegenwart einer Base wie Natriumkarbonat, Kaliumkarbonat oder Natronlauge oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

l) Zur Herstellung von Verbindungen der allgemeinen Formel I, in der X eine Carbonylgruppe und Y eine gegebenenfalls durch $R_c$ oder $R_d$ oder $R_c$ und $R_d$ substituierte Ethylengruppe darstellt, in der eine Methylengruppe durch eine Carbonylgruppe ersetzt ist:

Cyclisierung einer gegebenenfalls im Reaktionsgemisch gebildeten Verbindung der allgemeinen Formel

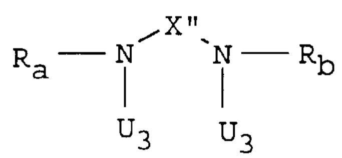

, (XX)

in der
$R_a$ und $R_b$ wie eingangs definiert sind,
X" eine Carbonylgruppe,
einer der Reste $U_3$ oder $U_4$ ein Wasserstoffatom und der andere der Reste $U_3$ oder $U_4$ eine gegebenenfalls durch $R_c$ oder $R_d$ oder $R_c$ und $R_d$ substituierte Ethylengruppe, in der eine Methylengruppe durch eine $Z_7$-CO-Gruppe ersetzt ist, wobei $Z_7$ eine nukleophile Austrittsgruppe wie ein Halogenatom, eine Hydroxy-, Alkoxy-, Aryloxy- oder Arylalkoxygruppe, z. B. ein Chlor- oder Bromatom, eine Hydroxy-, Methoxy-, Ethoxy-, Phenoxy- oder Benzyloxygruppe, darstellt.

Die Umsetzung wird vorzugsweise in einem Lösungsmittel wie Methylenchlorid, Acetonitril, Tetrahydrofuran, Toluol, Dimethylformamid oder Dimethylsulfoxid gegebenenfalls in Gegenwart einer Base wie Natriumhydrid, Kaliumkarbonat, Kaliumtert.butylat oder N-Ethyl-diisopropylamin oder gegebenenfalls in Gegenwart eines wasserentziehenden Mittels wie Triphenylphospin/Azodicarbonsäure-diethylester oder N,N'-Carbonyldiimidazol bei Temperaturen zwischen -20 und 200°C, vorzugsweise bei Temperaturen zwischen 0 und 160°C, durchgeführt.
Erhält man erfindungsgemäß eine Verbindung der allgemeinen Formel I, welche eine ungesättigte Kohlenstoff-Kohlenstoff-Bindung enthält, so kann diese mittels katalytischer Hydrierung in eine entsprechende gesättigte Verbindung der allgemeinen Formel I übergeführt werden.

Die katalytische Hydrierung erfolgt vorzugsweise mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar.

Bei den vorstehend beschriebenen Umsetzungen können gegebenenfalls vorhandene reaktive Gruppen wie Hydroxy-, Carboxy-, Phosphono-, O-Alkyl-phosphono-, Amino-, Alkylamino-, Iminooder Amidinogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe,
als Schutzreste für eine Phosphonogruppe eine Alkylgruppe wie die Methyl-, Ethyl-, Isopropyl- oder n-Butylgruppe, die Phenyl- oder Benzylgruppe,
als Schutzrest für eine gegebenenfalls durch eine Alkylgruppe substituierte Amidinogruppe die Benzyloxycarbonylgruppe und
als Schutzrest für eine Amino-, Alkylamino- oder Iminogruppe die Formyl-, Acetyl-, Trifluoracetyl-, Ethoxycarbonyl-, tert.Butoxycarbonyl-, Benzyloxycarbonyl-, Benzyl-, Methoxybenzyl- oder 2,4-Dimethoxybenzylgruppe, für die Iminogruppe zusätzlich die Methylgruppe und für die Aminogruppe die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Ether.

Die Abspaltung eines Trifluoracetylrestes erfolgt vorzugsweise durch Behandlung mit einer Säure wie Salzsäure

gegebenenfalls in Gegenwart eines Lösungsmittels wie Essigsäure oder Methanol bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Die Abspaltung einer Methylgruppe von einer Methyliminogruppe erfolgt vorzugsweise in Gegenwart von Chlorameisensäure-1-chloralkylestern wie Chlorameisensäure-1-chlorethylester vorzugsweise in Gegenwart einer Base wie 1,8-Bis-(dimethylamino)-naphthalin in Gegenwart eines Lösungsmittels wie Methylenchlorid, 1,2-Dichlorethan, Toluol oder Dioxan bei Temperaturen zwischen 0 und 150°C, vorzugsweise bei Temperaturen zwischen 20°C und der Siedetemperatur des Reaktionsgemisches, und nachfolgender Behandlung mit einem Alkohol wie Methanol bei Temperaturen zwischen 20°C und der Siedetemperatur des verwendeten Alkohols.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung nur eines Alkylrestes von einer O,O'-Dialkylphosphonogruppe erfolgt beispielsweise mit Natriumjodid in einem Lösungsmittel wie Aceton, Ethylmethylketon, Acetonitril oder Dimethylfurmamid bei Temperaturen zwischen 40 und 150°C, vorzugsweise jedoch bei Temperaturen zwischen 60 und 100°C.

Die Abspaltung beider Alkylreste von einer O,O'-Dialkylphosphonogruppe erfolgt beispielsweise mit Jodtrimethylsilan, Bromtrimethylsilan oder Chlortrimethylsilan/Natriumjodid in einem Lösungsmittel wie Methylenchlorid, Chloroform oder Acetonitril bei Temperaturen zwischen O°C und der Siedetemperatur des Reaktionsgemisches, vorzugsweise jedoch bei Temperaturen zwischen 20 und 60°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiver Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxyl-, Sulfo-, Phosphono-, O-Alkyl-phosphono- oder Tetrazol-5-yl-Gruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren (siehe Beispiele).

So wird beispielsweise in "The Organic Chemistry of Heterocyclic Compounds", Band 37, von C. Temple, Jr., Verlag John Wiley & Sons, 1981, in den Kapiteln 13, 14 und 19 die Herstellung von entsprechenden Triazol-Verbindungen beschrieben.

In Houben-Weyl, "Methoden der Organischen Chemie", Band E4, von H. Hagemann, Georg Thieme Verlag, 1983, wird beispielsweise ab Seite 368 die Herstellung von entsprechenden cyclischen Harnstoffverbindungen beschrieben. Außerdem wird im gleichen Band ab Seite 355 beispielsweise auch die Herstellung von entsprechenden gegebenenfalls als Ausgangsverbindungen benötigten offenkettigen Harnstoffverbindungen beschrieben.

So erhält man beispielsweise ein entsprechendes cyclisches Harnstoffderivat durch Cyclisierung eines entsprechend substituierten Harnstoffs, welcher seinerseits durch Umsetzung eines entsprechenden Amins mit einem entsprechenden Isocyanat erhalten wird, oder durch Umsetzung eines entsprechend substituierten Diamins mit einem Kohlensäurederivat wie Phosgen oder
ein entsprechendes Triazolonderivat durch Cyclisierung eines entsprechenden Semicarbazids, welches seinerseits durch Umsetzung eines entsprechenden Isocyanats mit einem entsprechenden Hydrazid erhalten wird.

In den so erhaltenen cyclischen Harnstoffderivaten kann gegebenenfalls anschließend eine Carbonylgruppe in eine entsprechende Thiocarbonyl- oder Carbiminogruppe mittels bekannten Methoden übergeführt werden.

In den so erhaltenen cyclischen Ausgangsverbindungen bzw. bereits in den für ihre Herstellung erforderlichen Ausgangsverbindungen kann eine gegebenenfalls vorhandene Estergruppe mittels Hydrolyse in eine Carboxylgruppe oder eine gegebenenfalls vorhandene Carboxylgruppe in eine Estergruppe übergeführt werden.

Wie bereits eingangs erwähnt, weisen die neuen cyclischen Derivate der allgemeinen Formel I und deren Salze, insbesondere deren physiologisch verträgliche Salze mit anorganischen oder organischen Säuren oder Basen, wertvolle Eigenschaften auf. So weisen die neuen Verbindungen der allgemeinen Formel I wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Beispielsweise wurden die Verbindungen der allgemeinen Formel I auf ihre biologischen Wirkungen wie folgt untersucht:

1. <u>Hemmung der Bindung von $^3$H-BIBU 52 an Humanthrombozyten:</u>

Eine Suspension von Humanthrombozyten in Plasma wird mit $^3$H-BIBU 52 [= (3S,5S)-5-[(4'-Amidino-4-biphenylyl) oxymethyl]-3-[(carboxyl)methyl]-2-pyrrolidinon[3-$^3$H-4-biphenylyl]], das den literaturbekannten Liganden $^{125}$J-Fibrinogen ersetzt, (siehe deutsche Patentanmeldung P 42 14 245.8 der gleichen Anmelderin vom 30. 04. 1992, internes Zeichen: Case 5/1093-FL) und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^3$H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma nocheinmal scharf abzentrifugiert. Das PRP wird mit autologem Plasma 1:10 verdünnt. 750 µl werden mit 50 µl physiologischer Kochsalzlösung, 100 µl Testsubstanzlösung, 50 µl $^{14}$C-Sucrose (3.700 Bq) und 50 µl $^3$H-BIBU 52 (Endkonzentration: 5 nM) bei Raumtemperatur 20 Minuten inkubiert. Zur Messung der unspezifischen Bindung wird anstelle der Testsubstanz 5 µl BIBU 52 (Endkonzentration: 30 µM) eingesetzt. Die Proben werden 20 Sekunden bei 10000 x g zentrifugiert und der Überstand abgezogen. 100 µl hiervon werden zur Bestimmung des freien Liganden gemessen. Das Pellet wird in 500 µl 0,2N NaOH gelöst, 450 µl werden mit 2 ml Szintillator und 25 µl 5N HCl versetzt und gemessen. Das im Pellet noch verbliebene Restplasma wird aus dem $^{14}$C-Gehalt bestimmt, der gebundene Ligand aus der $^3$H-Messung. Nach Abzug der unspezifischen Bindung wird die Pelletaktivität gegen die Konzentration der Testsubstanz aufgetragen und die Konzentration für eine 50%ige Hemmung der Bindung ermittelt.

2. <u>Antithrombotische Wirkung:</u>

<u>Methodik</u>

Die Thrombozytenaggregation wird nach der Methode von Born und Cross (J. Physiol. <u>170</u>, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

<u>Collagen-induzierte Aggregation</u>

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37°C inkubiert.

Aus den erhaltenen Meßzahlen wird graphisch eine $EC_{50}$ bestimmt, die sich auf eine 50%ige Änderung der "optical

density" im Sinne einer Aggregationshemmung bezieht.

Die nachfolgende Tabelle enthält die gefundenen Ergebnisse:

| Substanz (Beispiel Nr.) | $^3$H-BIBU 52-Bindungstest $IC_{50}$[nM] | Hemmung der Plättchenaggregation $EC_{50}$[nM] |
|---|---|---|
| 1 | 190 | >10000 |
| 2(1) | 5400 | >10000 |
| 2(2) | 24 | 40 |
| 6 | 110 | 40 |
| 8(1) | 36 | 150 |
| 9 | 85 | 190 |
| 9(1) | 760 | 990 |
| 19 | 59 | 230 |
| 19(1) | 120 | 350 |
| 19(3) | 81 | 430 |

Außerdem hemmen beispielsweise die Verbindungen der Beispiele 7 und 19 die durch Collagen-induzierte Thrombozytenaggregation ex vivo am Rhesusaffen nach oraler Gabe von 1 mg/kg über 5 bzw. über 8 Stunden.

Die erfindungsgemäßen Verbindungen sind gut verträglich, da beispielsweise nach intravenöser Gabe von 30 mg/kg der Verbindung des Beispiels 8(1) oder 19 an der Maus keines der 3 getesteten Tiere verstarb.

Auf Grund ihrer Hemmwirkung auf Zell-Zell- bzw. Zell-Matrix-Wechselwirkungen eignen sich die neuen cyclischen Harnstoffderivate der allgemeinen Formel I und ihre physiologisch verträglichen Salze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 µg und 30 mg/kg Körpergewicht, vorzugsweise bei 1 µg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, α-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zu, sammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/ Polyäthylenglykol, Propylenglykol, Stearylalkohol, Carboxymethylcellulose oder fetthaltigen Substanze wie Hartfett oder deren geeigneten Gemischen, in übliche galenische Zubereitungen wie Tabletten, Dragees, Kapseln, Pulver, Suspensionen, Lösungen, Sprays oder Zäpfchen einarbeiten.

Die nachfolgenden Beispiele sollen die Erfindung näher erläutern:

Beispiel I

1-(Isochinolin-N-oxid-6-yl)-3-[4-[2-(methoxycarbonyl)ethyl]-phenyl]-imidazolidin-2-on

2,6 g 1-(Isochinolin-6-yl)-3-[4-[2-(methoxycarbonyl)ethyl]-phenyl]-imidazolidin-2-on werden in 175 ml Methylenchlorid unter Erwärmen gelöst. Es wird im Eisbad abgekühlt und mit 1,46 g 3-Chlorperoxybenzoesäure (90 %) versetzt. Nach 24 Stunden Rühren bei 0°C wird mit Methylenchlorid verdünnt und je zweimal mit Natriumhydrogencarbonat- und Natriumthiosulfatlösung und dann Wasser ausgeschüttelt. Die organische Phase wird abgetrennt, getrocknet und einrotiert.

Ausbeute: 2,4 g (88 % der Theorie),

$R_f$-Wert: 0,15 (Kieselgel; Methylenchlorid/Methanol/Essigester = 20:1:1)

Beispiel II

N-(2-Hydroxyethyl)-N'-(isochinolin-6-yl)-N-[4-[2-(methoxycarbonyl)ethyl]phenyl] -harnstoff

Zu 7,2 g Imidazol und 10,1g N,N'-Carbonyldiimidazol in 100 ml Dimethylformamid werden bei einer Temperatur von 0 bis 10°C 9,0 g 6-Aminoisochinolin in 70 ml Dimethylformamid zugetropft. Nach 2 Stunden Rühren bei Raumtemperatur werden 15,3 g N-(2-Hydroxyethyl)-4-[2-(methoxycarbonyl)ethyl]-anilin in 20 ml Dimethylformamid zugetropft und 2 1/2 Tage bei Raumtemperatur gerührt. Es wird mit 750 ml Essigester verdünnt und je zweimal mit Wasser und gesättigter Kochsalzlösung ausgeschüttelt. Die organische Phase wird abgetrennt, getrocknet und eingeengt. Der Rückstand wird durch Chromatographie über eine Kieselgelsäule mit Essigester/Methylenchlorid/Methanol = 70:30:10 gereinigt.
Ausbeute: 4,8 g (19 % der Theorie),
$R_f$-Wert: 0,48 (Kieselgel; Methylenchlorid/Methanol/Essigester = 20:1:1)

Analog Beispiel II werden folgende Verbindungen erhalten:

(1) N-(2,2-Diethoxyethyl)-N'-(isochinolin-6-yl)-N-[4-[2-(methoxycarbonyl)ethyl]phenyl]-harnstoff $R_f$-Wert: 0,50 (Kieselgel; Methylenchlorid/Methanol = 20:1)

(2) N-(3-Ethyl-2,3,4,5-tetrahydro-1H-3-benzazepin-7-yl)-N'-(2,2-diethoxyethyl)-N'-[trans-4-[2-(methoxycarbonyl) ethyl]-cyclohexyl]-harnstoff
Schmelzpunkt: 101-104°C
$R_f$-Wert: 0,63 (Kieselgel; Methylenchlorid/Methanol/konz. wäßeriges Ammoniak = 90:10:2)

(3) N-(7-Ethyl-6,7,8,9-tetrahydro-5H-pyrazino[2,3-d]azepin-2-yl)-N'-(2,2-diethoxyethyl)-N'-[trans-4-[2-(methoxycarbonyl)-ethyl]cyclohexyl]-harnstoff
Durchführung mit 1,1'-Carbonyl-di-(1,2,4-triazol)
Schmelzpunkt: 100-102°C
$R_f$-Wert: 0,41 (Kieselgel; Methylenchlorid/Methanol /Methanol = 9:1)

| | | | | | | |
|---|---|---|---|---|---|---|
| Ber. | C | 62,40 | H | 8,73 | N | 13,48 |
| Gef. | | 62,19 | | 8,79 | | 13,62 |

(4) N-(7-Benzyl-6,7,8,9-tetrahydro-5H-pyrimido[4,5-d]azepin-2-yl)-N'-(2,2-diethoxyethyl)-N'-[trans-4-[2-(methoxycarbonyl)-ethyl]cyclohexyl]-harnstoff
Durchführung mit 1,1'-Carbonyl-di-(1,2,4-triazol)
$R_f$-Wert: 0,23 (Reversed Phase Kieselgel; Methanol/5%ige wäßerige Kochsalzlösung = 6:4)

Beispiel III

N-(3-tert.Butyloxycarbonyl-2,3,4,5-tetrahyäro-1H-3-benzazepin-7-yl)-N-(2-hydroxyethyl)-N'--[4-[2-(methoxycarbonyl) ethyl]-phenyl]-harnstoff

4,5 g 3-(tert.Butyloxycarbonyl)-7-[(2-hydroxyethyl)amino]-2,3,4,5-tetrahydro-1H-3-benzazepin und 3,7 g 4-[2-(Methoxycarbonyl)ethyl]-phenylisocyanat (hergestellt aus dem entsprechenden Amin durch Umsetzung mit Phosgen) werden in 35 ml Dioxan 3,5 Stunden bei Raumtemperatur gerührt. Das Reaktionsgemisch wird eingeengt, der Rückstand wird mit tert.Butyl-methylether verrieben und abgesaugt. Das Produkt wird mit tert.Butyl-methylether gewaschen und getrocknet.
Ausbeute: 6,3 g (76 % der Theorie),
Schmelzpunkt: 115-117°C,
$R_f$-Wert: 0,30 (Kieselgel; Cyclohexan/Essigester = 1:1)
Analog Beispiel III werden folgende Verbindungen erhalten:

(1) 1-Acetyl-4-[4-[2-(methoxycarbonyl)ethyl]phenyl]-semicarbazid (Umsetzung von Acetylhydrazin mit 4-[2-(Methoxycarbonyl)-ethyl]-phenylisocyanat)
Schmelzpunkt: 159-165°C
$R_f$-Wert: 0,37 (Kieselgel; Methylenchlorid/Methanol = 9:1)

(2) N-(2-Hydroxyethyl)-N-[trans-4-[2-(methoxycarbonyl)ethyl]-cyclohexyl]-N'-(3-trifluoracetyl-2,3,4,5-tetrahydro-1H-3-benzazepin-7-yl)-harnstoff
Schmelzpunkt: 150-152°C
$R_f$-Wert: 0,31 (Kieselgel; Cyclohexan/Essigester = 1:1)

(3) N-(2,2-Diethoxyethyl)-N-(3-trifluoracetyl-2,3,4,5-tetrahydro-1H-3-benzazepin-7-yl)-N'-[trans-4-[2-(methoxycarbonyl)-ethyl]-cyclohexyl]-harnstoff
Das eingesetzte [trans-4-[2-(Methoxycarbonyl)ethyl]cyclohexyl]-isocyanat wird aus dem entsprechenden Aminhydrochlorid durch Umsetzung mit Phosgen erhalten. Das eingesetzte 7-[(2,2-Diethoxyethyl)amino]-3-trifluoracetyl-2,3,4,5-tetrahydro-1H-3-benzazepin [$R_f$-Wert: 0,76 (Kieselgel; Cyclohexan/Essigester = 3:2)] wird durch Umsetzung von 7-Amino-3-trifluoracetyl-2,3,4,5-tetrahydro-1H-3-benzazepin mit Bromacetaldehyd-diethylacetal in Gegenwart von N-Ethyl-diisopropylamin erhalten.
$R_f$-Wert: 0,26 (Kieselgel; Cyclohexan/Essigester = 3:2)

(4) N-[trans-[[(Methoxycarbonyl)methyl]oxy] cyclohexyl]-N'-(2,2-diethoxyethyl)-N'-(3-trifluoracetyl-2,3,4,5-tetrahydro-1H-3-benzazepin-7-yl)-harnstoff
Das eingesetzte [trans-4-[[(Methoxycarbonyl)methyl]oxy]-cyclohexyl]-isocyanat wird aus dem entsprechenden Aminhydrochlorid durch Umsetzung mit Phosgen erhalten.
$R_f$-Wert: 0,20 (Kieselgel; Cyclohexan/Essigester = 1:1)

(5) N-(2-Hydroxyethyl)-N-[4-[trans-2-(methoxycarbonyl)-ethenyl]phenyl]-N'-(3-trifluoracetyl-2,3,4,5-tetrahydro-1H-3-benzazepin-7-yl)-harnstoff
Das eingesetzte (3-Trifluoracetyl-2,3,4,5-tetrahydro-1H-3-benzazepin-7-yl)-isocyanat wird aus dem entsprechenden Amin durch Umsetzung mit Phosgen erhalten.
Schmelzpunkt: ab 118°C
$R_f$-Wert: 0,18 (Kieselgel; Cyclohexan/Essigester = 1:1)

(6) N-[4-(2-(Ethoxycarbonyl)-1-propyl]phenyl]-N'-(2,2-diethoxyethyl)-N'-(3-trifluoracetyl-2,3,4,5-tetrahydro-1H-3-benzazepin-7-yl)-harnstoff
Das eingesetzte [4-[2-(Ethoxycarbonyl)-1-propyl]phenyl]-isocyanat wird durch Umsetzung des entsprechenden Amins mit Phosgen erhalten.
$R_f$-Wert: 0,35 (Kieselgel; Cyclohexan/Essigester = 2:1)

(7) N-[trans-4-[2-(Methoxycarbonyl)ethyl]cyclohexyl]-N'-[(benzyloxycarbonyl)methyl]-N'-(3-trifluoracetyl-2,3,4,5-tetrahydro-1H-3-benzazepin-7-yl)-harnstoff
Das eingesetzte 7-[[(Benzyloxycarbonyl)methyl]amino]-3-trifluoracetyl-2,3,4,5-tetrahydro-1H-3-benzazepin [$R_f$-Wert: 0,24 (Kieselgel; Cyclohexan/Essigester = 4:1)] wird durch Umsetzung von 7-Amino-3-trifluoracetyl-2,3,4,5-tetrahydro-1H-3-benzazepin mit Bromessigsäure-benzylester in Gegenwart von N-Ethyl-diisopropylamin erhalten.
$R_f$-Wert: 0,51 (Kieselgel; Cyclohexan/Essigester = 1:1

(8) N-(3-Hydroxypropyl)-N-[trans-4-[2-Methoxycarbonyl)-ethyl]cyclohexy]-N'-(3-trifluoracetyl-2,3,4,5-tetrahydro-1H-3-benzazepin-7-yl)-harnstoff
Schmelzpunkt: 129-130°C

(9) 1-Acetyl-4-[trans-4-[2-(methoxycarbonyl)ethyl]-cyclohexyl]-semicarbazid
Schmelzpunkt: 176-179°C
$R_f$-Wert: 0,24 (Kieselgel; Methylenchlorid/Methanol = 95:5)

(10) 1-Formyl-4-[trans-4-[2-(methoxycarbonyl)ethyl]-cyclohexyl]-semicarbazid
Schmelzpunkt: 169-171°C

(11) N-[4-[2-(Ethoxycarbonyl)-1-phenyl-ethyl]phenyl]-N'-(2,2-diethoxyethyl)-N'-(3-trifluoracetyl-2,3,4,5-tetrahydro-1H-3-benzazepin-7-yl)-harnstoff
Das eingesetzte [4-[2-(Ethoxycarbonyl)-1-phenyl-ethyl]phenyl]-isocyanat wird aus dem entsprechenden Amin durch Umsetzung mit Phosgen erhalten.
Das Rohprodukt wird direkt zur Herstellung der Verbindung des Beispiels 5(6) eingesetzt.

(12) 1-Formyl-4-(3-trifluoracetyl--2,3,4,5-tetrahydro-1H-3-benzazepin-7-yl)-semicarbazid
$R_f$-Wert: 0,41 (Kieselgel; Methylenchlorid/Methanol = 9:1)

Beispiel IV

3-(tert.Butyloxycarbonyl)-7-[(2-hydroxyethyl)amino]-2,3,4,5-tetrahydro-1H-3-benzazepin

7,2 g 7-Amino-3-(tert.butyloxycarbonyl)-2,3,4,5-tetrahydro-1H-3-benzazepin (hergestellt aus 7-Nitro-2,3,4,5-tetrahydro-1H-3-benzazepin durch Umsetzung mit Pyrokohlensäure-di-tert.-butylester und nachfolgender Hydrierung in Gegenwart von Palladium auf Aktivkohle) in 130 ml Methanol werden unter Rühren mit 1,53 ml Eisessig und 1,8 g Glykolaldehyd (dimer) versetzt. Danach werden 1,9 g Natriumcyanborhydrid zugegeben und eine Stunde bei Raumtemperatur gerührt. Das Reaktionsgemisch wird eingedampft, der Rückstand zwischen Essigester und Wasser verteilt. Die organische Phase wird abgetrennt, zweimal mit Wasser gewaschen, getrocknet und einrotiert. Der Rückstand wird durch Chromatographie über eine Kieselgelsäule mit Cyclohexan/Essigester (4:6) gereinigt.
Ausbeute: 5,4 g (64 % der Theorie),
Schmelzpunkt: 86-89°C,
$R_f$-Wert: 0,39 (Kieselgel; Cyclohexan/Essigester = 4:6)
Analog Beispiel IV wird folgende Verbindung erhalten:

(1) trans-4-[(2-Hydroxyethyl)amino]-zimtsäure-methylester Schmelzpunkt: 117-118°C
$R_f$-Wert: 0,25 (Kieselgel; Cyclohexan/Essigester = 1:1)

Beispiel V

(4aRS,6RS,8aRS)-6-[(2,2-Dimethoxyethyl)amino]-2-methyl-1,2,3,4,4a,5,6,7,8,8a-decahydroisochinolin

Zu einer Lösung von 9,0 g (4aRS,8aRS)-2-Methyl-6-oxo-1,2,3,4,4a, 5,6,7,8,8a-decahydroisochinolin und 6,8 g Aminoacetaldehyd-dimethylacetal in 90 ml Acetonitril werden 31 ml 3N Salzsäure gegeben und 30 Minuten bei Raumtemperatur gerührt. Dann wird im Eisbad abgekühlt und 4,4 g Natriumcyanborhydrid portionsweise eingetragen. Nach 30 Minuten Rühren bei Raumtemperatur und Stehenlassen über Nacht wird eingeengt, mit Eiswasser verdünnt und ein pH-Wert von 10-11 eingestellt. Es wird dreimal mit Essigester ausgeschüttelt. Die vereinigten Essigesterextrakte werden zweimal mit gesättigter Kochsalzlösung gewaschen, getrocknet und eingeengt. Der Rückstand wird durch Chromatographie über eine Aluminiumoxidsäule (basisch) gereinigt.
Ausbeute: 7,35 g (53 % der Theorie),
$R_f$-Wert: 0,25 (Aluminiumoxid; Essigester)

(1) (4aRS, 6SR, 8aRS)-6-[(2,2-Dimethoxyethyl)amino]-2-methyl-1,2,3,4,4a,5,6,7,8,8a-decahydroisochinolin wird als Nebenprodukt von Beispiel V isoliert.
$R_f$-Wert: 0,56 (Aluminiumoxid; Essigester)

Beispiel VI

7-Iod-3-trifluoracetyl-2,3,4,5-tetrahydro-1H-3-benzazepin

2,6 g 7-Amino-3-trifluoracetyl-2,3,4,5-tetrahyäro-1H-3-benzazepin werden mit 50 ml 10%iger Salzsäure kurz unter Rühren erhitzt, dann auf 0°C abgekühlt und mit 695 mg Natriumnitrit in 14 ml Wasser unter weiterer Kühlung bei 0°C tropfenweise versetzt. Danach wird eine Lösung von 1,7 g Kaliumjodid und 1,3 g Jod in 10 ml Wasser bei 0°C zugetropft und über Nacht bei Raumtemperatur gerührt. Anschließend wird abdekantiert und der erhaltene Rückstand in Methylenchlorid gelöst. Die organische Phase wird mit Natriumdisulfit-Lösung und Wasser gewaschen, abgetrennt, getrocknet, filtriert und einrotiert. Das Rohprodukt wird durch Chromatographie über eine Kieselgelsäule mit Cyclohexan/Essigester (4:1) gereinigt. Ausbeute: 2,4 g (65 % der Theorie),
Schmelzpunkt: 80-82°C
$R_f$-Wert: 0,61 (Kieselgel; Cyclohexan/Essigester = 4:1)

Beispiel VII

7-Amino-3-trifluoracetyl-2,3,4,5-tetrahydro-1H-3-benzazepin

17,3 g 7-Nitro-3-trifluoracetyl-2,3,4,5-tetrahyäro-1H-3-benzazepin werden in 200 ml Essigester mit 3 g 10%igem Palladium auf Aktivkohle bei Raumtemperatur und einem Wasserstoffdruck von 50 psi eine Stunde hydriert. Der Katalysator wird abgesaugt und das Filtrat eingedampft. Der Rückstand wird mit tert.Butyl-methylether erhitzt und abgekühlt. Der Niederschlag wird abgesaugt, mit tert.Butyl-methylether gewaschen und getrocknet.
Ausbeute: 12,2 g (79 % der Theorie),
Schmelzpunkt: 102-104°C
$R_f$-Wert: 0,31 (Kieselgel; Cyclohexan/Essigester = 2:1)
Analog Beispiel VII werden folgende Verbindungen erhalten:

(1) 7-Amino-3-tert.butyloxycarbonyl-2,3,4,5-tetrahydro-1H-3-benzazepin Das als Ausgangsmaterial eingesetzte 3-tert.Butyloxycarbonyl-7-nitro-2,3,4,5-tetrahydro-1H-3-benzazepin (Schmelzpunkt: 100-102°C) wird durch Umsetzung von 7-Nitro-2,3,4,5-tetrahydro-1H-3-benzazepin-hydrochlorid mit Pyrokohlensäure-di-tert.butylester in Gegenwart von Natronlauge erhalten. $R_f$-Wert: 0,40 (Kieselgel; Cyclohexan/Essigester = 7:3)

(2) 7-Amino-3-ethyl-2,3,4,5-tetrahydro-1H-3-benzazepin Durchführung mit Raney-Nickel in Methanol.
$R_f$-Wert: 0,58 (Kieselgel; Toluol/Dioxan/Methanol/konz. wäßeriges Ammoniak = 20:50:20:3)

(3) 3-(4-Aminophenyl)-3-phenyl-propionsäure-ethylesterhydrochlorid
Durchführung in Ethanol in Gegenwart von ethanolisher Salzsäure. Das Ausgangsmaterial 3-(4-Nitrophenyl)-3-phenylacrylsäure-ethylester [$R_f$-Wert: 0,36 (Kieselgel; Cyclohexan/Methylenchlorid = 1:1)] wird durch Umsetzung von 4-Nitrobenzophenon mit Phosphonoessigsäure-triethylester erhalten.
$R_f$-Wert: 0,40 (Reversed Phase Kieselgel; Methanol/5%ige wäßerige Kochsalzlösung = 6:4)

Beispiel VIII

7-Nitro-3-trifluoracetyl-2,3,4,5-tetrahydro-1H-3-benzazepin

Zu einer Lösung von 23,3 g 3-Trifluoracetyl-2,3,4,5-tetrahydro-1H-3-benzazepin [$R_f$-Wert: 0,76 (Kieselgel; Cyclohexan/ Essigester = 1:1), hergestellt durch Umsetzung von 2,3,4,5-Tetrahydro-1H-3-benzazepin mit Trifluoressigsäureanhydrid in Gegenwart von N-Ethyl-diisopropylamin] in 60 ml konz. Schwefelsäure wird bei 0 bis 8°C eine Mischung aus 9,7 g Kaliumnitrat und 50 ml konz. Schwefelsäure innerhalb von 1,5 Stunden zugetropft. Es wird auf Raumtemperatur erwärmt, auf 1 l; Eis gegossen und mit Essigester extrahiert. Die vereinigten Essigesterphasen werden mit Wasser und mit gesättigter Kochsalzlösung gewaschen, getrocknet und eingedampft. Der Rückstand wird mit tert. Butyl-methylether erhitzt. Anschließend wird abgekühlt, das Produkt abgesaugt und mit tert.Butyl-methylether gewaschen.
Ausbeute: 16,2 g (59 % der Theorie),
Schmelzpunkt: 116-119°C
$R_f$-Wert: 0,57 (Kieselgel; Cyclohexan/Essigester = 2:1)
Analog Beispiel VIII wird folgende Verbindung erhalten:

(1) 7-Nitro-2,3,4,5-tetrahydro-1H-3-benzazepin-hydrochlorid Durchführung mit Schwefelsäure/Kaliumnitrat und Isolierung als Hydrochlorid
Schmelzpunkt: 235-238°C (Zers.)

| | | | | | | | | |
|---|---|---|---|---|---|---|---|---|
| Ber. | C | 52,52 | H | 5,74 | N | 12,25 | Cl | 15,50 |
| Gef. | | 52,44 | | 5,80 | | 12,07 | | 15,36 |

Beispiel IX

4-[4-[2-(Methoxycarbonyl)ethyl]phenyl]-5-methyl-4H-1,2,4-triazol-3-on

8,1 g 1-Acetyl-4-[4-[2-(methoxycarbonyl)ethyl]phenyl]-semicarbazid werden mit 60 ml 1N Natronlauge 1,5 Stunden auf dem Dampfbad erhitzt. Anschließend wird etwas abgekühlt, filtriert und das Filtrat mit Zitronensäure schwach

angesäuert. Es wird filtriert und das Filtrat mit konzentrierter Salzsäure versetzt. Der Niederschlag wird filtriert, mit Wasser gewaschen und getrocknet. Das Zwischenprodukt wird über Nacht in Methanol mit etwas methanolischer Salzsäure gerührt. Das Reaktionsgemisch wird eingeengt und der Rückstand mit tert.-Butyl-methylether verrieben, abgesaugt und getrocknet. Ausbeute: 4,98 g (65 % der Theorie),
$R_f$-Wert: 0,40 (Kieselgel; Toluol/Dioxan/Ethanol/Eisessig = 90:10:10:6)

| | | | | | | |
|---|---|---|---|---|---|---|
| Ber. | C | 59,76 | H | 5,79 | N | 16,08 |
| Gef. | | 59,54 | | 5,86 | | 16,05 |

Analog Beispiel IX werden folgende Verbindungen erhalten:

(1) 4-[trans-4-[2-(Methoxycarbonyl)ethyl]cyclohexyl]-5-methyl-4H-1,2,4-triazol-3-on Schmelzpunkt: 179-181°C

(2) 4-[trans-4-[2-(Methoxycarbonyl)ethyl]cyclohexyl]-4H-1,2,4-triazol-3-on Schmelzpunkt: 142-144°C

(3) 4-(3-tert.Butyloxycarbonyl-2,3,4,5-tetrahyäro-1H-3-benzazepin-7-yl)-4H-1,2,4-triazol-3-on
Ausgangsmaterial: Verbindung des Beispiels III(12)
Das Zwischenprodukt wird anstatt mit methanolischer Salzsäure mit Pyrokohlensäure-di-tert.butylester umgesetzt.
Schmelzpunkt: 185-186°C
$R_f$-Wert: 0,25 (Kieselgel; Cyclohexan/Essigester = 2:3)

Beispiel X

N-[trans-4-[2-(Methoxycarbonyl)ethyl]cyclohexyl]-ethanolamin

5,0 g N-Benzyl-N-[trans-4-[2-(methoxycarbonyl)ethyl]cyclohexyl]-ethanolamin werden in 150 ml Methanol mit 1,0 g 10%igem Palladium auf Aktivkohle 1 3/4 Stunden bei 50°C und einem Wasserstoffdruck von 50 psi hydriert. Es wird filtriert und eingedampft.
Ausbeute: 3,3 g (92 % der Theorie),
$R_f$-Wert: 0,20 (Kieselgel; Methylenchlorid/Methanol/konz. wäßriges Ammoniak = 9:1:0,4)
Analog Beispiel X werden folgende Verbindungen erhalten:

(1) (trans-4-Aminocyclohexyl)oxyessigsäure-tert.butylester
Das Ausgangsmaterial, [trans-(4-Dibenzylamino)cyclohexyl]-oxyessigsäure-tert.butylester [$R_f$-Wert: 0,51; (Kieselgel; Cyclohexan/Essigester = 4:1)], wird durch Umsetzung von trans-4-(Dibenzylamino)cyclohexanolmit Bromesssigsäure-tert.butylester in Toluol/50%ige Natronlauge in Gegenwart von Tetrabutylammonium-hydrogensulfat erhalten.
$R_f$-Wert: 0,56 (Reversed Phase Kieselgel; Methanol/5%ige wäßrige Kochsalzlösung = 6,4).

(2) N-(3-Hydroxypropyl)-N-[trans-4-[2-(methoxycarbonyl)-ethyl]cyclohexyl]-amin
$R_f$-Wert: 0,10 (Kieselgel; Methylenchlorid/Methanol = 9:1)

(3) [[trans-4-[2-(Methoxycarbonyl)ethyl]cyclohexyl]amino]-essigsäure-methylester-hydrochlorid
Schmelzpunkt: 166-168°C
Hydrierung in Gegenwart von methanolischer Salzsäure.
$R_f$-Wert: 0,69 (Kieselgel; Methylenchlorid/Methanol/konz. wäßeriges Ammoniak = 95:5:1)

Beispiel XI

N-Benzyl-N-[trans-4-[2-(methoxycarbonyl)ethyl] cyclohexyl] ethanolamin

6,2 g N-[trans-4-[2-(Methoxycarbonyl)ethyl]cyclohexyl]-benzylamin-hydrochlorid, 12,8 g N-Ethyl-diisopropylamin und 5,0 g 2-Bromethanol werden 22 Stunden bei 100°C gerührt und dann abgekühlt. Es wird zwischen Essigester und Wasser verteilt, die wäßrige Phase wird mit Essigester ausgeschüttelt und die vereinigten Essigesterphasen mit gesättigter Kochsalzlösung gewaschen. Die Essigesterphase wird eingedampft und der Rückstand durch Chromatographie über eine Kieselgelsäule mit Methylenchlorid/Methanol (9:1) gereinigt.
Ausbeute: 5,1 g (80 % der Theorie),

$R_f$-Wert: 0,67 (Kieselgel; Methylenchlorid/Methanol = 9:1)
Analog Beispiel XI werden folgende Verbindungen erhalten:

(1) N-(3-Hydroxypropyl)-N-[trans-4-[2-(methoxycarbonyl)-ethyl]cyclohexyl]-benzylamin
$R_f$-Wert: 0,70 (Kieselgel; Methylenchlorid/Methanol = 9:1).

(2) N-Benzyl-[[trans-4-[(2-Methoxycarbonyl)ethyl] cyclohexyl]-amino]-essigsäure-methylester
$R_f$-Wert: 0,86 (Kieselgel; Cyclohexan/Essigester = 3:7)

(3) 3-Ethyl-7-nitro-2,3,4,5-tetrahydro-1H-3-benzazepin
$R_f$-Wert: 0,32 (Kieselgel; Methylenchlorid/Methanol = 95:5)

Beispiel XII

N-[trans-4-[2-(Methoxycarbonyl)ethyl]cyclohexyl]-benzylaminhydrochlorid

8,0 g 3-(trans-4-Aminocyclohexyl)propionsäure-methylesterhydrochlorid, 4,3 g Benzaldehyd und 5,0 ml Triethylamin in 150 ml Methanol werden mit 1,0 g Raney-Nickel bei 50°C und einem Wasserstoffdruck von 50 psi 4 Stunden hydriert. Nach dem Abkühlen wird abgesaugt und das Filtrat eingedampft. Der Rückstand wird zwischen Essigester und Wasser verteilt, die wäßrige Phase wird mit Natronlauge auf pH 8-9 gestellt und mit Essigester extrahiert. Die vereinigten Essigesterphasen mit gesättigter Kochsalzlösung gewaschen, getrocknet und einrotiert. Der Rückstand wird in Diethylether suspendiert und mit methanolischer Salzsäure versetzt. Der Niederschlag wird abgesaugt, mit Diethylether gewaschen und getrocknet.
Ausbeute: 7,4 g (66 % der Theorie),
Schmelzpunkt: 170-172°C

| | | | | | | | | |
|---|---|---|---|---|---|---|---|---|
| Ber. | C | 65,47 | H | 8,40 | N | 4,49 | Cl | 11,37 |
| Gef. | | 65,38 | | 8,44 | | 4,46 | | 11,40 |

Beispiel XIII

(trans-4-Aminocyclohexyl)oxyessigsäure-methylester-hydrochlorid

Über eine im Eisbad gekühlte Lösung von 59,4 g (trans-4-Aminocyclohexyl)oxyessigsäure-tert.butylester in 500 ml Methanol wird eine Stunde lang Salzsäuregas geleitet und dann über Nacht bei Raumtemperatur gerührt. Es wird zur Trockene eingeengt, der Rückstand mit Aceton verrieben und der Feststoff abgesaugt und getrocknet.
Ausbeute: 34,3 g (59 % der Theorie),
Schmelzpunkt: 157-160°C.

Beispiel XIV

2-(3-Trifluoracetyl-2,3,4,5-tetrahydro-1H-3-benzazepin-7-yl)-3,4-dihydro-2H,5H-1,2,5-thiadiazol-1,1-dioxid

a) N-(2-Chlorethyl)-N'-(3-trifluoracetyl-2,3,4,5-tetrahydro-1H-3-benzazepin-7-yl)-schwefelsäurediamid

Zu einer Mischung aus 7,6 g 7-Amino-3-trifluoracetyl-2,3,4,5-tetrahydro-1H-3-benzazepin und 7,7 ml N-Ethyl-diisopropylamin in 70 ml Methylenchlorid werden bei -5°C 4,8 g [(2-Chlorethyl)amino]sulfonylchlorid in 30 ml Methylenchlorid zugetropft. Es wird eine Stunde bei 0°C und über Nacht bei Raumtemperatur gerührt. Das Reaktionsgemisch wird mit Methylenchlorid verdünnt und mit Wasser, 1N Salzsäure und nochmals mit Wasser gewaschen. Die organische Phase wird getrocknet, eingeengt und der Rückstand durch Chromatographie über eine Kieselgelsäule mit Cyclohexan/Essigester (1:1) gereinigt.
Ausbeute: 7,8 g (72 % der Theorie),
Schmelzpunkt: 128-130°C
$R_f$-Wert: 0,64 (Kieselgel; Cyclohexan/Essigester = 1:1)

b) 2-(3-Trifluoracetyl-2,3,4,5-tetrahydro-1H-3-benzazepin-7-yl)-3,4-dihydro-2H,5H-1,2,5-thiadiazol-1,1-dioxid

7,8 g der Verbindung des Beispiels XIVa) werden in 20 ml Dimethylformamid gelöst und mit 2,5 g Kalium-tert. butylat versetzt. Nach 3 Stunden Rühren bei Raumtemperatur wird auf 300 ml einer 15%igen wäßeringen Zitronensäurelösung gegeben und dreimal mit Essigester extrahiert. Die vereinigten organischen Phasen werden getrocknet, eingeengt und der Rückstand durch Chromatographie über eine Kieselgelsäule mit
Cyclohexan/Essigester (1:1) gereinigt.
Ausbeute: 2,1 g (30 % % der Theorie),
(Schmelzpunkt: 165-167°C
$R_f$-Wert: 0,30 (Kieselgel; Cyclohexan/Essigester = 1:1)

Beispiel XV

(3-Methyl-2,3,4,5-tetrahydro-1H-3-benzazepin-7-yl)-boronsäure

Zu 7,1 g 7-Jod-3-methyl-2,3,4,5-tetrahydro-1H-3-benzazepin [$R_f$-Wert: 0,85 (Kieselgel; Toluol/Dioxan/Methanol/ konz.wäßriges Ammoniak = 20:50:20:5); hergestellt aus der Verbindung des Beispiels VI durch Umsetzung mit Natronlauge/Methanol und anschließender Umsetzung mit Formalin/Ameisensäure] in 50 ml Diethylether werden bei -70 bis -75°C 17,7 ml 1,6 M n-Butyllithium in Hexan zugetropft. Nach 30 Minuten Rühren bei -70°C werden 5,7 ml Triisopropylborat in 30 ml Diethylether zugetropft. Es wird eine Stunde bei -75°C gerührt, dann auf -50°C erwärmt und 70 ml Wasser zugegeben. Es wird eine Stunde unter Erwärmung auf Raumtemperatur gerührt, dann wird die wäßrige Phase abgetrennt. Die wäßrige Phase wird mit Eisessig auf einen pH-Wert von 8-8,5 eingestellt, das Produkt wird abgesaugt, mit wenig kaltem Wasser gewaschen und bei 60°C getrocknet.
Ausbeute: 3,7 g (73 % der Theorie),
Schmelzpunkt: 153-155°C (sintert ab 148°C)
$R_f$-Wert: 0,80 (Reversed Phase Kieselgel; Methanol/5%ige wäßrige Kochsalzlösung = 6:4)

Beispiel XVI

1-[2-(Ethoxycarbonyl)ethyl]-3-[4-(trifluormethylsulfonyloxy)-phenyl]-imidazolidin-2-on

a) N-(2,2-Diethoxyethyl)-4-benzyloxy-anilin

Hergestellt aus 4-Benzyloxyanilin durch Umsetzung mit Bromacetaldehyd-diethylacetal in Gegenwart von N-Ethyl-diisopropylamin $R_f$-Wert: 0,42 (Kieselgel; Cyclohexan/Essigester = 85:15)

b) N-(4-Benzyloxyphenyl)-N-(2,2-diethoxyethyl)-N'-[2-(ethoxycarbonyl)ethyl]-harnstoff

Hergestellt aus N-(2,2-Diethoxyethyl)-4-benzyloxy-anilin durch Umsetzung mit [2-(Ethoxycarbonyl)ethyl]-isocyanat
$R_f$-Wert: 0,45 (Kieselgel; Cyclohexan/Essigester = 1:1)

c) 1-(4-Benzyloxyphenyl)-3-[2-(ethoxycarbonyl)ethyl]-3H-imidazol-2-on

Hergestellt durch Behandlung von N-(4-Benzyloxyphenyl)-N-(2,2-diethoxyethyl)-N'-[2-(ethoxycarbonyl)ethyl]-harnstoff mit Trifluoressigsäure in Methylenchlorid.
Schmelzpunkt: 66-68°C
$R_f$-Wert: 0,41 (Kieselgel; Cyclohexan/Essigester = 1:1)

d) 1-(4-Hydroxyphenyl)-3-[2-(ethoxycarbonyl)ethyl]-imidazolidin-2-on

Hergestellt aus 1-(4-Benzyloxyphenyl)-3-[2-(ethoxycarbonyl)-ethyl]-3H-imidazol-2-on durch katalytische Hydrierung in Gegenwart von Palladium auf Kohle in Ethanol bei 40°C und einem Wasserstoffdruck von 50 psi.
$R_f$-Wert: 0,20 (Kieselgel; Methylenchlorid/Methanol = 100:2)

e) 1-[2-(Ethoxycarbonyl)ethyl]-3-[4-(trifluormethylsulfonyloxy)-phenyl]-imidazolidin-2-on

Hergestellt aus 1-(4-Hydroxyphenyl)-3-[2-(ethoxycarbonyl)-ethyl]-imidazolidin-2-on durch Umsetzung mit Trifluormethansul fonsäure-anhydrid in Pyridin bei 0°C.
Schmelzpunkt: 81-83°C
$R_f$-Wert: 0,66 (Kieselgel; Methylenchlorid/Methanol = 100:1)

| | | | | | | | | |
|---|---|---|---|---|---|---|---|---|
| Ber. | C | 43,90 | H | 4,18 | N | 6,83 | S | 7,81 |
| Gef. | | 43,92 | | 4,20 | | 6,98 | | 7,91 |

Beispiel 1

1-(1-Aminoisochinolin-6-yl)-3-[4-(2-carboxyethyl)phenyl]-imidazolidin-2-on x 1 Wasser x 1,2 Essigsäure

300 mg 3-[4-(2-Carboxyethyl)phenyl]-1-(1-chlorisochinolin-6-yl)-imidazolidin-2-on, 895 mg Acetamid und 524 mg Kaliumcarbonat werden in einer Reibschale gut vermischt und anschließend unter Stickstoff 3 Stunden unter Rühren auf 200°C erhitzt. Nach dem Abkühlen wird mit 20 ml Wasser versetzt und mit 1N Salzsäure auf einem pH-Wert von 8-9 gestellt. Der Niederschlag wird abgesaugt und mehrmals mit Wasser gewaschen. Nach dem Trocknen wird der Niederschlag in 50 ml Methylenchlorid/Methanol (4:1) suspendiert und 30 Minuten im Ultraschallbad beschallt. Der Niederschlag wird abgesaugt und die Behandlung mit Methylenchlorid/Methanol im Ultraschallbad zweimal wiederholt. Der Filterrückstand wird in einem Gemisch aus 30 ml Methanol und 10 ml Eisessig unter leichtem Erwärmen gerührt. Es wird vom Ungelösten abfiltriert und die Mutterlauge zur Trockene eingeengt. Der Rückstand wird nochmals mit Toluol versetzt und eingeengt. Dann wird bei 60°C im Vakuum getrocknet.
Ausbeute: 70 mg (20 % der Theorie),
Schmelzpunkt: >250°C,
$R_f$-Wert: 0,78 (Reversed Phase Kieselgel; Methanol/5±ige wässrige Kochsalzlösung = 8:4)

| | | | | | | |
|---|---|---|---|---|---|---|
| Ber. | C | 60,25 | H | 5,79 | N | 12,01 |
| Gef. | | 60,20 | | 5,43 | | 11,54 |

Massenspektrum: $M^+$ = 376

Beispiel 2

3-[4-(2-Carboxyethyl)phenyl]-1-(1-chlorisochinolin-6-yl)-imidazolidin-2-on

1 g 1-(1-Chlorisochinolin-6-yl)-3-[4-[2-(methoxycarbonyl)-ethyl]phenyl]-imidazolidin-2-on, 100 ml Dioxan, 80 ml Wasser und 8,5 ml 1N Natronlauge werden 3 Stunden bei Raumtemperatur gerührt. Es werden 9 ml 1N Salzsäure zugegeben, das Gemisch wird eingeengt und der Niederschlag abgesaugt. Das Produkt wird mit Wasser gewaschen und bei 50°C getrocknet.
Ausbeute: 0,78 g (80 % der Theorie),
Schmelzpunkt: 236-240°C (Zers.)
$R_f$-Wert: 0,36 (Kieselgel; Methylenchlorid/Methanol/Essigester = 20:1:1)
Analog Beispiel 2 werden folgende Verbindungen erhalten:

(1) 1-[4-(2-Carboxyethyl)phenyl]-3-(isochinolin-6-yl)-3H-imidazol-2-on
Schmelzpunkt: 305-310°C,
$R_f$-Wert: 0,49 (Kieselgel; Methylenchlorid/Methanol = 9:1)

| | | | | | | |
|---|---|---|---|---|---|---|
| Ber. | C | 70,18 | H | 4,77 | N | 11,69 |
| Gef. | | 69,95 | | 4,93 | | 11,64 |

(2) 1-[4-(2-Carboxyethyl)phenyl]-3-(1,2,3,4-tetrahydroisochinolin-6-yl)-imidazolidin-2-on
$R_f$-Wert: 0,44 (Reversed Phase Kieselgel; Methanol/5±ige wäßrige Kochsalzlösung = 6:4)

| | | | | | | |
|---|---|---|---|---|---|---|
| Ber. | C | 67,54 | H | 6,21 | N | 11,25 |
| Gef. | | 67,58 | | 6,42 | | 11,23 |

(3) 1-[4-(2-Carboxyethyl)phenyl]-3-(2,3,4,5-tetrahydro-1H-3-benzazepin-7-yl)-3H-imidazol-2-on

(4) 2-[trans-4-[2-(Methoxycarbonyl)ethyl]cyclohexyl]-5-(2,3,4,5-tetrahydro-1H-3-benzazepin-7-yl)-3,4-dihydro-2H,5H-1,2,5-thiadiazol-1,1-dioxid
Durchführung in Methanol/Natronlauge; Ausgangsmaterial: Verbindung des Beispiels 18.
$R_f$-Wert: 0,36 (Reversed Phase Kieselgel; Methanol/5%ige wäßrige Kochsalzlösung = 6:4)

(5) 3-[4-(2-Carboxyethyl)phenyl]-1-(2,3,4,5-tetrahydro-1H-3-benzazepin-7-yl)-imidazolidin-2,4-dion

(6) 1-[4-(2-Carboxy-2-(n-butylsulfonylaminol-ethyl]phenyl]-3-(2,3,4,5-tetrahydro-1H-3-benzazepin-7-yl)-imidazolidin-2-on

(7) 1-[4-(2-Carboxy-2-(n-pentylcarbonylamino)-ethyl]phenyl]-3-(2,3,4,5-tetrahydro-1H-3-benzazepin-7-yl)-imidazolidin-2-on

(8) 1-[4-(2-Carboxyethyl)phenyl]-3-(5,6,7,8-tetrahydropyrido[3,4-d]pyrimidin-2-yl)-imidazolidin-2-on

(9) 1-[4-[(Carboxymethyl)sulfonyl]phenyl]-3-(2,3,4,5-tetrahydro-1H-3-benzazepin-7-yl)-imidazolidin-2-on

(10) 1-[(4-Carboxy-1-piperidinyl)carbonylmethyl]-3-(2,3,4,5-tetrahydro-1H-3-benzazepin-7-yl)-imidazolidin-2-on

(11) 1-[2-[(2-Carboxyethyl)aminocarbonyl]ethyl]-3-(2,3,4,5-tetrahydro-1H-3-benzazepin-7-yl)-imidazolidin-2-on

(12) 1-[4-(2-Carboxyethyl)phenyl]-3-(2,3,4,5-tetrahydro-1H-2-benzazepin-7-yl)-imidazolidin-2-on

(13) 1-[4-(2-Carboxyethyl)phenyl]-3-(1,1-dimethyl-1,2,3,4-tetrahydro-isochinolin-6-yl)-imidazolidin-2-on

Beispiel 3

1-(1-Chlorisochinolin-6-yl)-3-[4-[2-(methoxycarbonyl)ethyl] phenyl]-imidazolidin-2-on

2,2 g 1-(Isochinolin-N-oxid-6-yl)-3-[4-[2-(methoxycarbonyl)-ethyl]phenyl]-imidazolidin-2-on und 25 ml Phosphoroxychlorid werden 2 Stunden unter Rückfluß gekocht. Das Phosphoroxychlorid wird anschließend abdestilliert und der Rückstand mit Eis versetzt. Der pH des Gemisches wird mit gesättigter Natriumhydrogencarbonat-Lösung auf einen Wert von 8-9 eingestellt. Es wird mehrmals mit Methylenchlorid extrahiert, die organischen Phasen werden getrocknet, eingeengt und der Rückstand durch Chromatographie über eine Kieselgelsäule mit Methylenchlorid/Methanol (20:1) gereinigt.
Ausbeute: 1,0 g (43 % der Theorie),
$R_f$-Wert: 0,66 (Kieselgel; Methylenchlorid/Methanol = 20:1)

Beispiel 4

1-(Isochinolin-6-yl)-3-[4-[2-(methoxycarbonyl)ethyl]phenyl]-imidazolidin-2-on

Zu einer Lösung von 3,0 g N-(2-Hydroxyethyl)-N'-(isochinolin-6-yl)-N-[4-[2-(methoxycarbonyl)ethyl]phenyl]-harnstoff und 2,0 g Triphenylphosphin in 105 ml Acetonitril werden bei einer Innentemperatur von 42 bis 52°C 1,31 ml Azodicarbonsäure-diethylester in 36 ml Acetonitril zugegeben. Nach 2 Stunden Rühren bei 45°C wird auf -5°C abgekühlt und weitere 2 Stunden gerührt. Der Niederschlag wird abgesaugt und mit wenig kaltem Acetonitril gewaschen.
Ausbeute: 2,6 g (90 % der Theorie),
Schmelzpunkt: 213-215°C,
$R_f$-Wert: 0,60 (Kieselgel; Methylenchlorid/Methanol = 20:1:1)
Analog Beispiel 4 werden folgende Verbindungen erhalten:

(1) 1-(3-tert.Butyloxycarbonyl-2,3,4,5-tetrahyäro-1H-3-benzazepin-7-yl)-3-[4-[2-(methoxycarbonyl)ethyl]phenyl]-imidazolidin-2-on
Schmelzpunkt: 165-167°C
$R_f$-Wert: 0,77 (Kieselgel; Methylenchlorid/Essigester = 9:1)

(2) 1-[trans-4-[2-(Methoxycarbonyl)ethyl]cyclohexyl]-3-(3-trifluoracetyl-2,3,4,5-tetrahydro-1H-3-benzazepin-7-yl)-imidazolidin-2-on
Schmelzpunkt: 250-255°C (Zers.)
$R_f$-Wert: 0,45 (Kieselgel; Cyclohexan/Essigester = 1:1)

(3) 1-[4-[trans-2-(Methoxycarbonyl)ethenyl]phenyl]-3-(3-trifluoracetyl-2,3,4,5-tetrahydro-1H-3-benzazepin-7-yl)-imidazolidin-2-on
Schmelzpunkt: 216-220°C
$R_f$-Wert: 0,38 (Kieselgel; Cyclohexan/Essigester : 1:1)

(4) 1-[trans-4-[2-(Methoxycarbonyl)ethyl]cyclohexyl]-3-(3-trifluoracetyl-2,3,4,5-tetrahydro-1H-3-benzazepin-7-yl)-3,4,5,6-tetrahydro-1H-pyrimidin-2-on
Schmelzpunkt: 125-127°C
$R_f$-Wert: 0,54 (Kieselgel; Cyclohexan/Essigester= 1:2)

Beispiel 5

1-(Isochinolin-6-yl)-3-[4-[2-(methoxycarbonyl)ethyl]phenyl]-3H-imidazol-2-on

7,3 g N-(2,2-Diethoxyethyl)-N'-(isochinolin-6-yl)-N-[4-[2(methoxycarbonyl)ethyl]phenyl]-harnstoff werden in 35 ml Trifluoressigsäure 2 Stunden bei Raumtemperatur gerührt. Danach wird teilweise eingeengt und mit Eiswasser versetzt. Es wird unter Rühren und Kühlen mit Natronlauge alkalisch gestellt, der Niederschlag wird abgesaugt und mit wenig Methanol gewaschen. Das Produkt wird dann aus Methanol umkristallisiert. Ausbeute: 4,45 g (76 % der Theorie), Schmelzpunkt: 158-161°C,
$R_f$-Wert: 0,29 (Kieselgel; Methylenchlorid/Methanol = 95:5)

| | | | | | | |
|---|---|---|---|---|---|---|
| Ber. | C | 70,76 | H | 5,13 | N | 11,25 |
| Gef. | | 70,52 | | 5,08 | | 11,35 |

Analog Beispiel 5 werden folgende Verbindungen erhalten:

(1) 1-[trans-4-[2-(Methoxycarbonyl)ethyl]cyclohexyl]-3-(3-trifluoracetyl-2,3,4,5-tetrahydro-1H-3-benzazepin-7-yl) 3H-imidazol-2-on
Durchführung durch trockenes Erhitzen auf 160-170°C.
Schmelzpunkt: 121-124°C,
$R_f$-Wert: 0,35 (Kieselgel; Cyclohexan/Essigester = 1:1).

(2) 1-[trans-4-[[(Methoxycarbonyl)methyl]oxy]cyclohexyl]-3-(3-trifluoracetyl-2,3,4,5-tetrahydro-1H-3-benzazepin-7-yl)-3H-imidazol-2-on
Schmelzpunkt: 148-149°C
$R_f$-Wert: 0,28 (Kieselgel; Essigester/Cyclohexan = 2:1)

(3) 1-[4-[2-(Ethoxycarbonyl)-1-propyl]phenyl]-3-(3-trifluoracetyl-2,3,4,5-tetrahydro-1H-3-benzazepin-7-yl)-3H-imidazol-2-on
$R_f$-Wert: 0,32 (Kieselgel; Cyclohexan/Essigester = 2:1)

(4) 1-[trans-4-[2-(Methoxycarbonyl)ethyl]cyclohexyl]-3-(3-ethyl-2,3,4,5-tetrahydro-1H-3-benzazepin-7-yl)-3H-imidazol-2-on
$R_f$-Wert: 0,30 (Reversed Phase Kieselgel; Methanol/5%ige wäßrige Kochsalzlösung = 6:4)

(5) 1-[trans-4-[2-(Methoxycarbonyl)ethyl]cyclohexyl]-3-(7-ethyl-6,7,8,9-tetrahydro-5H-pyrazino[2,3-d]azepin-2-yl)-3H-imidazol-2-on
Schmelzpunkt: 135-136°C

| | | | | | | |
|---|---|---|---|---|---|---|
| Ber. | C | 64,61 | H | 7,78 | N | 16,38 |
| Gef. | | 64,82 | | 7,84 | | 16,27 |

(6) 1-[4-[2-(Ethoxycarbonyl)-1-phenyl-ethyl]phenyl]-3-(3-trifluoracetyl-2,3,4,5-tetrahydro-1H-3-benzazepin-7-yl)-3H-imidazol-2-on
$R_f$-Wert: 0,24 (Kieselgel; Cyclohexan/Essigester = 7:3)

(7) 1-[trans-4-[2-(Methoxycarbonyl)ethyl]cyclohexyl]-3-(7-benzyl-6,7,8,9-tetrahydro-5H-pyrimido[4,5-d]azepin-2-yl)-3H-imidazol-2-on
Schmelzpunkt: 131-134°C
$R_f$-Wert: 0,38 (Kieselgel; Methylenchlorid/Methanol = 20:1)

Beispiel 6

1-[4-[2-(Methoxycarbonyl)ethyl]phenyl]-3-(1,2,3,4-tetrahydroisochinolin-6-yl)-imidazolidin-2-on-acetat

2,0 g 1-(Isochinolin-6-yl)-3-[4-[2-(methoxycarbonyl)ethyl]-phenyl]-3H-imidazol-2-on in 50 ml Eisessig werden mit 0,5 g Platinoxid bei Raumtemperatur und einem Wasserstoffdruck von 50 psi 40 Minuten hydriert. Es wird vom Katalysator abfiltriert, eingeengt und der Rückstand aus Methanol umkristallisiert.
Ausbeute: 1,7 g (72 % der Theorie),
Schmelzpunkt: 170-173°C (Zers.)

| | | | | | | |
|---|---|---|---|---|---|---|
| Ber. | C | 65,59 | H | 6,65 | N | 9,56 |
| Gef. | | 65,90 | | 6,78 | | 9,69 |

$R_f$-Wert: 0,30 (Kieselgel; Toluol/Dioxan/Methanol/konz. wäßriges Ammoniak = 20:50:20:5)
Massenspektrum: $M^+$ = 379

Analog Beispiel 6 werden folgende Verbindungen erhalten:

(1) 1-[trans-4-[2-(Methoxycarbonyl)ethyl]cyclohexyl]-3-(1,2,3,4-tetrahydro-isochinolin-6-yl)-imidazolidin-2-on-acetat

(2) 1-[trans-4-(2-carboxyethyl)cyclohexyl]-3-(3-methyl-2,3,4,5,5a,6,7,8,9,9a-dekadydro-1H-3-benzazepin-7-yl)-imidazolidin-2-on x 1,35 HCl x 1,9 $H_2O$
Durchführung in Dioxan/Wasser mit Platin-Rhodium-Katalysator.
Als Ausgangsmaterial wird die Verbindung des Beispiels 19 eingesetzt.
Massenspektrum: $M^+$ = 405

| | | | | | | | | |
|---|---|---|---|---|---|---|---|---|
| Ber | C | 56,49 | H | 9,10 | N | 8,59 | Cl | 9,79 |
| Gef. | | 56,63 | | 8,81 | | 8,60 | | 9,92 |

(3) 1-[trans-4-(2-Carboxyethyl)cyclohexyl]-3-(2,3,4,5,5a,6,7,8,9,9a-dekahydro-1H-3-benzazepin-7-yl)-imidazolidin-2-on x 1,05 HCl x 0,9 $H_2O$
Durchführung in Dioxan/Wasser mit Platin-Rhodium-Katalysator.
Als Ausgangsmaterial wird die Verbindung des Beispiels 14(1) eingesetzt.
Massenspektrum: $M^+$ = 391

| | | | | | | | | |
|---|---|---|---|---|---|---|---|---|
| Ber. | C | 59,43 | H | 9,03 | N | 9,45 | Cl | 8,05 |
| Gef. | | 59,49 | | 8,83 | | 9,35 | | 8,30 |

Beispiel 7

1-[4-[2-(Isobutyloxycarbonyl)ethyl]phenyl]-3-(1,2,3,4-tetrahydroisochinolin-6-yl)-imidazolidin-2-on-hydrochlorid x 1 Wasser

Über eine Suspension von 150 mg 1-[4-(2-Carboxyethyl)phenyl]-3-(1,2,3,4-tetrahydro-isochinolin-6-yl)-imidazolidin-2-on in 4,6 ml Isobutanol wird unter Rühren 30 Minuten lang Salzsäure geleitet. Es wird über Nacht bei Raumtemperatur gerührt, mit Aceton versetzt und der Niederschlag abgesaugt. Das Produkt wird in Aceton suspendiert, abgesaugt, mit Aceton und Diethylether gewaschen und getrocknet.
Ausbeute: 140 mg (77 % der Theorie),

| | | | | | | | | |
|---|---|---|---|---|---|---|---|---|
| Ber. | C | 65,56 | H | 7,04 | N | 9,18 | Cl | 7,74 |
| Gef. | | 65,38 | | 7,03 | | 9,47 | | 7,92 |

$R_f$-Wert: 0,19 (Reversed Phase Kieselgel; Methanol/5%ige Kochsalzlösung = 6:4)
Massenspektrum: $M^+$ = 421
Analog Beispiel 7 werden folgende Verbindungen erhalten:

(1) 1-[4-[2-(Isopropyloxycarbonyl)ethyl]phenyl]-3-(2,3,4,5-tetrahydro-1H-3-benzazepin-7-yl)-imidazolidin-2-on-hydrochlorid

| | | | | | | | | |
|---|---|---|---|---|---|---|---|---|
| Ber. | C | 63,08 | H | 7,20 | N | 8,83 | Cl | 7,45 |
| Gef. | | 62,79 | | 7,14 | | 8,83 | | 7,80 |

$R_f$-Wert: 0,21 (Reversed Phase Kieselgel; Methanol/5%ige Kochsalzlösung = 6:4)
Massenspektrum: $M^+$ = 421

(2) 1-[4-[2-(Cyclohexyloxycarbonyl)ethyl]phenyl]-3-(2,3,4,5-tetrahydro-1H-3-benzazepin-7-yl)-imidazolidin-2-on-hydrochlorid

(3) 1-[4-[2-(Methoxycarbonyl)ethyl]phenyl]-3-(3-methyl-2,3,4,5-tetrahydro-1H-3-benzazepin-7-yl)-imidazolidin-2-on-hydrochlorid x 0,75 $H_2O$
Schmelzpunkt: 248-250°C,

| | | | | | | | | |
|---|---|---|---|---|---|---|---|---|
| Ber. | C | 63,01 | H | 6,94 | N | 9,19 | Cl | 7,75 |
| Gef. | | 63,09 | | 6,98 | | 9,22 | | 7,88 |

$R_f$-Wert: 0,28 (Reversed Phase Kieselgel; Methanol/5%ige wäßrige Kochsalzlösung = 6:4)

(4) 1-[4-[2-(Ethoxycarbonyl)ethyl]phenyl]-3-(3-methyl-2,3,4,5-tetrahydro-1H-3-benzazepin-7-yl)-imidazolidin-2-on-hydrochlorid
$R_f$-Wert: 0,22 (Reversed Phase Kieselgel; Methanol/5%ige wäßrige Kochsalzlösung = 6:4)

(5) 1-[4-[2-(Isopropyloxycarbonyl)ethyl]phenyl]-3-(3-methyl-2,3,4,5-tetrahydro-1H-3-benzazepin-7-yl)-imidazolidin-2-on-hydrochlorid
$R_f$-Wert: 0,19 (Reversed Phase Kieselgel; Methanol/5%ige wäßrige Kochsalzlösung = 6:4)

(6) 1-[trans-4-[2-(Isopropyloxycarbonyl)ethyl]cyclohexyl]-3-(3-methyl-2,3,4,5-tetrahydro-1H-3-benzazepin-7-yl)-imidazolidin-2-on-hydrochlorid
Verwendung von Thionylchlorid und Salzsäuregas.
Schmelzpunkt: >250°C,
$R_f$-Wert: 0,17 (Reversed Phase Kieselgel; Methanol/5%ige wäßrige Kochsalzlösung = 6:4)

| | | | | | | | | |
|---|---|---|---|---|---|---|---|---|
| Ber. | C | 65,32 | H | 8,43 | N | 8,79 | Cl | 7,42 |
| Gef. | | 65,10 | | 8,41 | | 8,91 | | 7,66 |

(7) 1-[trans-4-[2-(Ethoxycarbonyl)ethyl]cyclohexyl]-3-(3-methyl-2,3,4,5-tetrahydro-1H-3-benzazepin-7-yl)-imidazolidin-2-on-hydrochlorid
Verwendung von Thionylchlorid und Salzsäuregas.
Schmelzpunkt: >250°C,
$R_f$-Wert: 0,24 (Reversed Phase Kieselgel; Methanol/5%ige wäßrige Kochsalzlösung = 6:4)

| | | | | | | | | |
|---|---|---|---|---|---|---|---|---|
| Ber. | C | 64,71 | H | 8,25 | N | 9,09 | Cl | 7,64 |
| Gef. | | 64,09 | | 8,22 | | 9,08 | | 7,72 |

(8) 1-[trans-4-[2-(Methoxycarbonyl)ethyl]cyclohexyl]-3-(3-methyl-2,3,4,5-tetrahydro-1H-3-benzazepin-7-yl)-imidazolidin-2-on-hydrochlorid
Verwendung von Thionylchlorid und Salzsäuregas.
Schmelzpunkt: >250°C,
$R_f$-Wert: 0,29 (Reversed Phase Kieselgel; Methanol/5%ige wäßrige Kochsalzlösung = 6:4)

| | | | | | | | | |
|---|---|---|---|---|---|---|---|---|
| Ber. | C | 64,05 | H | 8,04 | N | 9,34 | Cl | 7,88 |
| Gef. | | 64,10 | | 7,97 | | 9,52 | | 8,11 |

(9) 1-[trans-4-[2-(Isopropyloxycarbonyl)ethyl]cyclohexyl]-3-(2,3,4,5-tetrahydro-1H-3-benzazepin-7-yl)-imidazolidin-2-on-hydrochlorid
Verwendung von Thionylchlorid und Salzsäuregas.
$R_f$-Wert: 0,20 (Reversed Phase Kieselgel; Methanol/5%ige wäßrige Kochsalzlösung = 6:4)

(10) 1-[trans-4-[2-(Ethoxycarbonyl)ethyl]cyclohexyl]-3-(2,3,4,5-tetrahydro-1H-3-benzazepin-7-yl)-imidazolidin-2-on-hydrochlorid
Verwendung von Thionylchlorid und Salzsäuregas.
$R_f$-Wert: 0,23 (Reversed Phase Kieselgel; Methanol/5%ige wäßrige Kochsalzlösung = 6:4)

| | | | | | | | | |
|---|---|---|---|---|---|---|---|---|
| Ber. | C | 64,05 | H | 8,06 | N | 9,34 | Cl | 7,88 |
| Gef. | | 63,77 | | 8,23 | | 9,07 | | 7,91 |

(11) 1-[trans-4-[2-(Methoxycarbonyl)ethyl]cyclohexyl]-3-(2,3,4,5-tetrahydro-1H-3-benzazepin-7-yl)-imidazolidin-2-on-hydrochlorid
Verwendung von Thionylchlorid und Salzsäuregas.
$R_f$-Wert: 0,26 (Reversed Phase Kieselgel; Methanol/5%ige wäßrige Kochsalzlösung = 6:4)

| | | | | | | | | |
|---|---|---|---|---|---|---|---|---|
| Ber. | C | 63,36 | H | 7,86 | N | 9,64 | Cl | 8,13 |
| Gef. | | 63,34 | | 7,88 | | 9,73 | | 8,11 |

Beispiel 8

1-[4-(2-carboxyethyl)phenyl]-3-(2-methyl-1,2,3,4-tetrahydro-isochinolin-6-yl)-imidazolidin-2-on x 0,2 Wasser

350 mg 1-[4-[2-(Methoxycarbonyl)ethyl]phenyl]-3-(1,2,3,4-tetrahydroisochinolin-6-yl)-imidazolidin-2-on-acetat, 0,7 ml Wasser, 0,15 ml Ameisensäure und 0,7 ml 37%ige wäßrige Formaldehydlösung werden eine Stunde bei 65°C gerührt. Es wird Toluol zugesetzt und eingeengt. Es wird mit Toluol versetzt und erneut eingedampft. Der Rückstand wird mit 2 ml Tetrahydrofuran, 1 ml Wasser und 0,8 ml 4N Natronlauge 2 1/2 Tage bei Raumtemperatur gerührt. Es werden 220 mg Ammoniumchlorid in 1 ml Wasser zugegeben und 2 Stunden gerührt. Das Reaktionsgemisch wird teilweise einrotiert, mit Eis gekühlt und der Feststoff abgesaugt. Der Feststoff wird mit Wasser, Aceton und Diethylether gewaschen und anschließend getrocknet.
Ausbeute: 220 ml (72 % der Theorie),
Schmelzpunkt: 245-248°C,

| | | | | | | |
|---|---|---|---|---|---|---|
| Ber. | C | 68,98 | H | 6,68 | N | 10,97 |
| Gef. | | 68,91 | | 6,69 | | 10,92 |

$R_f$-Wert: 0,41 (Reversed Phase Kieselgel; Methanol/5%ige wäßrige Kochsalzlösung = 6:4)
Massenspektrum: $M^+$ = 379

Analog Beispiel 8 wird folgende Verbindung erhalten:

(1) 1-[4-(2-Carboxyethyl)phenyl]-3-(3-methyl-2,3,4,5-tetrahydro-1H-3-benzazepin-7-yl)-imidazolidin-2-on-hydrochlorid x 1,5 Wasser
Die abschließende Esterspaltung wird statt mit Natronlauge mit Eisessig/Salzsäure durchgeführt.

| | | | | | | | | |
|---|---|---|---|---|---|---|---|---|
| Ber. | C | 60,45 | H | 6,84 | N | 9,20 | Cl | 7,76 |
| Gef. | | 60,45 | | 6,86 | | 9,18 | | 8,09 |

$R_f$-Wert: 0,31 (Kieselgel; Methylenchlorid/Methanol/konz. wäßriges Ammoniak = 4:1:0,2)
Massenspektrum: $M^+$ = 393

Beispiel 9

1-[4-(2-Carboxyethyl)phenyl]-3-(2,3,4,5-tetrahydro-1H-3-benzazepin-7-yl)-imidazolidin-2-on

800 mg 1-[4-[2-(Methoxycarbonyl)ethyl]phenyl]-3-(2,3,4,5-tetrahydro-1H-3-benzazepin-7-yl)-imidazolidin-2-on-hydrochlorid werden mit 10 ml halbkonzentrierter Salzsäure und 10 ml Eisessig gerührt. Nach 2 Stunden werden nochmals 5 ml halbkonzentrierte Salzsäure und 5 ml Eisessig zugegeben und über Nacht bei Raumtemperatur gerührt. Das Reaktionsgemisch wird eingedampft, der Rückstand in 10 ml Wasser suspendiert. Es wird mit 2N Natronlauge ein pH-Wert von 6 eingestellt, der Feststoff wird abgesaugt, mit Eiswasser gewaschen und dann getrocknet.
Ausbeute: 560 mg (79 % der Theorie),
$R_f$-Wert: 0,40 (Reversed Phase Kieselgel; Methanol/5%ige wäßrige Kochsalzlösung = 6:4)
Massenspektrum: $M^+$ = 379

Analog Beispiel 9 werden folgende Verbindungen erhalten:

(1) 1-[4-(2-Carboxyethyl)phenyl]-3-(4aRS,6RS,8aRS)-1,2,3, 4,4a,5,6,7,8,8a-decahydroisochinolin-6-yl)-imidazolidin-2-on-hydrochlorid
Isolierung als Hydrochlorid
$R_f$-Wert: 0,41 (Reversed Phase Kieselgel; Methanol/5%ige wäßrige Kochsalzlösung = 6:4)
Massenspektrum: $M^+$ = 371

(2) 1-[4-(2-Carboxyethyl)phenyl]-3-(3-ethyl-2,3,4,5-tetrahydro-1H-3-benzazepin-7-yl)-imidazolidin-2-on-hydrochlorid
$R_f$-Wert: 0,36 (Reversed Phase Kieselgel; Methanol/5%ige wäßrige Kochzsalzlösung = 6:4)
Massenspektrum: $M^+$ = 407

(3) 1-[4-(2-Carboxyethyl)phenyl]-3-(3-isopropyl-2,3,4,5-tetrahydro-1H-3-benzazepin-7-yl)-imidazolidin-2-on-hydrochlorid

(4) 1-(2-carboxyethyl)-3-[4-(isochinolin-6-yl)phenyl]-imidazolidin-2-on-hydrochlorid

(5) 1-[4-(2-Carboxyethyl)phenyl]-3-(4,4-dimethyl-1,2,3,4-tetrahydroisochinolin-6-yl)-imidazolidin-2-on-hydrochlorid

(6) 1-[4-(2-Carboxyethyl)phenyl]-3-(3-butyl-2,3,4,5-tetrahydro-1H-3-benzazepin-7-yl)-imidazolidin-2-on-hydrochlorid
$R_f$-Wert: 0,33 (Reversed Phase Kieselgel; Methanol/5%ige wäßrige Kochsalzlösung = 6:4)
Massenspektrum: $M^+$ = 435

(7) 1-[4-(2-Carboxyethyl)phenyl]-3-(1-amino-3,4-dihydro-isochinolin-6-yl)-imidazolidin-2-on-hydrochlorid

(8) 1-[4-(2-Carboxyethyl)phenyl]-3-(2-methyl-3,4-dihydrochinazolin-7-yl)-imidazolidin-2-on-hydrochlorid

(9) 1-[4-(2-Carboxyethyl)phenyl]-3-(4,4-dimethyl-3,4-dihydrochinazolin-7-yl)-imidazolidin-2-on-hydrochlorid

(10) 3-[trans-4-(2-Carboxyethyl)cyclohexyl]-1-(2,3,4,5-tetrahydro-1H-3-benzazepin-7-yl)-hydantoin-hydrochlorid
Schmelzpunkt: >250°C
$R_f$-Wert: 0,36 (Reversed Phase Kieselgel; Methanol/5%ige wäßrige Kochsalzlösung = 6:4).

| | | | | | | | | |
|---|---|---|---|---|---|---|---|---|
| Ber. | C | 60,61 | H | 6,94 | N | 9,64 | Cl | 8,13 |
| Gef. | | 60,45 | | 6,90 | | 9,61 | | 8,28 |

(11) 1-[trans-4-(2-Carboxyethyl)cyclohexyl]-3-(7-ethyl-6,7,8,9-tetrahydro-5H-pyridazino[2,3-d]azepin-2-yl)-imidazolidin-2-on x 2 HCl x 0,9 $H_2O$
Schmelzpunkt: 283-286°C
$R_f$-Wert: 0,46 (Reversed Phase Kieselgel; Methanol/5%ige wäß-rige Kochsalzlösung = 6:4).

| | | | | | | | | |
|---|---|---|---|---|---|---|---|---|
| Ber. | C | 52,36 | H | 7,35 | N | 13,88 | Cl | 14,05 |
| Gef. | | 52,70 | | 7,44 | | 13,86 | | 14,01 |

(12) 1-[trans-4-(2-Carboxyethyl)cyclohexyl]-3-(3-ethyl-2,3,4,5-tetrahydro-1H-3-benzazepin-7-yl)-3H-imidazol-2-on-hydrochlorid x 0,5 $H_2O$
$R_f$-Wert: 0,38 (Reversed Phase Kieselgel; Methanol/5%ige wäßrige Kochsalzlösung = 6:4)

| | | | | | | | | |
|---|---|---|---|---|---|---|---|---|
| Ber. | C | 63,08 | H | 7,72 | N | 9,19 | Cl | 7,76 |
| Gef. | | 63,26 | | 7,69 | | 9,28 | | 7,64 |

(13) 1-[trans-4-(2-Carboxyethyl)cyclohexyl]-3-(2,3,4,5-tetrahydro-1H-3-benzazepin-7-yl)-hydantoin-hydrochlorid
$R_f$-Wert: 0,16 (Kieselgel; Methylenchlorid/Methanol/konz. wäßriges Ammoniak = 80:20:2)

| | | | | | | | | |
|---|---|---|---|---|---|---|---|---|
| Ber. | C | 60,61 | H | 6,94 | N | 9,64 | Cl | 8,13 |
| Gef. | | 60,34 | | 6,94 | | 9,58 | | 8,27 |

(14) 1-[trans-4-(2-Carboxyethyl)cyclohexyl]-3-(2,3,4,5-tetrahydro-1H-3-benzazepin-7-yl-3,4,5,6-tetrahydro-1H-pyrimidin-2-on x 1,65 HCl x 0,7 $H_2O$
Schmelzpunkt: 230-235°C
$R_f$-Wert: 0,40 (Reversed Phase Kieselgel/Methanol/5%ige wäßrige Kochsalzlösung = 6:4)

| | | | | | | | | |
|---|---|---|---|---|---|---|---|---|
| Ber. | C | 58,27 | H | 7,71 | N | 8,86 | Cl | 12,33 |
| Gef. | | 58,04 | | 7,70 | | 8,82 | | 12,43 |

(15) 1-(2-Carboxyethyl)-3-[4-(3-methyl-2,3,4,5-tetrahydro-1H-3-benzazepin-7-yl)phenyl]-imidazolidin-2-on-hydrochlorid Schmelzpunkt: >250°C
$R_f$-Wert: 0,20 (Reversed Phase Kieselgel/Methanol/5%ige wäßrige Kochsalzlösung = 6:4)

(16) 1-[trans-4-(2-Carboxyethyl)cyclohexyl]-3-(6,7,8,9-tetrahydro-5H-pyrimido[4,5-d]azepin-2-yl)-imidazolidin-2-on-hydrochlorid
$R_f$-Wert: 0,60 (Reversed Phase Kieselgel/Methanol/5%ige wäßrige Kochsalzlösung = 6:4)

(17) 1-[trans-4-(2-Carboxyethyl)cyclohexyl]-3-(6,7,8,9-tetrahydro-5H-pyrazino[2,3-d]azepin-2-yl)-imidazolidin-2-on-hydrochlorid

(18) 1-[trans-4-(2-Carboxymethyl)cyclohexyl]-3-(6,7,8,9-tetrahydro-5H-pyrido[2,3-d]azepin-2-yl)-imidazolidin-2-on-hydrochlorid

(19) 1-[trans-4-(2-Carboxyethyl)cyclohexyl]-3-(1,2,3,4-tetrahydro-isochinolin-6-yl)-imidazolidin-2-on-hydrochlorid

(20) 1-[trans-4-(2-Carboxyethyl)cyclohexyl]-3-(2,3-dimethyl-3,4-dihydro-chinazolin-7-yl)-imidazolidin-2-on-hydrochlorid

(21) 1-[trans-4-(2-Carboxyethyl)cyclohexyl]-3-(3-ethyl-2,3,4,5-tetrahydro-1H-3-benzazepin-7-yl)-imidazolidin-2-on-hydrochlorid
Schmelzpunkt: >270°C
$R_f$-Wert: 0,35 (Reversed Phase Kieselgel/Methanol/5%ige wäßrige Kochsalzlösung = 6:4)

(22) 1-[trans-4-(2-Carboxyethyl)cyclohexyl]-3-[3-propyl-2,3,4,5-tetrahydro-1H-3-benzazepin-7-yl)-imidazolidin-2-on-x 1,07 HCl x 1 $H_2O$
Schmelzpunkt: >250°C
$R_f$-Wert: 0,32 (Reversed Phase Kieselgel/Methanol/5%ige wäßrige Kochsalzlösung = 6:4)

| | | | | | | | | |
|---|---|---|---|---|---|---|---|---|
| Ber. | C | 61,96 | H | 8,33 | N | 8,67 | Cl | 7,83 |
| Gef. | | 62,10 | | 8,14 | | 8,77 | | 7,94 |

(23) 1-[trans-4-(2-Carboxyethyl)cyclohexyl]-3-(3-allyl-2,3,4,5-tetrahydro-1H-3-benzazepin-7-yl)-imidazolidin-2-on-x 1,1 HCl x 1,5 $H_2O$
Schmelzpunkt: >250°C
$R_f$-Wert: 0,30 (Reversed Phase Kieselgel/Methanol/5%ige wäßrige Kochsalzlösung = 6:4)

| | | | | | | | | |
|---|---|---|---|---|---|---|---|---|
| Ber. | C | 60,94 | H | 8,00 | N | 8,53 | Cl | 7,91 |
| Gef. | | 61,32 | | 7,63 | | 8,47 | | 7,82 |

(24) 1-[trans-4-(2-Carboxyethyl)cyclohexyl]-3-(3-isobutyl-2,3,4,5-tetrahydro-1H-3-benzazepin-7-yl)-imidazolidin-2-on-hydrochlorid

(25) 1-[trans-4-(2-Carboxyethyl)cyclohexyl]-3-[3-(2-hydroxyethyl)-2,3,4,5-tetrahydro-1H-3-benzazepin-7-yl]-imidazolidin-2-on-hydrochlorid
Schmelzpunkt: 257-260°C
$R_f$-Wert: 0,46 (Reversed Phase Kieselgel/Methanol/5%ige wäßrige Kochsalzlösung = 6:4)

(26) 1-[trans-4-(2-Carboxyethyl)cyclohexyl]-3-[3-(carboxymethyl)-2,3,4,5-tetrahydro-1H-3-benzazepin-7-yl]-imidazolidin-2-on-hydrochlorid
Schmelzpunkt: >280°C
$R_f$-Wert: 0,39 (Reversed Phase Kieselgel/Methanol/5%ige wäßrige Kochsalzlösung = 6:4)

(27) 1-[trans-4-(2-Carboxyethyl)cyclohexyl]-3-(3-benzyl-2,3,4,5-tetrahydro-1H-3-benzazepin-7-yl)-imidazolidin-2-on-hydrochlorid
Schmelzpunkt: >250°C
$R_f$-Wert: 0,21 (Reversed Phase Kieselgel/Methanol/5%ige wäßrige Kochsalzlösung = 6:4)

(28) 1-[trans-4-(2-Carboxyethyl)cyclohexyl]-3-[3-(cyclopropylmethyl)-2,3,4,5-tetrahydro-1H-3-benzazepin-7-yl]-imidazolidin-2-on-hydrochlorid
Schmelzpunkt: >280°C
$R_f$-Wert: 0,31 (Reversed Phase Kieselgel/Methanol/5%ige wäßrige Kochsalzlösung = 6:4)

(29) 1-[trans-4-(2-Carboxyethyl)cyclohexyl]-3-(3-cyclopropyl-2,3,4,5-tetrahydro-1H-3-benzazepin-7-yl]-imidazolidin-2-on-hydrochlorid

(30) 1-[trans-4-(2-Carboxyethyl)cyclohexyl]-3-[3-(cyclohexylmethyl)-2,3,4,5-tetrahydro-1H-3-benzazepin-7-yl]-imidazolidin-2-on-hydrochlorid

(31) 1-[trans-4-(2-Carboxyethyl)cyclohexyl]-3-(3-cyclohexyl-2,3,4,5-tetrahydro-1H-3-benzazepin-7-yl)-imidazolidin-2-on-hydrochlorid

Beispiel 10

1-[4-[2-(Methoxycarbonyl)ethyl]phenyl]-3-(2,3,4,5-tetrahydro-1H-3-benzazepin-7-yl)-imidazolidin-2-on-hydrochlorid

2,3 g 1-(3-tert.Butyloxycarbonyl-2,3,4,5-tetrahydro-1H-3-benzazepin-7-yl)-3-[4-[2-(methoxycarbonyl)ethyl]phenyl]-imidazolidin-2-on werden in 30 ml methanolischer Salzsäure 2 Stunden bei Raumtemperatur gerührt. Das Reaktionsgemisch wird eingedampft, der Rückstand mit Eiswasser verrührt, abgesaugt, mit wenig Methanol und tert.Butylmethylether gewaschen und dann getrocknet.

Ausbeute: 1,8 g (90 % der Theorie),

| | | | | | | | | |
|---|---|---|---|---|---|---|---|---|
| Ber. | C | 64,25 | H | 6,56 | N | 9,77 | Cl | 8,25 |
| Gef. | | 64,08 | | 6,60 | | 9,92 | | 8,51 |

$R_f$-Wert: 0,26 (Reversed Phase Kieselgel; Methanol/5%ige wäßrige Kochsalzlösung = 6:4)
Massenspektrum: $M^+$ = 393

Beispiel 11

1-[(4aRS,6RS,8aRS)-1,2,3,4,4a,5,6,7,8,8a-Decahydroisochinolin 6-yl]-3-[4-[2-(methoxycarbonyl)ethyl]phenyl]-imidazolidin-2-on-hydrochlorid

Zu 216 mg 1-[4-[2-(Methoxycarbonyl)ethyl]phenyl]-3-[(4aRS,6RS,8aRS)-2-methyl-1,2,3,4,4a,5,6,7,8,8a-decahydroisochinolin-6-yl]-imidazolidin-2-on und 185 mg 1,8-Bis-(dimethylamino)-naphthalin in 5 ml 1,2-Dichlorethan werden 0,09 ml Chlorameisensäure-1-chlorethylester gegeben, 20 Minuten bei Raumtemperatur gerührt und dann 2,5 Stunden unter Rückfluß
erhitzt. Das Reaktionsgemisch wird eingeengt, der Rückstand mit 10 ml Methanol versetzt und 3 Stunden unter Rückluß erhitzt. Das Reaktionsgemisch wird mit methanolischer Salzsäure sauer gestellt, eingeengt und durch Chromatographie über eine Kieselgelsäule mit Methylenchlorid/Methanol (92:8) gereinigt.
Ausbeute: 200 mg (87 % der Theorie),
$R_f$-Wert: 0,35 (Reversed Phase Kieselgel; Methanol/5%ige wäßrige Kochsalzlösung = 6:4)

Beispiel 12

1-[4-[2-(Methoxycarbonyl) ethyl]phenyl]-3-[(4aRS,6RS,8aRS)-2-methyl-1,2,3,4,4a,5,6,7,8,8a-decahydroisochinolin-6-yl]-imidazolidin-2-on

950 mg 1-[4-[2-(Methoxycarbonyl)ethyl]phenyl]-3-[(4aRS,6RS, 8aRS)-2-methyl-1,2,3,4,4a,5,6,7,8,8a-decahydroisochinolin-6-yl]-3H-imidazol-2-on in 50 ml Essigester werden in Gegenwart von Palladium auf Aktivkohle 5 Stunden unter einem Wasserstoffdruck von 50 psi bei Raumtemperatur und anschließend 5 Stunden bei 50°C hydriert. Der Katalysator wird abfiltriert und das Filtrat eingeengt. Der Rückstand wird mit tert.Butyl-methylether kurz erwärmt, dann unter Rühren abgekühlt. Der Feststoff wird abgesaugt, mit tert.Butylmethylether gewaschen und getrocknet.
Ausbeute: 300 mg (31 % der Theorie),
Schmelzpunkt: 155-157°C
$R_f$-Wert: 0,29 (Reversed Phase Kieselgel; Methanol/5%ige wäßrige Kochsalzlösung = 6:4)

Analog Beispiel 12 werden folgende Verbindungen erhalten:

(1) 1-[trans-4-[2-(Methoxycarbonyl)ethyl]cyclohexyl]-3-(3-trifluoracetyl-2,3,4,5-tetrahydro-1H-3-benzazepin-7-yl)-imidazolidin-2-on
Schmelzpunkt: 165-168°C
$R_f$-Wert: 0,44 (Kieselgel; Cyclohexan/Essigester = 1:1)

(2) 1-trans-4-(-[[(Methoxycarbonyl)methyl]oxy]cyclohexyl]-3-(3-trifluoracetyl-2,3,4,5-tetrahydro-1H-3-benzazepin-7-yl)-imidazolidin-2-on
Schmelzpunkt: 146-147°C
$R_f$-Wert: 0,62 (Kieselgel; Essigester/Cyclohexan = 3:1)

(3) 1-[4-12-(Ethoxycarbonyl)-1-propyl]phenyl]-3-(3-trifluoracetyl-2,3,4,5-tetrahydro-1H-3-benzazepin-7-yl)-3H-imidazolidin-2-on
$R_f$-Wert: 0,32 (Kieselgel; Cyclohexan/Essigester = 2:1)

(4) 1-[2-[4-(Methoxycarbonyl)phenyl]ethyl]-3-(3-trifluoracetyl-2,3,4,5-tetrahydro-1H-3-benzazepin-7-yl)-imidazolidin-2-on
Schmelzpunkt: 143-144°C
$R_f$-Wert: 0,46 (Kieselgel; Cyclohexan/Essigester= 1:1)

(5) 1-[trans-4-[2-(Methoxycarbonyl)ethyl]cyclohexyl]-3-(3-ethyl-2,3,4,5-tetrahydro-1H-3-benzazepin-7-yl)-imidazolidin-2-on-hydrochlorid
Schmelzpunkt: ab 230°C (Zers.)
$R_f$-Wert: 0,22 (Reversed Phase Kieselgel; Methanol/5%ige wäßrige Kochsalzlösung = 6:4)

(6) 1-[trans-4-[2-(Methoxycarbonyl)ethyl]cyclohexyl]-3-(7-ethyl-6,7,8,9-tetrahydro-5H-pyrazino[2,3-d]azepin-2-yl)-imidazolidin-2-on
Schmelzpunkt: 136-138°C
$R_f$-Wert: 0,33 (Kieselgel; Methylenchlorid/Methanol = 9:1)

[7) 1-[4-[2-(Ethoxycarbonyl)-1-phenyl-ethyl]phenyl]-3(3-trifluoacetyl-2,3,4,5-tetrahydro-1H-benzazepin-7-yl) imidazolidin-2-on
$R_f$-Wert: 0,22 (Kieselgel; cyclohexan/Essigester = 7:3)

Beispiel 13

1-[4-[2-(Methoxycarbonyl)ethyl]phenyl]-3-[(4aRS,6RS,8aRS)-2-methyl-1,2,3,4,4a,5,6,7,8,8a-decahydroisochinolin-6-yl]-3H-imidazol-2-on

Zu 6,9 g (4aRS,6RS,8aRS)-6-[(2,2-Dimethoxyethyl)amino]-2-methyl-1,2,3,4,4a,5,6,7,8,8a-decahydroisochinolin in 30 ml Dioxan werden 6,4 g 4-[2-(Methoxycarbonyl)ethyl]-phenylisocyanat gegeben und 2 Stunden bei Raumtemperatur gerührt. Das Reaktionsgemisch wird eingeengt, in 100 ml Methanol aufgenommen, mit methanolischer Salzsäure versetzt und 20 Minuten unter Rückfluß erhitzt. Das Reaktionsgemisch wird eingeengt und der Rückstand mit 200 ml Wasser verrührt. Es wird vom Niederschlag abgesaugt und das Filtrat wird mit Essigester extrahiert. Die organische Phase wird verworfen. Die wäßrige Phase wird mit Kaliumcarbonatlösung alkalisch gestellt und mehrmals mit Essigester extrahiert. Die vereinigten Essigesterextrakte werden einmal mit gesättigter Kochsalzlösung gewaschen, getrocknet und eingeengt. Das Rohprodukt wird durch Chromatographie über eine Aluminiumoxidsäule (basisch) mit Essigester und anschließendem Verreiben mit tert.Butyl-methylether gereinigt.
Ausbeute: 1,03 g (9 % der Theorie),
$R_f$-Wert: 0,55 (Kieselgel; Toluol/Dioxan/Methanol/konz. wäßriges Ammoniak 20:50:20:5)

Analog Beispiel 13 werden folgende Verbindungen erhalten:

(1) 1-[4-[2-(Methoxycarbonyl)ethyl]phenyl]-3-[(4aRS,6SR, 8aRS)-2-methyl-1,2,3,4,4a,5,6,7,8,8a-decahydroisochinolin-6-yl]-3H-imidazol-2-cn x 0,3 Wasser
Schmelzpunkt: 140-145°C

| | | | | | | |
|---|---|---|---|---|---|---|
| Ber. | C | 68,56 | H | 7,91 | N | 10,43 |
| Gef. | | 68,49 | | 7,97 | | 10,51 |

(2) 1-[2-[4-(Methoxycarbonyl)phenyl]ethyl]-3-(3-trifluoracetyl-2,3,4,5-tetrahydro-1H-3-benzazepin-7-yl)-3H-imidazol-2-on
Amin und Isocyanat werden in Dioxan zuerst bei Raumtemperatur dann auf dem Dampfbad gerührt. Die Behandlung mit methanolischer Salzsäure entfällt. Das eingesetzte [2-[4-(Methoxycarbonyl)phenyl]ethyl]-isocyanat wird durch Umsetzung des entsprechenden Amin-hydrochlorids mit Phosgen erhalten.
Schmelzpunkt: 165-167°C
$R_f$-Wert: 0,31 (Kieselgel; Cyclohexan/Essigester = 1:1).

Beispiel 14

4-[4-[2-(carboxyethyl)phenyl]-5-methyl-2-(2,3,4,5-tetrahydro-1H-3-benzazepin-7-yl)-4H-1,2,4-triazol-3-on-hydrochloridhydrat

410 mg 4-[4-[2-(Methoxycarbonyl)ethyl]phenyl]-5-methyl-2-(3-trifluoracetyl-2,3,4,5-tetrahydro-1H-3-benzazepin-7-yl)-4H-1,2,4-triazol-3-on werden mit 10 ml Eisessig und 10 ml halbkonzentrierter Salzsäure 7 Stunden bei 90°C gerührt. Nach dem Abkühlen wird eingedampft, der Rückstand mit Wasser verrührt, abgesaugt und mit Wasser und Aceton gewaschen.
Ausbeute: 240 mg (57 % der Theorie),

Schmelzpunkt: >250°C
$R_f$-Wert: 0,43 (Reversed Phase Kieselgel; Methanol/5%ige wäßrige Kochsalzlösung = 6:4)

| | | | | | | | | |
|---|---|---|---|---|---|---|---|---|
| Ber. | C | 59,12 | H | 6,09 | N | 12,53 | Cl | 7,93 |
| Gef. | | 58,96 | | 6,13 | | 12,31 | | 7,74 |

Analog Beispiel 14 werden folgende Verbindungen erhalten:

(1) 1-[trans-4-(2-carboxyethyl)cyclohexyl]-3-(2,3,4,5-tetrahydro-1H-3-benzazepin-7-yl)-imidazolidin-2-on-hydrochlorid
Schmelzpunkt: >250°C
$R_f$-Wert: 0,46 (Reversed Phase Kieselgel; Methanol/5%±ige wäßrige Kochsalzlösung = 6:4)

| | | | | | | | | |
|---|---|---|---|---|---|---|---|---|
| Ber. | C | 62,62 | H | 7,64 | N | 9,98 | Cl | 8,40 |
| Gef. | | 62,24 | | 7,67 | | 10,01 | | 8,86 |

(2) 1-[trans-4-(2-carboxyethyl)cyclohexyl]-3-(1,2,3,4,5,6-hexahydro-3-benzazocin-8-yl)-imidazolidin-2-on-hydrochlorid

(3) 2-[4-(2-Carboxyethyl)phenyl]-4-(2,3,4,5-tetrahydro-1H-3-benzazepin-7-yl)-4H-1,2,4-triazol-3-on-hydrochlorid

(4) 4-[4-(2-Carboxyethyl)phenyl]-5-phenyl-2-(2,3,4,5-tetrahydro-1H-3-benzazepin-7-yl)-4H-1,2,4-triazol-3-on-hydrochlorid

(5) 4-[4-(2-Carboxyethyl)phenyl]-2-(2,3,4,5-tetrahydro-1H-3-benzazepin-7-yl)-5-trifluormethyl-4H-1,2,4-triazol-3-on-hydrochlorid

(6) 1-[4-(2-Carboxyethyl)phenyl]-3-(2,3,4,5-tetrahydro-1H-3-benzazepin-7-yl)-3,4,5,6-tetrahydro-1H-pyrimidin-2-on-hydrochlorid

(7) 4-[4-(2-carboxyethyl)phenyl]-5-ethyl-2-(2,3,4,5-tetrahydro-1H-3-benzazepin-7-yl)-4H-1,2,4-triazol-3-on-hydrochlorid

(8) 5-[4-(2-Carboxyethyl)phenyl]-4-methyl-2-(2,3,4,5-tetrahydro-1H-3-benzazepin-7-yl)-4H-1,2,4-triazol-3-on-hydrochlorid

(9) 4-[4-(2-Carboxyethyl)phenyl]-3-methyl-1-(1,2,3,4-tetrahydroisochinolin-6-yl)-imidazolidin-2-on-hydrochlorid

(10) 2-[trans-4-(2-Carboxyethyl)cyclohexyl]-4-(2,3,4,5-tetra-hydro-1H-3-benzazepin-7-yl)-2H,4H-1,2,4-triazol-3,5-dionhydrochlorid

(11) 1-[3-(2-carboxyethyl)phenyl]-3-(2,3,4,5-tetrahydro-1H-3-benzazepin-7-yl)-imidazolidin-2-on-hydrochlorid

(12) 1-[4-(carboxymethyl)phenyl]-3-(2,3,4,5-tetrahydro-1H-3-benzazepin-7-yl)-imidazolidin-2-on-hydrochlorid

(13) 2-[trans-4-(2-Carboxyethyl)cyclohexyl]-5-(2,3,4,5-tetrahydro-1H-3-benzazepin-7-yl)-3,4-dihydro-2H,5H-1,2,5-thiadiazol-1,1-dioxid
$R_f$-Wert: 0,50 (Reversed Phase Kieselgel; Methanol/5%ige wäßrige Kochsalzlösung = 6:4)

(14) 1-14-(2-carboxy-1-octyl)phenyl]-3-(2,3,4,5-tetrahydro-1H-3-benzazepin-7-yl)-imidazolidin-2-on-hydrochlorid

(15) 2-[trans-4-(2-Carboxyethyl)cyclohexyl]-4-(2,3,4,5-tetrahydro-1H-3-benzazepin-7-yl)-1-methyl-2H,4H-1,2,4-triazol-3,5-dion-hydrochlorid

(16) 1-[4-(3-Carboxypropyl) phenyl]-3-(2,3,4,5-tetrahydro-1H-3-benzazepin-7-yl)-imidazolidin-2-on-hydrochlorid

(17) 1-(5-Carboxypentyl)-3-(2,3,4,5-tetrahydro-1H-3-benzazepin-7-yl)-imidazolidin-2-on-hydrochlorid

(18) 1-[4-(2-Carboxyethyl)-2-fluor-phenyl]-3-(2,3,4,5-tetrahydro-1H-3-benzazepin-7-yl)-imidazolidin-2-on-hydrochlorid

(19) 1-[4-(2-Carboxyethyl)-3-methyl-phenyl]-3-(2,3,4,5-tetrahydro-1H-3-benzazepin-7-yl)-imidazolidin-2-on-hydrochlorid

(20) 1-[trans-4-[(Carboxymethyl)oxy]cyclohexyl]-3-(2,3,4,5-tetrahydro-1H-3-benzazepin-7-yl)-imidazolidin-2-on-hydrochlorid
Schmelzpunkt: 316-317°C (Zers.)
$R_f$-Wert: 0,66 (Reversed Phase Kieselgel; Methanol/5%ige wäßrige Kochsalzlösung = 6:4)

| | | | | | | | | |
|---|---|---|---|---|---|---|---|---|
| Ber. | C | 59,50 | H | 7,13 | N | 9,91 | Cl | 8,36 |
| Gef. | | 59,26 | | 7,13 | | 9,94 | | 8,44 |

(21) 1-[4-(trans-2-Carboxyethenyl)phenyl]-3-(2,3,4,5-tetrahydro-1H-3-benzazepin-7-yl)-imidazolidin-2-on-hydrochlorid
$R_f$-Wert: 0,64 (Reversed Phase Kieselgel; Methanol/5%ige wäßerige Kochsalzlösung = 6:4)
Massenspektrum: $M^+$ = 377

(22) 1-[4-(2-Carboxy-1-propyl)phenyl]-3-(2,3,4,5-tetrahydro-1H-3-benzazepin-7-yl)-imidazolidin-2-on-hydrochlorid x $H_2O$
$R_f$-Wert: 0,34 (Reversed Phase Kieselgel; Mechanol/5%ige wäßerige Kochsalzlösung = 6:4)

| | | | | | | | | |
|---|---|---|---|---|---|---|---|---|
| Ber. | C | 61,66 | H | 6,71 | N | 9,38 | Cl | 7,91 |
| Gef. | | 61,56 | | 6,75 | | 9,30 | | 8,14 |

(23) 1-trans-4-(2-Carboxyethyl)cyclohexyl]-3-(2,3,4,5-tetrahydro-1H-3-benzazepin-7-yl)-3H-imidazol-2-on-hydrochlorid

(24) 1-[trans-4-(2-Carboxyethyl)cyclohexyl]-3-(2,3,4,5-tetrahydro-1H-2-benzazepin-7-yl)-imidazolidin-2-on-hydrochlorid

(25) 2-[trans-4-(2-Carboxyethyl)cyclohexyl]-4-(2,3,4,5-tetrahydro-1H-3-benzazepin-7-yl)-4H-1,2,4-triazol-3-on-hydrochlorid
Durchführung mit 2N Salzsäure
Ausgangsmaterial: Verbindung des Beispiels 18(1)
Schmelzpunkt: >220°C
$R_f$-Wert: 0,50 (Reversed Phase Kieselgel; Methanol/5%ige wäßerige Kochsalzlösung = 6:4)

| | | | | | | | | |
|---|---|---|---|---|---|---|---|---|
| Ber. | C | 59,92 | H | 6,94 | N | 13,31 | Cl | 8,42 |
| Gef. | | 60,03 | | 6,99 | | 13,32 | | 8,46 |

(26) 2-[trans-4-(2-Carboxyethyl)cyclohexyl]-4-(2,3,4,5-tetrahydro-1H-3-benzazepin-7-yl)-5-methyl-4H-1,2,4-triazol-3-on-hydrochlorid

(27) 2-[trans-4-(2-Carboxyethyl)cyclohexyl]-4-(2,3,4,5-tetrahydro-1H-3-benzazepin-7-yl)-5-phenyl-4H-1,2,4-triazol-3-on-hydrochlorid

(28) 4-[trans-4-(2-Carboxyethyl)cyclohexyl]-2-(2,3,4,5-tetrahydro-1H-3-benzazepin-7-yl)-4H-1,2,4-triazol-3-on-hydrochlorid
Schmelzpunkt: >240°C
$R_f$-Wert: 0,64 (Reversed Phase Kieselgel; Methanol/5%ige wäßerige Kochsalzlösung = 6:4)

(29) 4-[trans-4-(2-Carboxyethyl)cyclohexyl]-2-(2,3,4,5-tetrahydro-1H-3-benzazepin-7-yl)-5-methyl-4H-1,2,4-triazol-3-on-hydrochlorid
$R_f$-Wert: 0,48 (Reversed Phase Kieselgel; Methanol/5%ige wäßerige Kochsalzlösung = 6:4)

| | | | | | | | | |
|---|---|---|---|---|---|---|---|---|
| Ber. | C | 60,75 | H | 7,18 | N | 12,88 | Cl | 8,15 |
| Gef. | | 60,54 | | 7,26 | | 12,68 | | 8,54 |

(30) 4-[trans-4-(2-carboxyethyl)cyclohexyl]-2-(2,3,4,5-tetrahydro-1H-3-benzazepin-7-yl)-5-phenyl-4H-1,2,4-triazol-3-on-hydrochlorid

(31) 1-[4-(2-Carboxy-1-phenyl-ethyl)phenyl]-3-(2,3,4,5-tetrahydro-1H-3-benzazepin-7-yl)-imidazolidin-2-on-hydrochlorid
$R_f$-Wert: 0,31 (Reversed Phase Kieselgel; Methanol/5%ige wäßerige Kochsalzlösung = 6:4)

(32) 1-[1-12-Carboxyethyl)piperidin-4-yl]-3-(2,3,4,5-tetranydro-1H-3-benzazepin-7-yl)-imidazolidin-2-on-hydrochlorid

(33) 1-(trans-4-Carboxycyclohexyl)-3-[2-(2,3,4,5-tetrahydro-1H-3-benzazepin-7-yl)ethyl]-imidazolidin-2-on-hydrochlorid

(34) 1-[2-(4-carboxyphenyl)ethyl]-3-(2,3,4,5-tetrahydro-1H-3-benzazepin-7-yl)-imidazolidin-2-on-hydrochlorid x $H_2O$
$R_f$-Wert: 0,40 (Reversed Phase Kieselgel; Methanol/5%ige wäßerige Kochsalzlösung = 6:4)
Massenspektrum: $M^+$ = 379

| | | | | | | | | |
|---|---|---|---|---|---|---|---|---|
| Ber. | C | 60,89 | H | 6,50 | N | 9,68 | Cl | 8,17 |
| Gef. | | 60,57 | | 6,62 | | 9,77 | | 8,22 |

Beispiel 15

4-[4-[2-(Methoxycarbonyl)ethyl]phenyl]-5-methyl-2-(3-trifluoracetyl-2,3,4,5-tetrahydro-1H-3-benzazepin-7-yl)-4H-1,2,4-triazol-3-on

1,6 g 7-Iod-3-trifluoracetyl-2,3,4,5-tetrahyäro-1H-3-benzazepin, 1,1 g 4-[4-[2-(Methoxycarbonyl)ethyl]phenyl]-5-methyl-4H-1,2,4-triazol-3-on, 0,19 ml Tris-[2-(2-methoxyethoxy)-ethyl]-amin, 130 mg Kupfer(I)chlorid, 130 mg Kupfer (I)-iodid und 1,1 g Kaliumcarbonat werden in 30 ml Dimethylformamid 2 Stunden unter Rückfluß erhitzt. Nach dem Abkühlen wird eingedampft und der Rückstand zwischen Wasser und Methylenchlorid verteilt. Der Feststoff wird abgesaugt, die organische Phase wird abgetrennt, mit Wasser gewaschen, getrocknet, filtriert und einrotiert. Der Rückstand wird durch Chromatographie über eine Kieselgelsäule mit Cyclohexan/Essigester = 1:1 gereinigt.
Ausbeute: 340 mg (16 % der Theorie),
Schmelzpunkt: 160-162°C
Rf-Wert: 0,51 (Kieselgel; Cyclohexan/Essigester = 1:1)

Analog Beispiel 15 werden folgende Verbindungen erhalten:

(1) 4-[trans-4-[2-(Methoxycarbonyl)ethyl]cyclohexyl]-2-(3-trifluoracetyl-2,3,4,5-tetrahydro-1H-3-benzazepin-7-yl)-5-methyl-4H-1,2,4-triazol-3-on
Schmelzpunkt: 175-177°C
$R_f$-Wert: 0,50 (Kieselgel; Methylenchlorid/Essigester = 90:10)

(2) 4-[trans-4-[2-(Methoxycarbonyl)ethyl]cyclohexyl]-2-(3-trifluoracetyl-2,3,4,5-tetrahydro-1H-3-benzazepin-7-yl)-4H-1,2,4-triazol-3-on
$R_f$-Wert: 0,40 (Kieselgel; Methylenchlorid/Methanol = 100:1)

Beispiel 16

1-[trans-4-[2-(Methoxycarbonyl)ethyl]cyclohexyl]-3-(3-trifluoracetyl-2,3,4,5-tetrahydro-1H-3-benzazepin-7-yl)-hydantoin

Zu 4,1 l g (3-Trifluoracetyl-2,3,4,5-tetrahydro-1H-3-benzazepin-7-yl)-isocyanat und 3,7 g [[trans-4-[2-(Methoxycarbonyl)-ethyl]cyclohexyl]amino]-essigsäure-methylester-hydrochlorid in 50 ml Acetonitril werden 3,2 ml N-Ethyl-diisopropylamin gegeben und 2 1/2 Tage bei Raumtemperatur gerührt. Das Reaktionsgemisch wird eingeengt, der Rückstand in Essigester aufgenommen und mit Wasser und Kochsalzlösung gewaschen. Die organische Phase wird getrocknet, eingeengt und der Rückstand mit wenig Methanol im Eisbad kristallisiert. Das Produkt wird abgesaugt, mit kaltem Methanol gewaschen und getrocknet.
Ausbeute: 4,1 g (56 % der Theorie),
Schmelzpunkt: 118-120°C
$R_f$-Wert 0,85 (Kieselgel; Essigester)

Beispiel 17

1-[trans-4-[2-(Isopropoxycarbonyl)ethyl]cyclohexyl]-3-(3-propyl-2,3,4,5-tetrahydro-1H-3-benzazepin-7-yl)-imidazolidin-2-on-hydrochlorid

230 mg 1-[trans-4-[2-(Isopropoxycarbonyl)ethyl]cyclohexyl]-3-(3-allyl-2,3,4,5-tetrahydro-1H-3-benzazepin-7-yl)-imidazolidin-2-on-hydrochlorid werden in 10 ml Isopropanol bei 30°C und einem Wasserstoffdruck von 50 psi in Gegenwart von 50 mg 10%igem Palladium auf Aktivkohle 6 Stunden hydriert. Der Katalysator wird abgesaugt und das Filtrat eingedampft.
Ausbeute: 240 mg,
$R_f$-Wert 0,55 (Kieselgel; Methylenchlorid/Methanol/konz. wäßeriges Ammoniak = 95:5:1)

Beispiel 18

2-[trans-4-[2-(Methoxycarbonyl)ethyl]cyclohexyl]-5-(3-trifluoracetyl-2,3,4,5-tetrahydro-1H-3-benzazepin-7-yl)-3,4-dihydro-2H,5H-1,2,5-thiadiazol-1,1-dioxid

Zu 1,25 g 2-(3-Trifluoracetyl-2,3,4,5-tetrahydro-1H-3-benzazepin-7-yl)-3,4-dihydro-2H,5H-1,2,5-thiadiazol-1,1-dioxid, 0,64 g 3-(cis-4-Hydroxycyclohexyl)propionsäure-methylester [$R_f$-Wert: 0,58 (Kieselgel; Cyclohexan/Essigester = 1:1); isoliert aus dem cis/trans-Gemisch durch Chromatographie über eine Kieselgelsäule mit Cyclohexan/Essigester (1:1)] und 0,90 g Triphenylphosphin in 2 ml Acetonitril werden 0,54 ml Azodicarbonsäure-diethylester zugetropft. Es werden nochmal 1,5 g 3-(cis-4-Hydroxycyclohexyl)propionsäure-methylester sowie eine Mischung aus 0,90 g Triphenylphosphin und 0,54 ml Azodicarbonsäure-diethylester in 2 ml Acetonitril zugegeben. Nach 30 Minuten Rühren bei Raumtemperatur wird nochmals eine Mischung aus 0,90 g Triphenylphosphin und 0,54 ml Azodicarbonsäure-diethylester zugegeben. Nach Rühren bei Raumtemperatur über Nacht wird einrotiert und der Rückstand durch Chromatographie über eine Kieselgelsäule mit
Cyclohexan/Essigester/Methylenchlorid (1:1:1) gereinigt.
Ausbeute: 700 mg (38 % der Theorie),
Schmelzpunkt: 169-173°C
$R_f$-Wert: 0,61 (Kieselgel; Cyclohexan/Essigester/Methylenchlorid = 1:1:1)

Analog Beispiel 18 wird folgende Verbindung erhalten:

(1) 2-[trans-4-[2-(Methoxycarbonyl)ethyl]cyclohexyl]-4-(3-tert.butyloxycarbonyl-2,3,4,5-tetrahydro-1H-3-benzazepin-7-yl)-4H-1,2,4-triazol-3-on
Schmelzpunkt: 162-164°C
$R_f$-Wert: 0,43 (Kieselgel; Cyclohexan/Essigester/Methylenchlorid = 1:1:1)

Beispiel 19

1-[trans-4-(2-Carboxyethyl)cyclohexyl]-3-(3-methyl-2,3,4,5-tetrahydro-1H-3-benzazepin-7-yl)-imidazolidin-2-on-hydrochlorid

Zu einem Gemisch aus 5,0 g 1-[trans-4-(2-Carboxyethyl)cyclohexyl]-3-(2,3,4,5-tetrahydro-1H-3-benzazepin-7-yl)-imidazolidin-2-on-hydrochlorid, 4,5 ml Ameisensäure, 3,6 ml 37±igem wäßrigem Formaldehyd und 20 ml Wasser werden unter Eiskühlung 2,0 g Natriumhydrogencarbonat zugegeben und dann auf 65°C erhitzt. Nach 8 Stunden wird abgekühlt, über Nacht gerührt und das Gemisch einrotiert. Der Rückstand wird mit Wasser gerührt und wieder eingedampft. Anschließend wird der Rückstand erneut mit Wasser gerührt und mit Salzsäure auf einen pH-Wert von 1 gebracht. Es wird einrotiert, der Rückstand mit wenig Wasser verrührt und abgesaugt. Der Filterkuchen wird mit Aceton gerührt, das Produkt wird abgesaugt, mit Aceton gewaschen und im Vakuum getrocknet.

Ausbeute: 3,7 g (71 % der Theorie),
Schmelzpunkt: 292-295°C (Zers.)
$R_f$-Wert: 0,38 (Reversed Phase Kieselgel; Methanol/5%ige wäßrige Kochsalzlösung = 6:4)
Analog Beispiel 19 werden folgende Verbindungen erhalten:

(1) 1-[trans-4-[(Carboxymethyl)oxy]cyclohexyl]-3-(3-methyl-2,3,4,5-tetrahydro-1H-3-benzazepin-7-yl)-imidazolidin-2-on-hydrochlorid x NaCl x 0,5 $H_2O$
Rf-Wert: 0,62 (Reversed Phase Kieselgel; Methanol/5%ige wäßerige Kochsalzlösung = 6:4)

| | | | | | | | | |
|---|---|---|---|---|---|---|---|---|
| Ber. | C | 52,28 | H | 6,58 | N | 8,31 | Cl | 13,49 |
| Gef. | | 52,28 | | 6,68 | | 8,33 | | 14,03 |

(2) 1-[4-(2-Carboxy-1-propyl)phenyl]-3-(3-methyl-2,3,4,5-tetrahydro-1H-3-benzazepin-7-yl)-imidazolidin-2-on-hydrochlorid x 0,5 $H_2O$
$R_f$-Wert: 0,29 (Reversed Phase Kieselgel; Methanol/5%ige wäßerige Kochsalzlösung = 6:4)

| | | | | | | | | |
|---|---|---|---|---|---|---|---|---|
| Ber. | C | 63,63 | H | 6,90 | N | 9,28 | Cl | 7,83 |
| Gef. | | 63,32 | | 6,99 | | 9,32 | | 7,81 |

(3) 3-[trans-4-(2-Carboxyethyl)cyclohexyl]-1-(3-methyl-2,3,4,5-tetrahydro-1H-3-benzazepin-7-yl)-hydantoin-hydrochlorid Schmelzpunkt: >250°C
$R_f$-Wert: 0,32 (Reversed Phase Kieselgel; Methanol/5%ige wäßerige Kochsalzlösung = 6:4)

| | | | | | | | | |
|---|---|---|---|---|---|---|---|---|
| Ber. | C | 61,39 | H | 7,17 | N | 9,34 | Cl | 7,88 |
| Gef. | | 61,39 | | 7,25 | | 9,37 | | 7,92 |

(4) 1-[trans-4-(2-Carboxyethyl)cyclohexyl]-3-(3-methyl-2,3,4,5-tetrahydro-1H-3-benzazepin-7-yl)-hydantoin-hydrochlorid
$R_f$-Wert: 0,37 (Kieselgel; Methylenchlorid/Methanol/konz. wäßeriges Ammoniak = 80:20:2)

| | | | | | | | | |
|---|---|---|---|---|---|---|---|---|
| Ber. | C | 61,39 | H | 7,17 | N | 9,34 | Cl | 7,88 |
| Gef. | | 61,11 | | 7,26 | | 9,36 | | 7,86 |

(5) 2-[trans-4-(2-Carboxyethyl)cyclohexyl]-4-(3-methyl-2,3,4,5-tetrahydro-1H-3-benzazepin-7-yl)-1-methyl-2H,4H-1,2,4-triazol-3,5-dion-hydrochlorid

(6) 2-[trans-4-(2-Carboxyethyl)cyclohexyl]-4-(3-methyl-2,3,4,5-tetrahydro-1H-3-benzazepin-7-yl)-4H-1,2,4-triazol-3-on-hydrochlorid x 1,1 Wasser
Schmelzpunkt: >220°C
$R_f$-Wert: 0,48 (Reversed Phase Kieselgel; Methanol/5%ige wäßerige Kochsalzlösung = 6:4)

| | | | | | | | | |
|---|---|---|---|---|---|---|---|---|
| Ber. | C | 58,10 | H | 7,36 | N | 12,32 | Cl | 7,80 |
| Gef. | | 58,07 | | 7,36 | | 12,28 | | 7,83 |

(7) 2-[trans-4-(2-Carboxyethyl)cyclohexyl]-4-(3-methyl-2,3,4,5-tetrahydro-1H-3-benzazepin-7-yl)-5-methyl-4H-1,2,4-triazol-3-on-hydrochlorid

(8) 2-[trans-4-(2-Carboxyethyl)cyclohexyl]-4-(3-methyl-2,3,4,5-tetrahydro-1H-3-benzazepin-7-yl)-5-phenyl-4H-1,2,4-triazol-3-on-hydrochlorid

(9) 4-[trans-4-(2-Carboxyethyl)cyclohexyl]-2-(3-methyl-2,3,4,5-tetrahydro-1H-3-benzazepin-7-yl)-4H-1,2,4-triazol-3-on-hydrochlorid

(10) 4-[trans-4-(2-Carboxyethyl)cyclohexyl]-2-(3-methyl-2,3,4,5-tetrahydro-1H-3-benzazepin-7-yl)-5-methyl-4H-1,2,4-triazol-3-on x 1,1 HCl x 0,2 $H_2O$
Schmelzpunkt: 238-240°C

$R_f$-Wert: 0,37 (Reversed Phase Kieselgel; Methanol/5%ige wäßerige Kochsalzlösung = 6:4)

| | | | | | | | | |
|---|---|---|---|---|---|---|---|---|
| Ber. | C | 60,55 | H | 7,40 | N | 12,28 | Cl | 8,55 |
| Gef. | | 60,68 | | 7,53 | | 12,31 | | 8,36 |

(11) 4-[trans-4-(2-Carboxyethyl)cyclohexyl]-2-(3-methyl-2,3,4,5-tetrahydro-1H-3-benzazepin-7-yl)-5-phenyl-4H-1,2,4-triazol-3-on-hydrochlorid

(12) 1-[trans-4-(2-Carboxyethyl)cyclohexyl]-3-(3-methyl-2,3,4,5-tetrahydro-1H-3-benzazepin-7-yl)-3,4,5,6-tetrahydro-1H-pyrimidin-2-on x 1,05 HCl x 0,3 $H_2O$
Schmelzpunkt: 237-240°C
$R_f$-Wert: 0,37 (Reversed Phase Kieselgel; Methanol/5%ige wäßerige Kochsalzlösung = 6:4)

| | | | | | | | | |
|---|---|---|---|---|---|---|---|---|
| Ber. | C | 63,04 | H | 8,07 | N | 9,18 | Cl | 8,14 |
| Gef. | | 62,97 | | 8,05 | | 9,15 | | 8,16 |

(13) 1-[4-(2-Carboxy-1-phenyl-ethyl)phenyl]-3-(3-methyl-2,3,4,5-tetrahydro-1H-3-benzazepin-7-yl)-imidazolidin-2-on-hydrochlorid

(14) 1-[1-(2-Carboxyethyl)piperidin-4-yl]-3-(3-methyl-2,3,4,5-tetrahydro-1H-3-benzazepin-7-yl)-imidazolidin-2-on-hydrochlorid

(15) 1-(trans-4-Carboxycyclohexyl)-3-[2-(3-methyl-2,3,4,5-tetrahydro-1H-3-benzazepin-7-yl)ethyl]-imidazolidin-2-on-hydrochlorid

(16) 1-[2-(4-Carboxyphenyl)ethyl]-3-(3-methyl-2,3,4,5-tetrahydro-1H-3-benzazepin-7-yl)-imidazolidin-2-on-hydrochlorid

(17) 1-[trans-4-(2-Carboxyethyl)cyclohexyl]-3-(7-methyl-6,7,8,9-tetrahydro-5H-pyrimido[4,5-d]azepin-2-yl)-imidazolidin-2-on-hydrochlorid
$R_f$-Wert: 0,56 (Reversed Phase Kieselgel; Methanol/5%ige wäßerige Kochsalzlösung = 6:4)
Massenspektrum: $M^+$ = 401

(18) 1-[trans-4-(2-Carboxyethyl)cyclohexyl]-3-(7-methyl-6,7,8,9-tetrahydro-5H-pyrazino[2,3-d]azepin-2-yl)-imidazolidin-2-on-hydrochlorid

(19) 1-[trans-4-(2-Carboxyethyl)cyclohexyl]-3-(7-methyl-6,7,8,9-tetrahydro-5H-pyrido[2,3-d]azepin-2-yl)-imidazolidin-2-on-hydrochlorid

(20) 1-[trans-4-(2-Carboxyethyl)cyclohexyl]-3-(2-methyl-1,2,3,4-tetrahydro-isochinolin-6-yl)-imidazolidin-2-on-hydrochlorid

(21) 1-[trans-4-(2-Carboxyethyl)cyclohexyl]-3-(2-methyl-2,3,4,5-tetrahydro-1H-2-benzazepin-7-yl)-imidazolidin-2-on-hydrochlorid

(22) 1-[trans-4-(2-Carboxethyl)cyclohexyl]-3-(3-methyl-1,2,3,4,5,6-hexahydro-3-benzazocin-8-yl)-imidazolidin-2-on-hydrochlorid

(23) 2-[trans-4-(2-Carboxyethyl)cyclohexyl]-4-(3-methyl-2,3,4,5-tetrahydro-1H-3-benzazepin-7-yl)-2H,4H-1,2,4-triazol-3,5-dion-hydrochlorid

## Beispiel 20

### 1-(3-Ethyl-2,3,4,5-tetrahydro-1H-3-benzazepin-7-yl)-3-[4-[2-(methoxycarbonyl)ethyl]phenyl-imidazolidin-2-on

600 mg 1-[4-[2-(Methoxycarbonyl)ethyl]phenyl]-3-(2,3,4,5-tetrahydro-1H-3-benzazepin-7-yl)-imidazolidin-2-on-hydrochlorid und 128 mg Natriumhydrid (55%ig in Paraffinöl) werden in 15 ml Dimethylformamid 2,5 Stunden im Ultraschallbad behandelt. Es werden 125 µl Ethyljodid zugegeben und weitere 2,5 Stunden behandelt. Das Reaktionsgemisch wird mit 50 ml Wasser versetzt, der Niederschlag abgesaugt, mit Wasser gewaschen und getrocknet. Das Rohprodukt wird durch Chromatographie über eine Kieselgelsäule mit Methylenchlorid/Methanol/ konz. wäßriges Ammoniak (9:1:0,1) gereinigt.
Ausbeute: 260 mg (44 % der Theorie),
Schmelzpunkt: 168-170°C
$R_f$-Wert: 0,45 (Kieselgel; Methylenchlorid/Methanol/konz. wäßriges Ammoniak = 4:1:0,2)
Analog Beispiel 20 werden folgende Verbindungen erhalten:

(1) 1-(3-Butyl-2,3,4,5-tetrahydro-1H-3-benzazepin-7-yl)-3-[4-[2-(methoxycarbonyl)ethyl]phenyl]-imidazolidin-2-on
$R_f$-Wert: 0,64 (Kieselgel; Methylenchlorid/Methanol/konz. wäßriges Ammoniak = 4:1:0,2)

(2) 1-[trans-4-[2-(Isopropoxycarbonyl)ethyl]cyclohexyl]-3-(3-allyl-2,3,4,5-tetrahydro-1H-3-benzazepin-7-yl)-imidazolidin-2-on-hydrochlorid
Durchführung mit Allylbromid/N-Ethyl-diisopropylamin in Acetonitril
$R_f$-Wert: 0,63 (Kieselgel; Methylenchlorid/Methanol/konz. wäßriges Ammoniak = 95:5:1)

(3) 1-[trans-4-[2-(Methoxycarbonyl)ethyl]cyclohexyl]-3-(3-benzyl-2,3,4,5-tetrahydro-1H-3-benzazepin-7-yl)-imidazolidin-2-on-hydrochlorid
$R_f$-Wert: 0,30 (Kieselgel; Methylenchlorid/Methanol = 95:5)

(4) 1-[trans-4-[2-(Methoxycarbonyl)ethyl]cyclohexyl]-3-[3-(cyclopropylmethyl)-2,3,4,5-tetrahydro-1H-3-benzazepin-7-yl)-imidazolidin-2-on-hydrojodid
Durchführung in Acetonitril mit Cyclopropylmethylbromid in Gegenwart von Natriumjodid und N-Ethyl-diisopropylamin
Schmelzpunkt: 225-230°C (Zers.)
$R_f$-Wert: 0,72 (Kieselgel; Methylenchlorid/Methanol/konz. wäßeriges Ammoniak = 90:10:2)

(5) 1-[trans-4-[2-(Methoxycarbonyl)ethyl]cyclohexyl]-3-[3-(2-hydroxyethyl)-2,3,4,5-tetrahydro-1H-3-benzazepin-7-yl]-imidazolidin-2-on-hydrojodid
Durchführung in Acetonitril mit 2-Bromethanol in Gegenwart von Natriumjodid und N-Ethyl-diisopropylamin.
Schmelzpunkt: >200°C
$R_f$-Wert: 0,55 (Kieselgel; Methylenchlorid/Methanol/konz. wäßeriges Ammoniak = 90:10:2)

(6) 1-[trans-4-[2-(Methoxycarbonyl)ethyl]cyclohexyl]-3-[3-(methoxycarbonylmethyl)-2,3,4,5-tetrahydro-1H-3-benzazepin-7-yl]-imidazolidin-2-on
Durchführung in Acetonitril mit 2-Bromessigsäuremethylester in Gegenwart von Natriumjodid und N-Ethyl-diisopropylamin. Isolierung der freien Base.
Schmelzpunkt: 127-129°C
$R_f$-Wert: 0,28 (Reversed Phase Kieselgel; Methanol/5 %ige wäßerige Kochsalzlösung = 6:4)

Beispiel 21

3-[trans-4-[2-(Methoxycarbonyl)ethyl)cyclohexyl]-1-(2,3,4,5-tetrahydro-1H-3-benzazepin-7-yl)-hydantoin-hydrochlorid

Über 920 mg 3-[trans-4-[2-(Methoxycarbonyl)ethyl]cyclohexyl]-1-(3-trifluoracetyl-2,3,4,5-tetrahydro-1H-3-benzazepin-7-yl)-hydantoin in 50 ml Methanol wird 10 Minuten lang Salzsäuregas geleitet und anschließend 5 Stunden unter Rückfluß erhitzt. Es wird abgekühlt, das Produkt wird abgesaugt, mit Methanol und Diethylether gewaschen und bei 80°C getrocknet.
Ausbeute: 770 mg (95 % der Theorie),
Schmelzpunkt: >250°C
$R_f$-Wert: 0,23 (Reversed Phase Kieselgel; Methanol/5%ige wäßrige Kochsalzlösung = 6:4)
Analog Beispiel 21 werden folgende Verbindungen erhalten:

(1) 1-[trans-4-[2-(Methoxycarbonyl)ethyl]cyclohexyl]-3-(2,3,4,5-tetrahydro-1H-3-benzazepin-7-yl)-hydantoin-hydrochlorid
Schmelzpunkt: > 250°C

$R_f$-Wert: 0,43 (Reversed Phase Kieselgel; Methanol/5%ige wäßerige Kochsalzlösung = 6:4)

| | | | | | | | | |
|---|---|---|---|---|---|---|---|---|
| Ber. | C | 61,39 | H | 7,17 | N | 9,34 | Cl | 7,88 |
| Gef. | | 61,10 | | 7,21 | | 9,29 | | 8,03 |

(2) 1-[trans-4-[2-(Methoxycarbonyl)ethyl]cyclohexyl]-3-(2,3,4,5-tetrahydro-1H-3-benzazepin-7-yl)-3,4,5,6-tetrahydro-1H-pyrimidin-2-on-hydrochlorid
$R_f$-Wert: 0,31 (Reversed Phase Kieselgel; Methanol/5%ige wäßrige Kochsalzlösung = 6:4)

Beispiel 22

3-[trans-4-[2-(Methoxycarbonyl)ethyl]cyclohexyl]-1-13-tri-fluoracetyl-2,3,4,5-tetrahydro-1H-3-benzazepin-7-yl)-hydantoin

Zu 2,1 g N-[trans-4-[2-[Methoxycarbonyl)ethyl]cyclohexyl]-N'-[(benzyloxycarbonyl)methyl]-N'-(3-trifluoracetyl-2,3,4,5-tetrahydro-1H-3-benzazepin-7-yl)-harnstoff in 25 ml siedendem Toluol werden 40 mg Kalium-tert.butylat zugegeben und 30 Minuten unter Rückfluß erhitzt. Das Reaktionsgemisch wird abgekühlt mit einigen Tropfen Eisessig verrührt, eingeengt und durch Chromatographie über eine Kieselgelsäule mit Cyclohexan/Essigester (8:2 bis 7:3) sowie Kristallisation aus Methanol gereinigt.
Ausbeute: 0,95 g (55 % der Theorie),
Schmelzpunkt: 132-134°C
$R_f$-Wert: 0,59 (Kieselgel; Cyclohexan/Essigester = 1:1)

Beispiel 23

1-[trans-4-[2-(Methoxycarbonyl)ethyl]cyclohexyl]-3-(6,7,8,9-tetrahydro-5H-pyrimido[4,5-d]azepin-2-yl)-imidazolidin-2-on

1,0 g 1-[trans-4-[2-(Methoxycarbonyl)ethyl]cyclohexyl]-3-(7-benzyl-6,7,8,9-tetrahydro-5H-pyrimido[4,5-d]azepin-2-yl)-3H-imidazol-2-on werden in 15 ml Methanol in Gegenwart von 500 mg Palladium auf Aktivkohle (10 % Palladium) 17 Stunden bei Raumtemperatur und einem Wasserstoffdruck von 50 psi hydriert. Es wird vom Katalysator abfiltriert und das Filtrat eingedampft. Der Rückstand wird direkt zur Herstellung der Verbindung des Beispiels 9(16) verwendet.
$R_f$-Wert: 0,44 (Reversed Phase Kieselgel; Methanol/5%ige wäßrige Kochsalzlösung = 6:4)

Analog Beispiel 23 wird folgende Verbindung erhalten:

(1) 1-[trans-4-[2-(Methoxycarbonyl)ethyl]cyclohexyl]-3-(6,7,8,9-tetrahydro-5H-pyrido[2,3-d]azepin-2-yl)-imidazolidin-2-on-hydrochlorid.

Beispiel 24

1-[2-(Ethoxycarbonyl)ethyl]-3-[4-(3-methyl-2,3,4,5-tetrahydro-1H-3-benzazepin-7-yl)phenyl]-imidazolidin-2-on

3,1 g (3-Methyl-2,3,4,5-tetrahydro-1H-3-benzazepin-7-yl)-boronsäure, 4,1 g 1-[2-(Ethoxycarbonyl)ethyl]-3-[4-(trifluormethylsulfonyloxy)phenyl]-imidazolidin-2-on, 3,5 g Tetrakis-(triphenylphosphin)-palladium und 6,5 ml Triethylamin werden in 25 ml Dimethylformamid unter Stickstoff 4 Stunden bei 100°C gerührt. Das Reaktionsgemisch wird abgekühlt, im Vakuum eingeengt und der Rückstand in Methylenchlorid aufgenommen. Das Gemisch wird über Kieselgur filtriert, das Filtrat eingeengt und der Rückstand durch Chromatographie über eine Kieselgelsäule mit Methylenchlorid/Methanol (100:7) gereinigt.
Ausbeute: 2,7 g (63 % der Theorie),
$R_f$-Wert: 0,31 (Kieselgel; Methylenchlorid/Methanol = 100:7)

Beispiel 25

| Trockenampulle mit 2,5 mg Wirkstoff pro 1 ml | |
|---|---|
| Zusammensetzung: | |
| Wirkstoff | 2,5 mg |
| Mannitol | 50,0 mg |
| Wasser für Injektionszwecke ad | 1,0 ml |

Herstellung:
Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

Beispiel 26

| Trockenampulle mit 35 mg Wirkstoff pro 2 ml | |
|---|---|
| Zusammensetzung: | |
| Wirkstoff | 35,0 mg |
| Mannitol | 100,0 mg |
| Wasser für Injektionszwecke ad | 2,0 ml |

Herstellung:
Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

Beispiel 27

| Tablette mit 50 mg Wirkstoff | | |
|---|---|---|
| Zusammensetzung: | | |
| (1) | Wirkstoff | 50,0 mg |
| (2) | Milchzucker | 98,0 mg |
| (3) | Maisstärke | 50,0 mg |
| (4) | Polyvinylpyrrolidon | 15,0 mg |
| (5) | Magnesiumstearat | 2,0 mg |
| | | 215,0 mg |

Herstellung:
(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 28

| Tablette mit 350 g Wirkstoff | | |
|---|---|---|
| Zusammensetzung: | | |
| (1) | Wirkstoff | 350,0 mg |
| (2) | Milchzucker | 136,0 mg |
| (3) | Maisstärke | 80,0 mg |

(fortgesetzt)

| Tablette mit 350 g Wirkstoff | | |
|---|---|---|
| Zusammensetzung: | | |
| (4) | Polyvinylpyrrolidon | 30,0 mg |
| (5) | Magnesiumstearat | 4,0 mg |
| | | 600,0 mg |

Herstellung:
(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 29

| Kapseln mit 50 mg Wirkstoff | | |
|---|---|---|
| Zusammensetzung: | | |
| (1) | Wirkstoff | 50,0 mg |
| (2) | Maisstärke getrocknet | 58,0 mg |
| (3) | Milchzucker pulverisiert | 50,0 mg |
| (4) | Magnesiumstearat | 2,0 mg |
| | | 160,0 mg |

Herstellung:
(1) wird mit (3) verrieben. Diese Verreibung wird der Mischung aus (2) und (4) unter intensiver Mischung zugegeben. Diese Pulvermischung wird auf einer Kapselabfüllmaschine in Hartgelatine-Steckkapseln Größe 3 abgefüllt.

Beispiel 30

| Kapseln mit 350 mg Wirkstoff | | |
|---|---|---|
| Zusammensetzung: | | |
| (1) | Wirkstoff | 350,0 mg |
| 2) | Maisstärke getrocknet | 46,0 mg |
| (3) | Milchzucker pulverisiert | 30,0 mg |
| (4) | Magnesiumstearat | 4,0 mg |
| | | 430,0 mg |

Herstellung:
(1) wird mit (3) verrieben. Diese Verreibung wird der Mischung aus (2) und (4) unter intensiver Mischung zugegeben. Diese Pulvermischung wird auf einer Kapselabfüllmaschine in Hartgelatine-Steckkapseln Größe 0 abgefüllt.

**Patentansprüche**

**1.** Cyclische Derivate der allgemeinen Formel

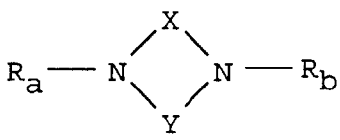

, (I)

in der

X eine am Stickstoffatom durch eine Cyanogruppe substituierte Carbiminogruppe, eine Carbonyl- oder Sulfonylgruppe,

Y eine gegebenenfalls durch $R_c$ oder $R_c$ und $R_d$ substituierte $-CH_2CH_2-$, $-CH_2CH_2CH_2-$, -CH=CH-, $-CH_2CO-$ oder $-COCH_2-$Gruppe,

eine gegebenenfalls durch $R_c$ oder $R_d$ substituierte -CO-NH-, -NH-CO-, -CH=N- oder -N=CH-Gruppe,

einer der Reste $R_a$ bis $R_d$ eine A-B-Gruppe, in der

A eine Gruppe der Formeln

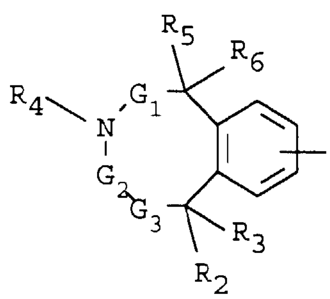

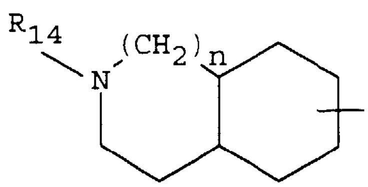

oder

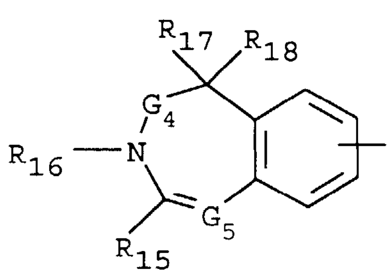

darstellt, wobei jeweils der Benzoteil der vorstehend erwähnten Gruppen durch ein Fluor-, Chlor- oder Bromatom, durch eine Alkyl-, Cyano-, Trifluormethyl-, Hydroxy- oder Alkoxygruppe substituiert sein kann oder eine

bis drei Methingruppen jeweils durch ein Stickstoffatom ersetzt sein können,

$G_1$ eine Bindung oder eine Methylengruppe, die durch eine Alkylgruppe mono- oder disubstituiert sein kann, wobei die Substituenten gleich oder verschieden sein können,

$G_2$ eine Bindung oder eine durch $R_7$ und $R_8$ substituierte Methylengruppe,

$G_3$ eine durch $R_9$ und $R_{10}$ substituierte Methylengruppe,

$G_4$ eine Bindung,

$G_5$ ein Stickstoffatom oder eine gegebenenfalls durch eine Alkylgruppe substituierte Methingruppe,

$R_2$ ein Wasserstoffatom oder eine Alkylgruppe,

$R_3$ ein Wasserstoffatom oder eine Alkylgruppe,

$R_4$ ein Wasserstoffatom, eine Cycloalkyl- oder Cycloalkylalkylgruppe mit jeweils 3 bis 7 Kohlenstoffatomen im Cycloalkylteil, eine Alkylgruppe mit 1 bis 6 Kohlenstoffatomen, eine Alkenylgruppe mit 3 bis 6 Kohlenstoffatomen, wobei die Alkenylgruppe nicht über den Vinylteil mit dem Stickstoffatom verbunden sein kann, eine Hydroxyalkyl-, Alkoxyalkyl-, Carboxyalkyl-, Alkoxycarbonylalkyl-, Aminocarbonylalkyl-, N-Alkylaminocarbonylalkyl-, N,N-Dialkylaminocarbonylalkyl-, Arylalkyl-, Alkoxycarbonyl-, Arylmethyloxycarbonyl-, Formyl-, Acetyl-, Trifluoracetyloder $R_{11}CO\text{-}O\text{-}(R_{12}CR_{13})\text{-}O\text{-}CO$-Gruppe, in welcher

$R_{11}$ eine Alkylgruppe,

$R_{12}$ ein Wasserstoffatom oder eine Alkylgruppe und

$R_{13}$ ein Wasserstoffatom darstellen,

oder $R_4$ zusammen mit $R_3$ eine geradkettige Alkylengruppe mit 2 oder 3 Kohlenstoffatomen,

$R_5$ ein Wasserstoffatom oder eine Alkylgruppe oder auch, falls $G_1$ eine Bindung darstellt, $R_4$ zusammen mit $R_5$ eine weitere Bindung,

$R_6$ ein Wasserstoffatom oder eine Alkylgruppe oder, falls $G_1$ eine Bindung und $R_4$ zusammen mit $R_5$ eine weitere Bindung darstellen, eine Aminogruppe,

$R_7$ ein Wasserstoffatom oder eine Alkylgruppe,

$R_8$ ein Wasserstoffatom oder eine Alkylgruppe oder $R_8$ zusammen mit $R_4$ eine geradkettige Alkylengruppe mit 3 oder 4 Kohlenstoffatomen,

$R_9$ ein Wasserstoffatom oder eine Alkylgruppe,

$R_{10}$ ein Wasserstoffatom oder eine Alkylgruppe oder $R_{10}$ zusammen mit $R_4$ eine geradkettige Alkylengruppe mit 3 oder 4 Kohlenstoffatomen,

$R_{14}$ ein Wasserstoffatom oder eine Alkylgruppe,

$R_{15}$ ein Wasserstoffatom oder eine Alkylgruppe,

$R_{16}$ ein Wasserstoffatom oder eine Alkylgruppe,

$R_{17}$ ein Wasserstoffatom oder eine Alkylgruppe oder $R_{16}$ zusammen mit $R_{17}$ eine weitere Bindung,

$R_{18}$ ein Wasserstoffatom oder eine Alkylgruppe oder auch, wenn $R_{16}$ und $R_{17}$ zusammen eine weitere Bindung darstellen, ein Chloratom oder eine Aminogruppe,

n die Zahl 1 oder 2 darstellen, und

B eine Bindung,

eine Alkylengruppe,

eine Arylengruppe,

eine gegebenenfalls durch eine oder zwei Alkylgruppen substituierte Pyridinylen-, Pyrimidinylen-, Pyrazinylen- oder Pyridazinylengruppe,

eine gegebenenfalls durch eine oder zwei Alkylgruppen substituierte Cyclohexylengruppe,

eine gegebenenfalls durch eine oder zwei Alkylgruppen substituierte Piperidinylengruppe, in der eine zu einem Stickstoffatom benachbarte Methylengruppe durch eine Carbonylgruppe ersetzt sein kann,

der zweite der Reste $R_a$ bis $R_d$ eine Gruppe der Formel

F - E - D -,

in der

D eine Alkylengruppe,

eine Arylengruppe,

eine gegebenenfalls durch eine oder zwei Alkylgruppen substituierte Pyridinylen-, Pyrimidinylen-, Pyrazinylen- oder Pyridazinylengruppe,

eine Indanylen-, Naphthylen-, 1,2,3,4-Tetrahydronaphthylenoder Benzosuberanylengruppe, in denen jeweils einer der Ringe an den Rest E und der andere der Ringe an den cyclischen Rest der allgemeinen Formel I gebunden ist,

eine gegebenenfalls durch eine oder zwei Alkylgruppen substituierte Cycloalkylengruppe mit 4 bis 7 Kohlenstoffatomen,

eine gegebenenfalls durch eine oder zwei Alkylgruppen substituierte Cycloalkylengruppe mit 5 bis 7 Kohlenstoffatomen, in der eine >CH-Einheit durch ein Stickstoffatom ersetzt ist, wobei außerdem in den vorstehend erwähnten 5- bis 7-gliedrigen Ringen jeweils eine zu einem Stickstoffatom benachbarte Methylengruppe durch eine Carbonylgruppe ersetzt sein kann,

oder, wenn E eine cyclische Iminogruppe darstellt, auch eine Alkylencarbonylgruppe, wobei die Carbonylgruppe jeweils an das Stickstoffatom der cyclischen Iminogruppe der Gruppe E gebunden ist,

oder auch, falls E keine Bindung darstellt, eine Bindung,

E eine Bindung,

eine Alkylengruppe, die durch eine Alkylgruppe mit 1 bis 6 Kohlenstoffatomen, durch eine Amino-, Aryl-, Alkylamino-, Dialkylamino-, $HNR_{21}$- oder N-Alkyl-$NR_{21}$-Gruppe substituiert sein kann, wobei

$R_{21}$ eine Alkylcarbonyl- oder Alkylsulfonylgruppe mit jeweils 1 bis 6 Kohlenstoffatomen im Alkylteil, eine Alkyloxycarbonylgruppe mit insgesamt 2 bis 5 Kohlenstoffatomen, eine Cycloalkylcarbonyl- oder Cycloalkylsulfonylgruppe mit jeweils 5 bis 7 Kohlenstoffatomen im Cycloalkylteil, eine Arylalkylcarbonyl-, Arylalkylsulfonyl-, Arylalkoxycarbonyl-, Arylcarbonyl- oder Arylsulfonylgruppe darstellt,

eine Alkenylengruppe mit 2 bis 4 Kohlenstoffatomen,

eine Arylengruppe,

eine gegebenenfalls durch eine oder zwei Alkylgruppen substituierte Pyridinylen-, Pyrimidinylen-, Pyrazinylen- oder Pyridazinylengruppe,

eine gegebenenfalls durch eine oder zwei Alkylgruppen substituierte Cycloalkylengruppe mit 5 bis 7 Kohlenstoffatomen, in der eine >CH-Einheit durch ein Stickstoffatom, welches mit einem Kohlenstoffatom des Restes D verknüpft ist, ersetzt ist,

eine gegebenenfalls durch eine oder zwei Alkylgruppen substituierte Cycloalkylengruppe mit 4 bis 7 Kohlenstoffatomen im Cycloalkylenteil,

oder auch, falls D keine Bindung darstellt, eine über den Rest W mit dem Rest D verknüpfte Alkylengruppe, in der W ein Sauerstoff- oder Schwefelatom, eine Sulfinyl-, Sulfonyl-, -$NR_{20}$-CO-oder -CO-$NR_{20}$-Gruppe darstellt, wobei $R_{20}$ ein Wasserstoffatom oder eine Alkylgruppe darstellt und die Alkylengruppe zusätzlich durch eine Alkylgruppe mit 1 bis 6 Kohlenstoffatomen, durch eine Amino-, Aryl-, Alkylamino-, Dialkylamino-, -$HNR_{21}$- oder N-Alkyl-$NR_{21}$-Gruppe substituiert sein kann, wobei das Heteroatom des zusätzlichen Substituenten durch mindestens 2 Kohlenstoffatome von einem Heteroatom des Restes W getrennt ist und $R_{21}$ wie vorstehend definiert ist, und

F eine Carbonylgruppe, die durch eine Hydroxy-, Alkoxy-, Arylalkoxy- oder $R_{22}$O-Gruppe substituiert ist, wobei

$R_{22}$ eine Cycloalkylgruppe mit 5 bis 7 Kohlenstoffatomen oder eine Cycloalkylalkylgruppe mit 5 bis 7 Kohlenstoffatomen im Cycloalkylteil darstellt,

eine $R_{23}$CO-O-$CHR_{24}$-O-CO-, Phosphono- oder O-Alkyl-phosphonogruppe darstellen, wobei

$R_{23}$ eine Alkyl-, Alkoxy-, Cycloalkyl- oder Cycloalkoxygruppe mit jeweils 5 bis 7 Kohlenstoffatomen im Cycloalkylteil und

$R_{24}$ ein Wasserstoffatom oder eine Alkylgruppe darstellen,

und der kürzeste Abstand zwischen dem Rest F und dem von dem Rest F am weitesten entfernten Stickstoffatom der Gruppe A-B-mindestens 11 Bindungen beträgt,

der dritte der Reste $R_a$ bis $R_d$ ein Wasserstoffatom, eine Alkoxygruppe, wobei die Alkoxygruppe nicht an ein Stickstoffatom gebunden sein kann, eine Alkyl-, Trifluormethyl- oder Arylgruppe und

der vierte der Reste $R_a$ bis $R_d$ ein Wasserstoffatom oder eine Alkylgruppe bedeuten,

wobei, soweit nichts anderes erwähnt wurde,

unter den bei der Definition der vorstehenden Reste erwähnten Arylteilen eine Phenylgruppe, die jeweils durch $R_{25}$ monosubstituiert, durch $R_{26}$ mono-, di- oder trisubstituiert oder durch $R_{25}$ monosubstituiert und zusätzlich durch $R_{26}$ mono- oder disubstituiert sein kann, wobei die Substituenten gleich oder verschieden sein können und

$R_{25}$ eine Cyano-, Aminocarbonyl-, Alkylaminocarbonyl-, Dialkylaminocarbonyl-, Alkylcarbonyl-, Alkylsulfenyl-, Alkylsulfinyl-, Alkylsulfonyl- oder Trifluormethylgruppe und

$R_{26}$ eine Alkyl-, Hydroxy- oder Alkoxygruppe, ein Fluor-, Chloroder Bromatom darstellen, wobei zwei Reste $R_{26}$ sofern diese an benachbarte Kohlenstoffatome gebunden sind, auch eine geradkettige Alkylengruppe mit 3 oder 4 Kohlenstoffatomen, eine 1,3-Butadien-1,4-diylengruppe oder eine Methylendioxygruppe darstellen können,

unter den bei der Definition der vorstehenden Reste erwähnten Arylenteilen eine Phenylengruppe die jeweils durch $R_{25}$ monosubstituiert, durch $R_{26}$ mono- oder disubstituiert oder durch $R_{25}$ monosubstituiert und zusätzlich durch $R_{26}$ monosubstituiert sein kann, wobei die Substituenten gleich oder verschieden sein können und

wie vorstehend erwähnt definiert sind,

zu verstehen ist, sowie, soweit nichts anderes erwähnt wurde, die vorstehend erwähnten Alkyl-, Alkylen- oder Alkoxyteile jeweils 1 bis 4 Kohlenstoffatome enthalten können, und jedes Kohlenstoffatom in den vorstehend erwähnten Alkylen- und Cycloalkylenteilen höchstens mit einem Heteroatom verknüpft ist,

deren Tautomere, deren Stereoisomere und Salze.

**2.** Cyclische Derivate der allgemeinen Formel I gemäß Anspruch 1, in der

X eine Carbonyl- oder Sulfonylgruppe,

Y eine gegebenenfalls durch eine oder zwei Methylgruppen substituierte $-CH_2CH_2-$, $-CH_2CH_2CH_2-$, -CH=CH-, $-CH_2CO-$ oder $-COCH_2-$Gruppe,

eine gegebenenfalls durch $R_c$ substituierte -CO-NH-, -CH=N- oder -N=CH-Gruppe,

einer der Reste $R_a$ bis $R_c$ eine A-B-Gruppe, in der

A eine Gruppe der Formeln

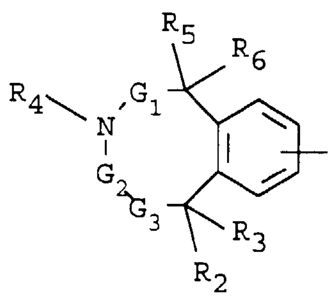

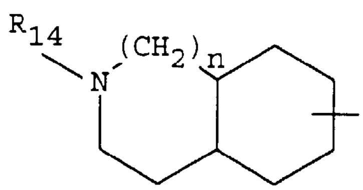

oder

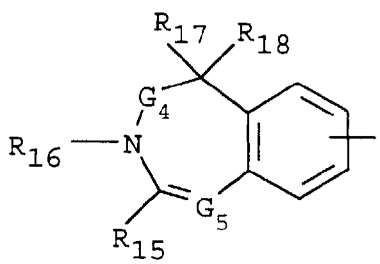

darstellt, wobei jeweils im Benzoteil der vorstehend erwähnten Gruppen eine oder zwei Methingruppen jeweils durch ein Stickstoffatom ersetzt sein können,

$G_1$ eine Bindung oder eine Methylengruppe,

$G_2$ eine Bindung,

$G_3$ eine Methylengruppe,

$G_4$ eine Bindung,

$G_5$ ein Stickstoffatom oder eine Methingruppe,

$R_2$ ein Wasserstoffatom,

$R_3$ ein Wasserstoffatom,

$R_4$ ein Wasserstoffatom, eine Cyclopropyl- oder Cyclopropylmethylgruppe, eine Alkylgruppe mit 1 bis 6 Kohlenstoffatomen, eine Allyl-, Hydroxyalkyl-, Carboxyalkyl-, Alkoxycarbonylalkyloder Benzylgruppe,

$R_5$ ein Wasserstoffatom,

$R_6$ ein Wasserstoffatom,

$R_{14}$ ein Wasserstoffatom oder eine Alkylgruppe,

$R_{15}$ ein Wasserstoffatom oder eine Alkylgruppe,

$R_{16}$ ein Wasserstoffatom oder eine Alkylgruppe,

$R_{17}$ ein Wasserstoffatom oder $R_{16}$ zusammen mit $R_{17}$ eine weitere Bindung,

$R_{18}$ ein Wasserstoffatom oder auch, wenn $R_{16}$ und $R_{17}$ zusammen eine weitere Bindung darstellen, eine Aminogruppe,

n die Zahl 1 oder 2 darstellen, und

B eine Bindung oder eine Phenylengruppe,

der zweite der Reste $R_a$ bis $R_c$ eine Gruppe der Formel

$$F - E - D -,$$

in der

D eine Alkylengruppe,

eine Phenylengruppe,

eine Cyclohexylengruppe oder

eine Piperidinylengruppe, wobei das Ringstickstoffatom mit der gegebenenfalls substituierten geradkettigen Alkylengruppe des Restes E verknüpft ist, oder

eine Bindung,

E eine geradkettige Alkylengruppe, die durch eine Alkyl- oder Phenylgruppe substituiert sein kann,

eine Alkenylengruppe mit 2 bis 4 Kohlenstoffatomen,

eine Phenylengruppe,

oder auch, falls D keine Bindung darstellt, eine über das Sauerstoffatom mit dem Rest D verknüpfte geradkettige 0-Alkylengruppe und

F eine Carbonylgruppe, die durch eine Hydroxy- oder Alkoxygruppe substituiert ist, darstellen

und der kürzeste Abstand zwischen dem Rest F und dem von dem Rest F am weitesten entfernten Stickstoffatom der Gruppe A-B-mindestens 11 Bindungen beträgt, und

der dritte der Reste $R_a$ bis $R_c$ ein Wasserstoffatom, eine Alkoxygruppe, wobei die Alkoxygruppe nicht an ein Stickstoffatom gebunden sein kann, eine Alkyl- oder Phenylgruppe bedeuten,

wobei, soweit nichts anderes erwähnt wurde, die vorstehend erwähnten Alkyl-, Alkylen- oder Alkoxyteile jeweils 1 bis 4 Kohlenstoffatome enthalten können, und jedes Kohlenstoffatom in den vorstehend erwähnten Alkylen- und Cycloalkylenteilen höchstens mit einem Heteroatom verknüpft ist,

deren Tautomere, deren Stereoisomere und Salze.

**3.** Cyclische Derivate der allgemeinen Formel I gemäß Anspruch 1, in der

X eine Carbonylgruppe,

Y eine $-CH_2CH_2-$, $-CH_2CH_2CH_2-$, $-CH=CH-$, $-CH_2CO-$ oder $-COCH_2-$Gruppe oder eine gegebenenfalls durch eine Methylgruppe substituierte $-N=CH-$Gruppe,

der Rest $R_a$ eine A-B-Gruppe, in der

A eine Gruppe der Formeln

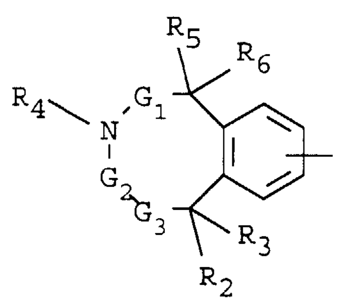

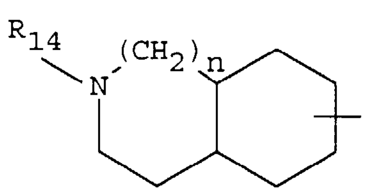

oder

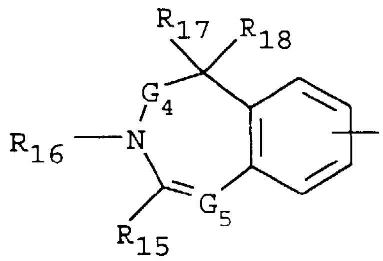

darstellt, wobei jeweils im Benzoteil der vorstehend erwähnten Gruppen eine oder zwei Methingruppen jeweils durch ein Stickstoffatom ersetzt sein können,

$G_1$ eine Bindung oder eine Methylengruppe,

$G_2$ eine Bindung,

$G_3$ eine Methylengruppe,

$G_4$ eine Bindung,

$G_5$ eine Methingruppe,

$R_2$ ein Wasserstoffatom,

$R_3$ ein Wasserstoffatom,

$R_4$ ein Wasserstoffatom, eine Alkylgruppe mit 1 bis 4 Kohlenstoffatomen, eine Allyl- oder Benzylgruppe,

$R_5$ ein Wasserstoffatom,

$R_6$ ein Wasserstoffatom,

$R_{14}$ ein Wasserstoffatom oder eine Methylgruppe,

$R_{15}$ ein Wasserstoffatom,

$R_{16}$ zusammen mit $R_{17}$ eine Bindung,

$R_{18}$ ein Wasserstoffatom oder eine Aminogruppe und n die Zahl 1 darstellen,

B eine Bindung,

der Rest $R_b$ eine Gruppe der Formel

F - E - D -,

in der

D eine $-CH_2CH_2-$Gruppe,

eine 1,4-Phenylengruppe oder

eine 1,4-Cyclohexylengruppe,

E eine gegebenenfalls durch eine Methylgruppe substituierte $-CH_2CH_2-$Gruppe, eine -CH=CH-, 1,4-Phenylen-

oder -O-$CH_2$-Gruppe, wobei das Sauerstoffatom der -O-$CH_2$-Gruppe mit dem Rest D verknüpft ist, und

F eine Carbonylgruppe, die durch eine Hydroxy- oder Alkoxygruppe mit 1 bis 4 Kohlenstoffatomen substituiert ist, bedeuten

und der kürzeste Abstand zwischen dem Rest F und dem von dem Rest F am weitesten entfernten Stickstoffatom der Gruppe A-B-mindestens 11 Bindungen beträgt,

deren Tautomere, deren Stereoisomere und Salze.

**4.** Folgende Verbindungen der allgemeinen Formel I gemäß Anspruch 1:

(a) 1-[4-[2-(Methoxycarbonyl)ethyl]phenyl]-3-(1,2,3,4-tetrahydroisochinolin-6-yl)-imidazolidin-2-on,

(b) 1-[4-(2-Carboxyethyl)phenyl]-3-(1,2,3,4-tetrahydroisochinolin-6-yl)-imidazolidin-2-on,

(c) 1-[4-(2-Carboxyethyl)phenyl]-3-(2-methyl-1,2,3,4-tetrahydroisochinolin-6-yl)-imidazolidin-2-on,

(d) 1-[4-[2-(Isobutyloxycarbonyl)ethyl]phenyl]-3-(1,2,3,4-tetrahydroisochinolin-6-yl)-imidazolidin-2-on,

(e) 1-[4-(2-Carboxyethyl)phenyl]-3-(2,3,4,5-tetrahydro-1H-3-benzazepin-7-yl)-imidazolidin-2-on,

(f) 1-[4-[2-(Methoxycarbonyl)ethyl]phenyl]-3-(2,3,4,5-tetrahydro-1H-3-benzazepin-7-yl)-imidazolidin-2-on,

(g) 1-[4-[2-(Isopropyloxycarbonyl)ethyl]phenyl]-3-(2,3,4,5-tetrahydro-1H-3-benzazepin-7-yl)-imidazolidin-2-on,

(h) 1-[4-(2-Carboxyethyl)phenyl]-3-(3-methyl-2,3,4,5-tetrahydro-1H-3-benzazepin-7-yl)-imidazolidin-2-on,

(i) 4-[4-[2-(Carboxyethyl)phenyl]-5-methyl-2-(2,3,4,5-tetrahydro-1H-3-benzazepin-7-yl)-4H-1,2,4-triazol-3-on,

(j) 1-[trans-4-(2-Carboxyethyl)cyclohexyl]-3-(2,3,4,5-tetrahydro-1H-3-benzazepin-7-yl)-imidazolidin-2-on,

(k) 1-[4-[2-(Methoxycarbonyl)ethyl]phenyl]-3-(3-methyl-2,3,4, 5-tetrahydro-1H-3-benzazepin-7-yl)-imidazolidin-2-on,

(l) 1-[4-[2-(Ethoxycarbonyl)ethyl]phenyl]-3-(3-methyl-2,3,4,5-tetrahydro-1H-3-benzazepin-7-yl)-imidazolidin-2-on,

(m) 1-[4-[2-(Isopropyloxycarbonyl)ethyl]phenyl]-3-(3-methyl-2,3,4,5-tetrahydro-1H-3-benzazepin-7-yl)-imidazolidin-2-on,

(n) 1-[trans-4-(2-Carboxyethyl)cyclohexyl]-3-(3-methyl-2,3,4, 5-tetrahydro-1H-3-benzazepin-7-yl)-imidazolidin-2-on,

(o) 1-[trans-4-[2-(Isopropyloxycarbonyl)ethyl]cyclohexyl]-3-(3-methyl-2,3,4,5-tetrahydro-1H-3-benzazepin-7-yl)-imidazolidin-2-on,

(p) 1-[trans-4-[2-(Ethoxycarbonyl)ethyl]cyclohexyl]-3-(3-methyl-2,3,4,5-tetrahydro-1H-3-benzazepin-7-yl)-imidazolidin-2-on,

(q) 1-[trans-4-[2-(Methoxycarbonyl)ethyl]cyclohexyl-3-(3-methyl-2,3,4,5-tetrahydro-1H-3-benzazepin-7-yl)-imidazolidin-2-on,

(r) 1-[trans-4-[(Carboxymethyl)oxy]cyclohexyl]-3-(3-methyl-2,3,4,5-tetrahydro-1H-3-benzazepin-7-yl)-imidazolidin-2-on,

(s) 3-[trans-4-(2-Carboxyethyl)cyclohexyl]-1-(3-methyl-2,3,4,5-tetrahydro-1H-3-benzazepin-7-yl)-hydantoin und

(t) 1-[trans-4-(2-Carboxyethyl)cyclohexyl]-3-(3-ethyl-2,3,4,5-tetrahydro-1H-3-benzazepin-7-yl)-imidazolidin-2-on,

deren Tautomere und deren Salze.

**5.** 1-[trans-4-(2-Carboxyethyl)cyclohexyl]-3-(3-methyl-2,3,4,5-tetrahydro-1H-3-benzazepin-7-yl)-imidazolidin-2-on, dessen Methyl-, Ethyl- und Isopropylester sowie deren Salze.

**6.** 1-[trans-4-(2-Carboxyethyl)cyclohexyl]-3-(3-methyl-2,3,4,5-tetrahydro-1H-3-benzazepin-7-yl)-imidazolidin-2-on und dessen Salze,

**7.** Physiologisch verträgliche Salze der Verbindungen nach mindestens einem der Ansprüche 1 bis 6 mit anorganischen oder organischen Säuren oder Basen.

**8.** Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

**9.** Verwendung einer Verbindung nach mindestens einem der Ansprüche 1 bis 7 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**10.** Verfahren zur Herstellung eines Arzneimittels gemäß Anspruch 8, dadurch gekennzeichnet, daß auf nichtchemischem Wege eine Verbindung nach mindestens einem der Ansprüche 1 bis 7 in einen oder mehrere inerte Trägerstoffe und/oder Verdünnungsmittel eingearbeitet wird.

**11.** Verfahren zur Herstellung der cyclischen Derivate gemäß den Ansprüchen 1 bis 7, dadurch gekennzeichnet, daß

a) zur Herstellung von Verbindungen der allgemeinen Formel I, in der F eine Carboxylgruppe darstellt, eine Verbindung der allgemeinen Formel

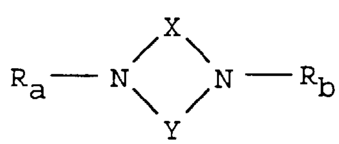

, (I)

in der
$R_a$, $R_b$, X und Y mit der Maßgabe wie in den Ansprüchen 1 bis 6 definiert sind, daß einer der Reste $R_a$ bis $R_d$ eine F'-E-D-Gruppe darstellt, in der
E und D wie in den Ansprüchen 1 bis 6 definiert sind und F' eine mittels Hydrolyse, Behandeln mit Säuren, Thermolyse oder Hydrogenolyse in eine Carboxylgruppe überführbare Gruppe bedeutet,
in eine Verbindung der allgemeinen Formel I, in der F eine Carboxylgruppe darstellt, mittels Hydrolyse, Behandeln mit Säuren, Thermolyse oder Hydrogenolyse übergeführt wird oder

b) zur Herstellung von Verbindungen der allgemeinen Formel I, in der $R_{16}$ und $R_{17}$ zusammen eine weitere Bindung, $G_4$ eine Bindung und $R_{18}$ eine Aminogruppe darstellen, eine Verbindung der allgemeinen Formel

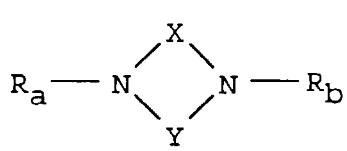

, (III)

in der

$R_a$ $R_b$, X und Y mit der Maßgabe wie in den Ansprüchen 1 bis 6 definiert sind, daß einer der Reste $R_a$ bis $R_d$ eine A-B-Gruppe darstellt, in der

B wie in den Ansprüchen 1 bis 6 erwähnt definiert ist und

A eine Gruppe der Formel

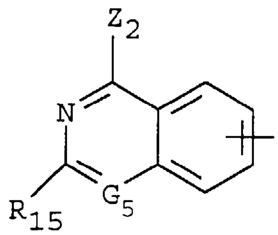

darstellt, wobei der Benzoteil, $R_{15}$ und $G_5$ wie in den Ansprüchen 1 bis 6 definiert sind und

$Z_2$ eine nukleophile Austrittsgruppe darstellt,

mit einer Verbindung der allgemeinen Formel

$$H - R_{27} , \qquad (IV)$$

in der
$R_{27}$ eine Aminogruppe darstellt, umgesetzt wird oder

c) zur Herstellung von Verbindungen der allgemeinen Formel I, in der $R_{16}$ und $R_{17}$ zusammen eine weitere Bindung, $G_4$ eine Bindung und $R_{18}$ ein Chloratom darstellen, eine Verbindung der allgemeinen Formel

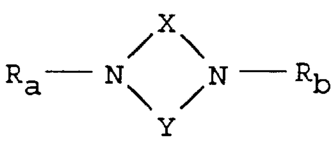

, (V)

in der

$R_a$, $R_b$, X und Y mit der Maßgabe wie in den Ansprüchen 1 bis 6 definiert sind, daß einer der Reste $R_a$ bis $R_d$ eine A-B-Gruppe darstellt, in der

B wie in den Ansprüchen 1 bis 6 erwähnt definiert ist und

A eine Gruppe der Formel

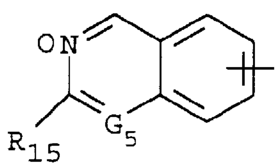

darstellt, wobei der Benzoteil, $R_{15}$ und $G_5$ wie in den Ansprüchen 1 bis 6 definiert sind, in Gegenwart eines Säurehalogenids umgesetzt wird oder

d) zur Herstellung von Verbindungen der allgemeinen Formel I, in der X eine durch eine Cyanogruppe substituierte Carbiminogruppe, eine Carbonyl- oder Sulfonylgruppe und Y eine gegebenenfalls durch $R_c$ oder $R_d$

oder $R_c$ und $R_d$ substituierte geradkettige Alkylengruppe mit 2 oder 3 Kohlenstoffatomen darstellt, eine Verbindung der allgemeinen Formel

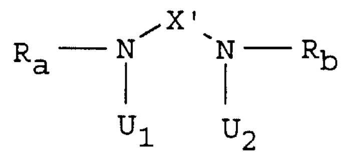

, (VI)

in der

$R_a$ und $R_b$ wie in den Ansprüchen 1 bis 6 definiert sind, X' eine durch eine Cyanogruppe substituierte Carbiminogruppe,
eine Carbonyl- oder Sulfonylgruppe,
einer der Reste $U_1$ oder $U_2$ ein Wasserstoffatom und

der andere der Reste $U_1$ oder $U_2$ eine gegebenenfalls durch $R_c$ oder $R_d$ oder $R_c$ und $R_d$ substituierte geradkettige Alkylengruppe mit 2 oder 3 Kohlenstoffatomen, an die zusätzlich endständig eine nukleophile Austrittsgruppe gebunden ist, cyclisiert wird oder

e) zur Herstellung von Verbindungen der allgemeinen Formel I, in der X eine Carbonylgruppe und Y eine der in den Ansprüchen 1 bis 6 erwähnten geradkettigen Alkylengruppen mit 2 oder 3 Kohlenstoffatomen darstellen, eine Verbindung der allgemeinen Formel

$$R_{28} - NH - T , \qquad (VII)$$

mit einem Isocyanat der allgemeinen Formel

$$O = C = N - R_{29} , \qquad (VIII)$$

in denen

einer der Reste $R_{28}$ oder $R_{29}$ die für $R_a$ in den Ansprüchen 1 bis 6 erwähnten Bedeutungen besitzt und der andere der Reste $R_{28}$ oder $R_{29}$ die für $R_b$ in den Ansprüchen 1 bis 6 erwähnten Bedeutungen besitzt und
T eine im Alkylidenteil gegebenenfalls durch $R_c$ oder $R_d$ oder durch $R_c$ und $R_d$ substituierte Gruppe der Formel

$$-(CH_2)_m - HC(OR_{30})_2$$

darstellt, wobei wobei m die Zahl 1 oder 2 und
$R_{30}$ jeweils eine Alkylgruppe mit 1 bis 4 Kohlenstoffatomen darstellen,
umgesetzt und gegebenenfalls eine so erhaltene Verbindung anschließend hydriert wird oder

f) zur Herstellung von Verbindungen der allgemeinen Formel I, in der $G_1$ und $G_2$ jeweils eine Bindung, $G_3$ eine gegebenenfalls durch eine Alkylgruppe mit 1 bis 4 Kohlenstoffatomen substituierte Methylengruppe, $R_2$, $R_4$ und $R_5$ jeweils ein Wasserstoffatom, $R_3$ und $R_6$, die gleich oder verschieden sein können, jeweils ein Wasserstoffatom oder eine Alkylgruppe mit 1 bis 4 Kohlenstoffatomen darstellen, eine Verbindung der allgemeinen Formel

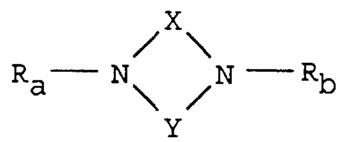 , (IX)

in der

$R_a$, $R_b$, X und Y mit der Maßgabe wie in den Ansprüchen 1 bis 6 definiert sind, daß einer der Reste $R_a$ bis $R_d$ eine A-B-Gruppe darstellt, in der

B wie in den Ansprüchen 1 bis 6 erwähnt definiert ist und

A eine Gruppe der Formel

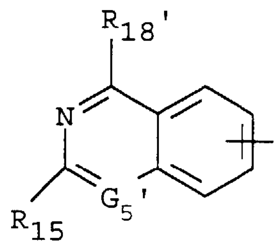

darstellt, wobei der Benzoteil und $R_{15}$ wie in den Ansprüchen 1 bis 6 definiert ist,

$G_5$' eine gegebenenfalls durch eine Alkylgruppe mit 1 bis 4 Kohlenstoffatomen substituierte Methingruppe und

$R_{18}$' ein Wasserstoffatom oder eine Alkylgruppe mit 1 bis 4 Kohlenstoffatomen darstellen, hydriert wird oder

g) zur Herstellung von Verbindungen der allgemeinen Formel I, in der F eine durch eine Alkoxygruppe mit 1 bis 4 Kohlenstoffatomen, durch eine Arylalkoxygruppe mit 1 bis 4 Kohlenstoffatomen im Alkoxyteil, wobei der Arylteil wie in den Ansprüchen 1 bis 6 erwähnt definiert ist, oder durch eine $R_{22}$O-Gruppe substituierte Carbonylgruppe darstellt, eine Verbindung der allgemeinen Formel

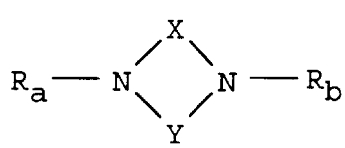 , (X)

in der

$R_a$ $R_b$, X und Y mit der Maßgabe wie in den Ansprüchen 1 bis 6 definiert sind, daß einer der Reste $R_a$ bis $R_d$ eine F"-E-D-Gruppe darstellt, in der

E und D wie in den Ansprüchen 1 bis 6 definiert sind und

F" eine Carboxy- oder Alkoxycarbonylgruppe darstellt,

mit einem Alkohol der allgemeinen Formel

$$HO - R_{31} ,\qquad (XI)$$

in der

$R_{31}$ die für $R_{22}$ in den Ansprüchen 1 bis 6 erwähnten Bedeutungen besitzt sowie eine Alkylgruppe mit 1 bis 4 Kohlenstoffatomen oder eine Arylalkylgruppe, in welcher der Arylteil wie in den Ansprüchen 1 bis 6 erwähnt definiert ist und der Alkylteil 1 bis 4 Kohlenstoffatome enthalten kann, darstellt, umgesetzt wird oder

h) zur Herstellung von Verbindungen der allgemeinen Formel I, in der $R_4$ einen der bei der Definition von $R_4$ in den Ansprüchen 1 bis 6 erwähnten gegebenenfalls substituierten Alkylreste, Alkenyl-, Cycloalkyl-, Cycloalkylalkyl- oder Aralkylreste darstellt, eine Verbindung der allgemeinen Formel

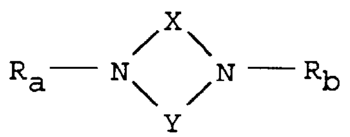

, (XI)

in der

$R_a$ $R_b$, X und Y mit der Maßgabe wie in den Ansprüchen 1 bis 6 definiert sind, daß einer der Reste $R_a$ bis $R_d$ eine A-B-Gruppe darstellt, in der

A und B mit der Maßgabe wie in den Ansprüchen 1 bis 6 definiert sind, daß $R_4$ ein Wasserstoffatom darstellt,

mit einer Verbindung der allgemeinen Formel

$$Z_3 - R_{32} ,\qquad (XIII)$$

in der

$R_{32}$ eine Alkylgruppe mit 1 bis 6 Kohlenstoffatomen, eine Cycloalkyl- oder Cycloalkylalkylgruppe, in denen der Cycloalkylteil jeweils 3 bis 7 Kohlenstoffatome und der Alkylteil 1 bis 4 Kohlenstoffatome enthalten kann, eine Alkenylgruppe mit 3 bis 6 Kohlenstoffatomen, eine Arylalkyl-, Hydroxyalkyl-, Alkoxyalkyl-, Carboxyalkyl-, Alkoxycarbonylalkyl-, Aminocarbonylalkyl-, N-Alkylaminocarbonylalkyl- oder N,N-Dialkylaminocarbonylgruppe, in denen der Arylteil und die Alkylteile wie in den Ansprüchen 1 bis 6 definiert sind, und
$Z_3$ eine nukleophile Austrittsgruppe oder
$Z_3$ zusammen mit einem benachbarten Wasserstoffatom des Restes $R_{32}$ ein Sauerstoffatom bedeuten, gegebenenfalls in Gegenwart eines Reduktionsmittels umgesetzt wird oder

i) zur Herstellung von Verbindungen der allgemeinen Formel I, in der X eine am Stickstoffatom durch eine Cyanogruppe substituierte Carbiminogruppe, eine Carbonyl- oder Sulfonylgruppe darstellt, eine Verbindung der allgemeinen Formel

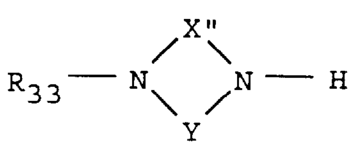

, (XIV)

mit einer Verbindung der allgemeinen Formel

$$Z_4 - R_{34} ,\qquad (XV)$$

in denen

Y wie in den Ansprüchen 1 bis 6 definiert ist,
X" eine am Stickstoffatom durch eine Cyanogruppe substituierte Carbiminogruppe, eine Carbonyl- oder Sulfonylgruppe,
einer der Reste $R_{33}$ oder $R_{34}$ die für $R_a$ in den Ansprüchen 1 bis 6 erwähnten Bedeutungen besitzt und der andere der Reste $R_{33}$ oder $R_{34}$ die für $R_b$ in den Ansprüchen 1 bis 6 erwähnten Bedeutungen besitzt und
$Z_4$ eine nukleophile Austrittsgruppe bedeuten, umgesetzt wird oder

j) zur Herstellung von Verbindungen der allgemeinen Formel I, in der $R_4$ eine Alkoxycarbonyl-, Arylmethyloxycarbonyl-, Formyl-, Acetyl-, Trifluoracetyl- oder $R_{11}$CO-O- ($R_{12}CR_{13}$)-O-CO-Gruppe, in denen $R_{11}$ bis $R_{13}$ und der Arylteil wie in den Ansprüchen 1 bis 6 definiert sind und der Alkoxyteil 1 bis 4 Kohlenstoffatome enthalten kann, darstellt, eine Verbindung der allgemeinen Formel

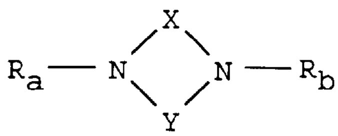
, (XVI)

in der

$R_a$ $R_b$, X und Y mit der Maßgabe wie in den Ansprüchen 1 bis 6 definiert sind, daß $R_4$ ein Wasserstoffatom darstellt, mit einer Verbindung der allgemeinen Formel

$$Z_5 - R_{35} , \qquad (XVII)$$

in der
$R_{35}$ eine Alkoxycarbonylgruppe mit insgesamt 2 bis 5 Kohlenstoffatomen, eine Arylmethyloxycarbonylgruppe, in der der Arylteil wie in den Ansprüchen 1 bis 6 definiert ist, eine $R_{11}$CO-O-($R_{12}CR_{13}$)-O-CO-, Formyl-, Acetyl- oder Trifluoracetylgruppe, wobei $R_{11}$ bis $R_{13}$ wie in den Ansprüchen 1 bis 6 definiert sind, und
$Z_5$ eine nukleophile Austrittsgruppe darstellen, umgesetzt wird oder
k) zur Herstellung von Verbindungen der allgemeinen Formel I, in der F eine Carbonylgruppe darstellt, die durch eine Alkoxygruppe mit 1 bis 4 Kohlenstoffatomen, durch eine Arylalkoxygruppe, in der der Arylteil wie in den Ansprüchen 1 bis 6 erwähnt definiert ist und der Alkoxyteil 1 bis 4 Kohlenstoffatome enthalten kann, durch eine $R_{22}$O- oder $R_{23}$CO-O-CH$R_{24}$-O-Gruppe substituiert ist, wobei $R_{22}$ bis $R_{24}$ wie in den Ansprüchen 1 bis 6 erwähnt definiert sind, eine Verbindung der allgemeinen Formel

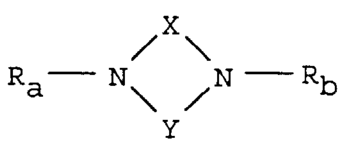
, (XVIII)

in der

$R_a$, $R_b$, X und Y mit der Maßgabe wie in den Ansprüchen 1 bis 6 definiert sind, daß einer der Reste $R_a$ bis $R_d$ eine F'''-E-D-Gruppe darstellt, in der

E und D wie in den Ansprüchen 1 bis 6 definiert sind und

F''' eine Carboxylgruppe darstellt,

mit einer Verbindung der allgemeinen Formel

$$Z_6 - R_{36} , \qquad (XIX)$$

in der

$R_{36}$ eine Alkylgruppe mit 1 bis 4 Kohlenstoffatomen, eine Arylalkylgruppe, in der der Arylteil wie in den Ansprüchen 1 bis 6 erwähnt definiert ist und der Alkylteil 1 bis 4 Kohlenstoffatome enthalten kann, eine $R_{22}$- oder $R_{23}$CO-O-CH$R_{24}$-Gruppe, wobei $R_{22}$ bis $R_{24}$ wie in den Ansprüchen 1 bis 6 erwähnt definiert sind, und
$Z_6$ eine nukleophile Austrittsgruppe bedeuten, umgesetzt wird oder

l) zur Herstellung von Verbindungen der allgemeinen Formel I, in der X eine Carbonylgruppe und Y eine gegebenenfalls durch $R_c$ oder $R_d$ oder $R_c$ und $R_d$ substituierte Ethylengruppe darstellt, in der eine Methylengruppe durch eine Carbonylgruppe ersetzt ist, eine gegebenenfalls im Reaktionsgemisch gebildete Verbindung der allgemeinen Formel

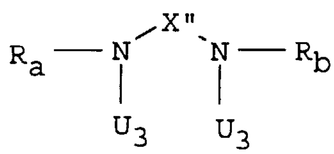
, (XX)

in der

$R_a$ und $R_b$ wie in den Ansprüchen 1 bis 6 definiert sind,
X" eine Carbonylgruppe,
einer der Reste $U_3$ oder $U_4$ ein Wasserstoffatom und der andere der Reste $U_3$ oder $U_4$ eine gegebenenfalls durch $R_c$ oder $R_d$ oder $R_c$ und $R_d$ substituierte Ethylengruppe, in der eine Methylengruppe durch eine $Z_7$-CO-Gruppe ersetzt ist, wobei $Z_7$ eine nukleophile Austrittsgruppe darstellt, cyclisiert wird und

gewünschtenfalls anschließend eine so erhaltene Verbindung der allgemeinen Formel I, welche eine ungesättigte Kohlenstoff-Kohlenstoff-Bindung enthält, mittels katalytischer Hydrierung in eine entsprechende gesättigte Verbindung der allgemeinen Formel I übergeführt wird und/oder

erforderlichenfalls ein während den Umsetzungen zum Schutze von reaktiven Gruppen verwendeter Schutzrest abgespalten wird und/oder
eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

**Claims**

**1.** Cyclic derivatives of general formula,

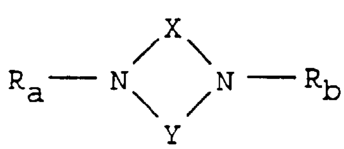
, (I)

wherein

X denotes a carbimino group substituted at the nitrogen atom by a cyano group, or a carbonyl, or a sulphonyl group,

Y denotes a $-CH_2CH_2-$, $-CH_2CH_2CH_2-$, -CH=CH-, $-CH_2CO-$ or $-COCH_2-$ group which is optionally substituted by $R_c$ or by $R_c$ and $R_d$,

or a -CO-NH-, -NH-CO-, -CH=N- or -N=CH- group optionally substituted by $R_c$ or $R_d$;

one of the groups $R_a$ to $R_d$, denotes an A-B group wherein

A is a group of one of the formulae

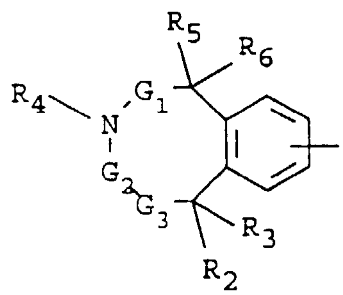

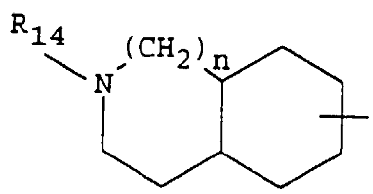

or

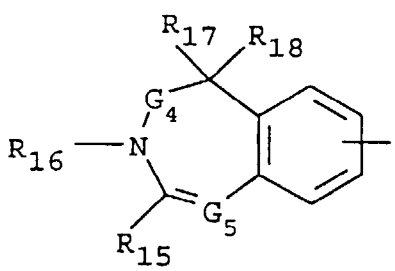

wherein the benzo moiety of the above-mentioned groups may each be substituted by a fluorine, chlorine or bromine atom, or by an alkyl, cyano, trifluoromethyl, hydroxy or alkoxy group, or one to three methine groups may each be replaced a nitrogen atom,

$G_1$ represents a bond or a methylene group which may be mono or disubstituted by an alkyl group, wherein the substituents may be identical or different,

$G_2$ denotes a bond or a methylene group substituted by $R_7$ and $R_8$,

$G_3$ denotes a methylene group substituted by $R_9$ and $R_{10}$,

$G_4$ denotes a bond,

$G_5$ denotes a nitrogen atom or a methine group optionally substituted by an alkyl group,

$R_2$ denotes a hydrogen atom or an alkyl group,

$R_3$ denotes a hydrogen atom or an alkyl group,

$R_4$ denotes a hydrogen atom, a cycloalkyl or cycloalkylalkyl group each having 3 to 7 carbon atoms in the cycloalkyl moiety, a $C_{1-6}$-alkyl group, a $C_{3-6}$-alkenyl group (wherein the alkenyl group may not be linked to the nitrogen atom via the vinyl moiety), or a hydroxyalkyl, alkoxyalkyl, carboxyalkyl, alkoxycarbonylalkyl, aminocarbonylalkyl, N-alkylaminocarbonylalkyl, N,N-dialkylaminocarbonylalkyl, arylalkyl, alkoxycarbonyl, arylmethyloxycarbonyl, formyl, acetyl, trifluoroacetyl, or $R_{11}CO\text{-}O\text{-}(R_{12}CR_{13})$ -O-CO group, wherein

$R_{11}$ denotes an alkyl group,

$R_{12}$ denotes a hydrogen atom or an alkyl group, and

$R_{13}$ denotes a hydrogen atom,

or $R_4$ together with $R_3$ may denote a straight chain alkylene group having 2 or 3 carbon atoms,

$R_5$ denotes a hydrogen atom or an alkyl group, or, if $G_1$ denotes a bond, $R_4$ together with $R_5$ may denote another bond,

$R_6$ denotes a hydrogen atom or an alkyl group, or, if $G_1$ denotes a bond and $R_4$ together with $R_5$ denotes another bond, $R_6$ may also denote an amino group,

$R_7$ denotes a hydrogen atom or an alkyl group,

$R_8$ denotes a hydrogen atom or an alkyl group, or $R_8$ together with $R_4$ may denote a straight chain alkylene group having 3 or 4 carbon atoms,

$R_9$ denotes a hydrogen atom or an alkyl group,

$R_{10}$ denotes a hydrogen atom or an alkyl group, or $R_{10}$ together with $R_4$ denotes a straight chain alkylene group having 3 to 4 carbon atoms,

$R_{14}$ denotes a hydrogen atom or an alkyl group,

$R_{15}$ denotes a hydrogen atom or an alkyl group,

$R_{16}$ denotes a hydrogen atom or an alkyl group,

$R_{17}$ denotes a hydrogen atom or an alkyl group or $R_{16}$ together with $R_{17}$ may denote another bond,

$R_{18}$ denotes a hydrogen atom or an alkyl group or, if $R_{16}$ and $R_{17}$ together denote another bond, $R_{18}$ may denote a chlorine atom or an amino group,

n represents the number 1 or 2, and

B denotes a bond,

or an alkylene group,

or an arylene group,

or a pyridinylene, pyrimidinylene, pyrazinylene, or pyridazinylene group, which may optionally be substituted by one or two alkyl groups,

or a cyclohexylene group optionally substituted by one or two alkyl groups,

or a piperidinylene group which is optionally substituted by one or two alkyl groups, and in which a methylene group adjacent to a nitrogen atom may be replaced by a carbonyl group,

the second of the groups $R_a$ to $R_d$ denotes a group of formula

F - E - D-

wherein

D denotes an alkylene group,

or an arylene group,

or a pyridinylene, pyrimidinylene, pyrazinylene or pyridazinylene group, which may optionally be substituted by one or two alkyl groups,

or an indanylene, naphthylene, 1,2,3,4-tetrahyronaphthylene or benzosuberanylene group wherein one of the rings is bound to the group E and the other ring is bound to the cyclic group of general formula I,

or a $C_{4-7}$-cycloalkylene group optionally substituted by one or two alkyl groups,

or a $C_{5-7}$-cycloalkylene group which is optionally substituted by one or two alkyl groups, and in which a >CH unit is replaced by a nitrogen atom, whilst moreover in the above-mentioned 5 to 7 membered rings a methylene group adjacent to a nitrogen atom may be replaced by a carbonyl group,

or, if E denotes a cyclic imino group, D may also denote an alkylenecarbonyl group, wherein the carbonyl group is bound to the nitrogen atom of the cyclic imino group of group E,

or, if E does not represent a bond, D may represent a bond,

E denotes a bond,

or an alkylene group which may be substituted by a $C_{1-6}$-alkyl group, by an amino, aryl, alkylamino, dialkylamino group, or by an $HNR_{21}$ or N-alkyl-$NR_{21}$- group,

$R_{21}$ denotes an alkylcarbonyl or alkylsulphonyl group each having 1 to 6 carbon atoms in the alkyl moiety, an alkyloxycarbonyl group having a total of 2 to 5 carbon atoms, a cycloalkylcarbonyl or cycloalkylsulphonyl group each having 5 to 7 carbon atoms in the cycloalkyl moiety, or an arylalkylcarbonyl, arylalkylsulphonyl, arylalkoxycarbonyl, arylcarbonyl or arylsulphonyl group,

or a $C_{2-4}$ alkenylene group,

or an arylene group,

or a pyridinylene, pyrimidinylene, pyrazinylene or pyridazinylene group optionally substituted by one or two alkyl groups.

or a $C_{5-7}$-cycloalkylene group which is optionally substituted by one or two alkyl groups, and in which a >CH unit is replaced by a nitrogen atom linked to a carbon atom of the group D,

or a cycloalkylene group having 4 to 7 carbon atoms in the cycloalkylene moiety and which may optionally be substituted by one or two alkyl groups,

or, if D does not denote a bond, E may also denote an alkylene group linked to group D via a group W, wherein W denotes an oxygen or sulphur atom or a sulphinyl, sulphonyl, $-NR_{20}$-CO-, or -CO-$NR_{20}$- group, wherein $R_{20}$

may be a hydrogen atom or an alkyl group and the alkylene group may additionally be substituted by a $C_{1-6}$-alkyl group, by an amino, aryl, alkylamino, dialkylamino, $-HNR_{21}$- or N-alkyl-$NR_{21}$- group, wherein the heteroatom of the additional substituent is separated from a heteroatom of the group W by at least two carbon atoms and $R_{21}$ is as hereinbefore defined, and

F denotes a carbonyl group subsituted by a hydroxy, alkoxy, arylalkoxy group or by an $R_{22}O$ group wherein $R_{22}$ denotes a $C_{5-7}$-cycloalkyl group or a cycloalkylalkyl group having 5 to 7 carbon atoms in the cycloalkyl moiety,

or a $R_{23}$CO-O-$CHR_{24}$-O-CO- group, phosphono or O-alkylphosphono group, wherein

$R_{23}$ denotes an alkyl, alkoxy, cycloalkyl or cycloalkyloxy group each having 5 to 7 carbon atoms in the cycloalkyl moiety, and

$R_{24}$ denotes a hydrogen atom or an alkyl group,

and the shortest distance between the group F and the nitrogen atom of group A-B which is furthest from the group F is at least 11 bonds;

a third of the groups $R_a$ to $R_d$ denotes a hydrogen atom, or an alkoxy group, wherein the alkoxy group may not be bound to a nitrogen atom, or it denotes an alkyl, trifluoromethyl or aryl group and,

the fourth of the groups $R_a$ to $R_d$ denotes a hydrogen atom or an alkyl group;

wherein unless otherwise specified

each of the aryl moieties mentioned in the definition of the above groups is a phenyl group which may be monosubstituted by $R_{25}$, mono, di or trisubstituted by $R_{26}$, or monosubstituted by $R_{25}$ and additionally mono or disubstituted by $R_{26}$, wherein the substituents may be identical or different and

$R_{25}$ denotes a cyano, aminocarbonyl, alkylaminocarbonyl, dialkylaminocarbonyl, alkylcarbonyl, alkylsulphenyl, alkylsulphinyl, alkylsulphonyl or trifluoromethyl group and

$R_{26}$ denotes an alkyl, hydroxy or alkoxy group or a fluorine, chlorine or bromine atom, and if two $R_{26}$ groups are bound to adjacent carbon atoms they may also represent a straight chain $C_{3-4}$-alkylene group, a 1,3-butadien-1,4-diylene group or a methylenedioxy group,

and each of the arylene moieties mentioned in the definition of the above-mentioned groups is a phenylene group which may be monosubstituted by $R_{25}$, mono or disubstituted by $R_{26}$, or monosubstituted by $R_{25}$ and additionally monosubstituted by $R_{26}$, wherein the substituents may be identical or different and are defined as hereinbefore,

and unless otherwise specified, the above-mentioned alkyl, alkylene and alkoxy moieties may each contain 1 to 4 carbon atoms, and each carbon atom in the above mentioned alkylene and cycloalkylene moieties is linked to one heteroatom at most

and the tautomers, stereoisomers and salts thereof.

**2.** Cyclic derivatives of general formula I according to claim 1, wherein:

X denotes a carbonyl or sulphonyl group;

Y represents a $-CH_2CH_2-$, $-CH_2CH_2CH_2-$, -CH=CH-, $CH_2CO$- or $-COCH_2-$ group and which may optionally be substituted by 1 or 2 methyl groups,

or a -CO-NH-, -CH=N or -N=CH- group optionally substituted by $R_c$;

one of the groups $R_a$ to $R_c$ denotes an A-B group wherein

A denotes a group of one of the formulae

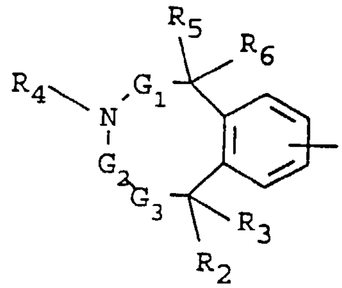

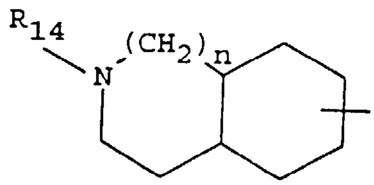

or

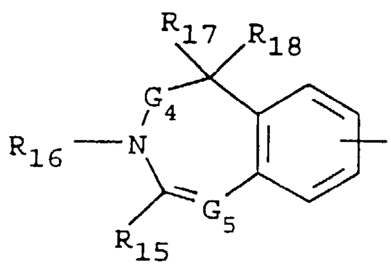

wherein in the benzo moiety of the above-mentioned groups, one to two methine groups may each be replaced by a nitrogen atom,

$G_1$ represents a bond or a methylene group

$G_2$ denotes a bond,

$G_3$ denotes a methylene group,

$G_4$ denotes a bond,

$G_5$ denotes a nitrogen atom or a methine group,

$R_2$ denotes a hydrogen atom,

$R_3$ denotes a hydrogen atom,

$R_4$ denotes a hydrogen atom, a cyclopropyl or cyclopropylmethyl group, a $C_{1-6}$-alkyl group, or an allyl, hydroxyalkyl, carboxyalkyl, alkoxycarbonylalkyl or benzyl group,

$R_5$ denotes a hydrogen atom,

$R_6$ denotes a hydrogen atom,

$R_{14}$ denotes a hydrogen atom or an alkyl group,

$R_{15}$ denotes a hydrogen atom or an alkyl group,

$R_{16}$ denotes a hydrogen atom or an alkyl group,

$R_{17}$ denotes a hydrogen atom or, $R_{16}$ together with $R_{17}$ denotes another bond,

$R_{18}$ denotes a hydrogen atom, or, if $R_{16}$ and $R_{17}$ together denote another bond, $R_{18}$ may also denote an amino group,

n represents the number 1 or 2, and

B denotes a bond or a phenylene group,

a second of the groups $R_a$ to $R_c$ denotes a group of formula

F - E - D-

wherein

D denotes an alkylene group,

or a phenylene group,

or a cyclohexylene group,

or a piperidinylene group, wherein the ring nitrogen atom is linked to the straight chain alkylene group of the group E, which may be substituted, or

a bond,

E denotes a straight chain alkylene group which may be substituted by an alkyl or phenyl group,

or a $C_{2-4}$-alkenylene group,

or a phenylene group,

or, if D does not denote a bond, E may denote a straight chain O-alkylene group linked to group D via the oxygen atom, and

F denotes a carbonyl group subsituted by a hydroxy or alkoxy group,

and the shortest distance between the group F and the nitrogen atom of group A-B which is furthest from the group F is at least 11 bonds; and

a third of the groups $R_a$ to $R_c$ denotes a hydrogen atom, an alkoxy group (wherein the alkoxy group cannot be bound to a nitrogen atom), or an alkyl or phenyl group,

and unless otherwise specified, the above-mentioned alkyl, alkylene or alkoxy moieties may each contain 1 to 4 carbon atoms, and each carbon atom in the above mentioned alkylene and cycloalkylene moieties is linked to one heteroatom at most;

and the tautomers, the stereoisomers and the salts thereof.

**3.** Cyclic derivatives of general formula I according to claim 1, wherein:

X denotes a carbonyl group,

Y denotes a $-CH_2CH_2-$, $-CH_2CH_2CH_2-$, -CH=CH-, $-CH_2CO-$ or $-COCH_2-$ group,

or a -N=CH- group optionally substituted by a methyl group,

the group $R_a$ denotes an A-B group wherein

A is a group of one of the formulae

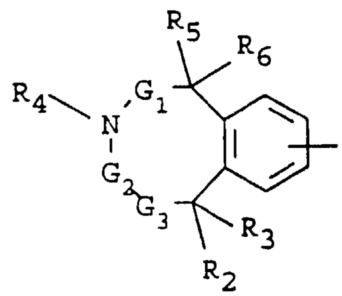

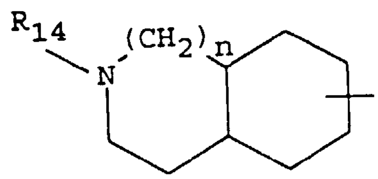

or

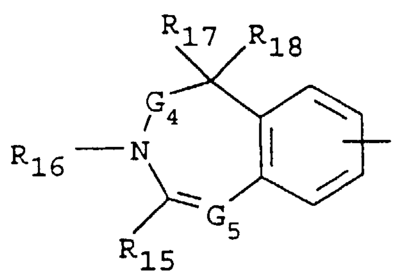

wherein in the benzo moiety of the above-mentioned groups 1 or 2 methine groups may each be replaced by a nitrogen atom,

$G_1$ denotes a bond or a methylene group,

$G_2$ denotes a bond,

$G_3$ denotes a methylene group,

$G_4$ denotes a bond,

$G_5$ denotes a methine group,

$R_2$ denotes a hydrogen atom,

$R_3$ denotes a hydrogen atom,

$R_4$ denotes a hydrogen atom, an alkyl group having 1 to 4 carbon atoms, or an allyl or benzyl group,

$R_5$ denotes a hydrogen atom,

$R_6$ denotes a hydrogen atom,

$R_{14}$ denotes a hydrogen atom or a methyl group,

$R_{15}$ denotes a hydrogen atom,

$R_{16}$ together with $R_{17}$ denotes a bond,

$R_{18}$ denotes a hydrogen atom or an amino group, and

n represents the number 1

B denotes a bond,

the group $R_b$ denotes a group of formula

F - E - D -

wherein

D denotes a $-CH_2CH_2-$ group,

or a 1,4-phenylene group,

or a 1,4-cyclohexylene group,

E denotes a $-CH_2CH_2-$ group, a -CH=CH-, 1,4-phenylene or $-O-CH_2-$ group substituted by a methyl group, wherein the oxygen atom of the $-O-CH_2-$ group is linked to the group D, and

F denotes a carbonyl group substituted by a hydroxy or alkoxy group having 1 to 4 carbon atoms,

and the shortest distance between the group F and the nitrogen atom of group A-B which is furthest from the group F is at least 11 bonds;

and the tautomers, stereoisomers and salts thereof.

**4.** The following compounds of general formula I according to claim 1:

(a) 1-[4-[2-(methoxycarbonyl)ethyl]phenyl]-3-(1,2,3,4-tetrahydroisoquinolin-6-yl)-imidazolidin-2-one,

(b) 1-[4-(2-carboxyethyl)phenyl]-3-(1,2,3,4-tetrahydroisoquinolin-6-yl)-imidazolidin-2-one,

(c) 1-[4-(2-carboxyethyl)phenyl]-3-(2-methyl-1,2,3,4-tetrahydroisoquinolin-6-yl)-imidazolidin-2-one,

(d) 1-[4-[2-(isobutyloxycarbonyl)ethyl]phenyl]-3-(1,2,3,4-tetrahydroisoquinolin-6-yl)-imidazolidin-2-one,

(e) 1-[4-(2-carboxyethyl)phenyl]-3-(2,3,4,5-tetrahydro-1H-3-benzazepin-7-yl)-imidazolidin-2-one,

(f) 1-[4-[2-(methoxycarbonyl)ethyl]phenyl]-3-(2,3,4,5-tetrahydro-1H-3-benzazepin-7-yl) -imidazolidin-2-one,

(g) 1-[4-[2-lisopropyloxycarbonyl)ethyllphenyl]-3-(2,3,4,5-tetrahydro-1H-3-benzazepin-7-yl)-imidazolidin-2-one,

(h) 1-[4-(2-carboxyethyl)phenyl]-3-(3-methyl-2,3,4,5-tetrahydro-1H-3-benzazepin-7-yl) -imidazolidin-2-one,

(i) 4-[4-[2-(carboxyethyl)phenyl]-5-methyl-2-(2,3,4,5-tetrahydro-1H-3-benzazepin-7-yl)-4H-1,2,4-triazol-3-one,

(j) 1-[trans-4-(2-carboxyethyl)cyclohexyl]-3-(2,3,4,5-tetrahydro-1H-3-benzazepin-7-yl)-imidazolidin-2-one,

(k) 1-[4-[2-(methoxycarbonyl)ethyl]phenyl]-3-(3-methyl-2,3,4,5-tetrahydro-1H-3-benzazepin-7-yl)-imidazolidin-2-one,

(l) 1-[4-12-(ethoxycarbonyl)ethyl]phenyl]-3-(3-methyl-2,3,4,5-tetrahydro-1H-3-benzazepin-7-yl)-imidazolidin-2-one,

(m) 1-[4-[2-(isopropyloxycarbonyl)ethyl]phenyl]-3-(3-methyl-2,3,4,5-tetrahydro-1H-3-benzazepin-7-yl)-imidazolidin-2-one,

(n) 1-[trans-4-(2-carboxyethyl)cyclohexyl]-3-(3-methyl-2,3,4,5-tetrahydro-1H-3-benzazepin-7-yl)-imidazolidin-2-one,

(o) 1-[trans-4-[2-(isopropyloxycarbonyl)ethyl]cyclo-hexyl]-3-(3-methyl-2,3,4,5-tetrahydro-1H-3-benzazepin-7-yl)-imidazolidin-2-one,

(p) 1-[trans-4-[2-(ethoxycarbonyl)ethyl]cyclohexyl]-3-(3-methyl-2,3,4,5-tetrahydro-1H-3-benzazepin-7-yl)-imidazolidin-2-one,

(q) 1-[trans-4-[2-(methoxycarbonyl)ethyl]cyclohexyl]-3-(3-methyl-2,3,4,5-tetrahydro-1H-3-benzazepin-7-yl)-imidazolidin-2-one,

(r) 1-[trans-4-[(carboxymethyl)oxy]cyclohexyl]-3-(3-methyl-2,3,4,5-tetrahydro-1H-3-benzazepin-7-yl)-imidazolidin-2-one,

(s) 3-[trans-4-(2-carboxyethyl)cyclohexyl]-1-(3-methyl-2,3,4,5-tetrahydro-1H-3-benzazepin-7-yl)--hydantoin and

(t) 1-[trans-4-(2-carboxyethyl)cyclohexyl]-3-(3-ethyl-2,3,4,5-tetrahydro-1H-3-benzazepin-7-yl)--imidazolidin-2-one,

and the tautomers and the salts thereof.

**5.** 1-[trans-4-(2-carboxyethyl)cyclohexyl]-3-(3-methyl-2,3,4,5-tetrahydro-1H-3-benzazepin-7-yl)-imidazolidin-2-one, the methyl, ethyl and isopropylesters as well as the salts thereof.

**6.** 1-[trans-4-(2-carboxyethyl)cyclohexyl]-3-(3-methyl-2,3,4,5-tetrahydro-1H-3-benzazepin-7-yl)-imidazolidin-2-one and the salts thereof.

**7.** Physiologically acceptable salts of the compounds according to at least one of claims 1 to 6 with inorganic or organic acids or bases.

**8.** Pharmaceutical compositions containing a compound according to at least one of claims 1 to 6 or a physiologically acceptable salt according to claim 7, optionally together with one or more inert carriers and/or diluents.

**9.** Use of a compound according to at least one of claims 1 to 7 for preparing a pharmaceutical composition which is suitable for combatting or preventing diseases in which smaller or larger cell-aggregates occur or cell-matrix

interactions are involved.

**10.** Process for preparing a pharmaceutical composition according to claim 8, characterised in that a compound according to at least one of claims 1 to 7 is incorporated in one or more inert carriers and/or diluents by a non-chemical method.

**11.** Process for preparing the cyclic derivatives according to claims 1 to 7, characterised in that

a) in order to prepare compounds of general formula I wherein F denotes a carboxyl group: a compound of general formula

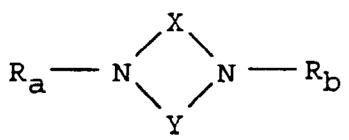

, (I)

wherein

$R_a$, $R_b$, X and Y are as defined in claims 1 to 6, with the proviso that one of the groups $R_a$ to $R_d$ denotes an F'-E-D group, wherein

E and D are as defined in claims 1 to 6 and F' represents a group which can be converted into a carboxyl group by hydrolysis, treatment with acids, thermolysis or hydrogenolysis

is converted into a compound of general formula I wherein F denotes a carboxyl group by hydrolysis treatment with acids, thermolysis or hydrogenolysis; or

b) in order to prepare compounds of general formula I wherein $R_{16}$ and $R_{17}$ together denote another bond, $G_4$ denotes a bond and $R_{18}$ denotes an amino group, a compound of general formula

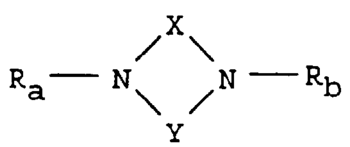

, (III)

wherein

$R_a$, $R_b$, X and Y are as defined in claims 1 to 6, with the proviso that one of the groups $R_a$ to $R_d$ denotes an A-B group wherein B is defined as in claims 1 to 6 and A denotes a group of the formula

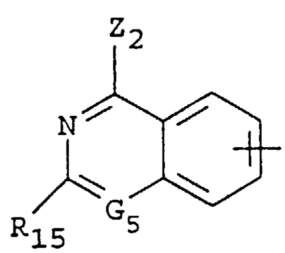

wherein the benzo moiety, $R_{15}$ and $G_5$ are defined as in claims 1 to 6, and

$Z_2$ denotes a nucleophilic leaving group,

is reacted with a compound of general formula

$$H - R_{27} \quad (IV)$$

wherein $R_{27}$ denotes an amino group, or

c) in order to prepare compounds of general formula I wherein $R_{16}$ and $R_{17}$ together denote another bond, $G_4$ denotes a bond and $R_{18}$ denotes a chlorine atom, a compound of general formula

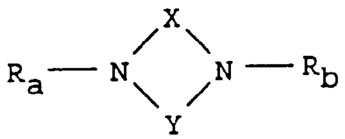

, (V)

wherein

$R_a$, $R_b$, X and Y are as defined in claims 1 to 6, with the proviso that one of the groups $R_a$ to $R_d$ represents an A-B group wherein B is defined as specified in claims 1 to 6 and A denotes a group of formula

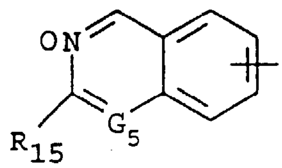

wherein the benzo moiety, $R_{15}$ and $G_5$ are defined as in claims 1 to 6, is reacted in the presence of an acid halide, or

d) in order to prepare compounds of general formula I wherein X denotes a cyano-substituted carbimino group or a carbonyl or sulphonyl group and Y denotes a straight chain $C_{2-3}$-alkylene group optionally substituted by $R_c$ or $R_d$ or by $R_c$ and $R_d$: cyclising a compound of general formula

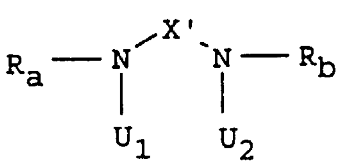

, (VI)

wherein

$R_a$ and $R_b$ are defined as in claims 1 to 6,
X' denotes a cyano-substituted carbimino group or a carbonyl or sulphonyl group,
one of the groups $U_1$ or $U_2$ denotes a hydrogen atom and the other group $U_1$ or $U_2$ denotes a straight chain $C_{2-3}$-alkylene group optionally substituted by $R_c$ or $R_d$ or by $R_c$ and $R_d$, to which additionally is terminally bound a nucleophilic leaving group; or

e) in order to prepare compounds of general formula I wherein X denotes a carbonyl group and Y denotes a straight chain $C_{2-3}$- alkylene group mentioned in claims 1 to 6, a compound of general formula

$$R_{28} - NH - T \qquad (VII)$$

is reacted with an isocyanate of general formula

$$O = C = N - R_{29} \qquad (VIII)$$

wherein one of the groups $R_{28}$ or $R_{29}$ has the meanings given for $R_a$ in claims 1 to 6 and the other group $R_{28}$ or $R_{29}$ has the meanings given for $R_b$ in claims 1 to 6 and T denotes a group of the formula

$$-(CH_2)_m - HC(OR_{30})_2$$

optionally substituted in the alkylidene moiety by $R_c$ or $R_d$ or by $R_c$ and $R_d$, wherein

m denotes the number 1 or 2 and

$R_{30}$ denotes a $C_{1-4}$-alkyl group,

and optionally a compound thus obtained is subsequently hydrogenated, or

f) in order to prepare compounds of general formula I wherein $G_1$ and G2 each represent a bond, $G_3$ represents a methylene group optionally substituted by a $C_{1-4}$-alkyl group, $R_2$, $R_4$ and $R_5$ each represent a hydrogen atom, and $R_3$ and $R_6$ which may be identical or different each represent a hydrogen atom or a $C_{1-4}$-alkyl group: hydrogenation of a compound of general formula

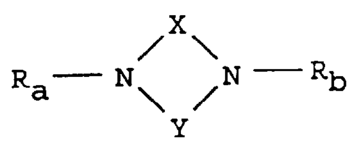

, (IX)

wherein

$R_a$, $R_b$, X and Y are defined as in claims 1 to 6, with the proviso that one of the groups $R_a$ to $R_d$ denotes an A-B group, wherein B is defined as in claims 1 to 6 and A denotes a group of the formula

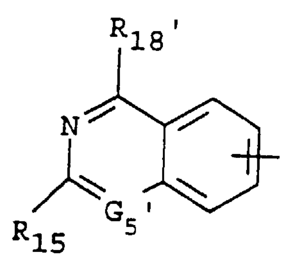

wherein

the benzo moiety and $R_{15}$ are defined as in claims 1 to 6,

$G_5$' denotes a methine group optionally substituted by a $C_{1-4}$-alkyl group, and

$R_{18}$' denotes a hydrogen atom or a $C_{1-4}$ alkyl group; or

g) in order to prepare compounds of general formula I wherein F denotes a carbonyl group substituted by a $C_{1-4}$-alkoxy group, by an arylalkoxy group (having 1 to 4 carbon atoms in the alkoxy moiety and wherein the aryl moiety is defined as in claims 1 to 6), or by an $R_{22}O$ group, a compound of general formula

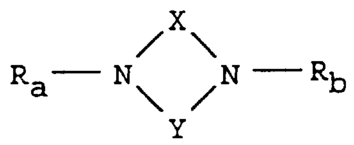

, (X)

wherein

$R_a$ $R_b$, X and Y are defined as in claims 1 to 6, with the proviso that one of the groups $R_a$ to $R_d$ denotes an F"-E-D group, wherein

E and D are defined as in claims 1 to 6 and

F" denotes a carboxy or alkoxycarbonyl group, is reacted with an alcohol of general formula

$$HO - R_{31} \quad (XI)$$

wherein

$R_{31}$ has the meanings given for $R_{22}$ in claims 1 to 6 or denotes a $C_{1-4}$-alkyl group or an arylalkyl group in which the aryl moiety is defined as in claims 1 to 6 and the alkyl moiety may contain 1 to 4 carbon atoms, or

h) in order to prepare compounds of general formula I wherein $R_4$ represents one of the optionally substituted alkyl, alkenyl, cycloalkyl, cycloalkylalkyl or aralkyl groups mentioned in claims 1 to 6 in the definition of groups $R_4$, a compound of general formula

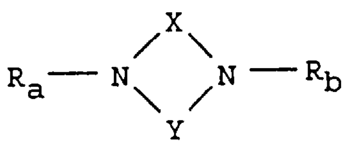

, (XI)

wherein

$R_a$, $R_b$, X and Y are defined as in claims 1 to 6, with the proviso that one of the groups $R_a$ to $R_d$ denotes an A-B group, wherein

A and B are defined as in claims 1 to 6, with the proviso that $R_4$ represents a hydrogen atom

is reacted with a compound of general formula

$$Z_3 - R_{32} \quad (XIII)$$

wherein

$R_{32}$ denotes a $C_{1-6}$-alkyl group, a cycloalkyl or cycloalkylalkyl group in which the cycloalkyl moiety

may contain 3 to 7 carbon atoms and the alkyl moiety may contain 1 to 4 carbon atoms, a $C_{3-6}$-alkenyl group, or an arylalkyl, hydroxyalkyl, alkoxyalkyl, carboxyalkyl, alkoxycarbonylalkyl, aminocarbonylalkyl, N-alkylaminocarbonylalkyl or N,N-dialkylaminocarbonyl group, wherein the aryl moiety and the alkyl moieties are defined as in claims 1 to 6, and

$Z_3$ denotes a nucleophilic leaving group, or

$Z_3$ together with an adjacent hydrogen atom of the group $R_{32}$ denotes an oxygen atom, optionally in the presence of a reducing agent; or

i) in order to prepare compounds of general formula I wherein X represents a carbimino group which is substituted by a cyano group at the nitrogen atom, or X denotes a carbonyl or sulphonyl group, a compound of general formula

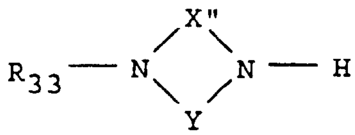

, (XIV)

is reacted with a compound of general formula

$$Z_4 - R_{34} \quad (XV)$$

wherein

Y is defined as in claims 1 to 6,

X" denotes a carbimino group substituted by a cyano group at the nitrogen atom, or X" denotes a carbonyl or sulphonyl group, one of the groups $R_{33}$ or $R_{34}$ has the meanings given for $R_a$ in claims 1 to 6 and the other group $R_{33}$ or $R_{34}$ has the meanings given for $R_b$ in claims 1 to 6 and $Z_4$ denotes a nucleophilic leaving group; or

j) in order to prepare compounds of general formula I wherein $R_4$ denotes an alkoxycarbonyl, arylmethyloxycarbonyl, formyl, acetyl, trifluoroacetyl, or $R_{11}CO-O-(R_{12}CR_{13})-O-CO-$ group, wherein $R_{11}$ to $R_{13}$ and the aryl moiety are defined as in claims 1 to 6 and the alkoxy moiety may contain 1 to 4 carbon atoms, a compound of general formula

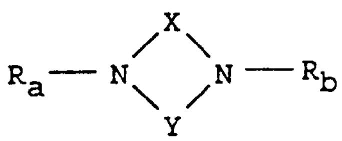

, (XVI)

wherein

$R_a$, $R_b$, X and Y are defined as in claims 1 to 6, with the proviso that $R_4$ denotes a hydrogen atom is reacted with a compound of general formula

$$Z_5 - R_{35} \quad (XVII)$$

wherein

$R_{35}$ represents an alkoxycarbonyl group having a total of 2 to 5 carbon atoms, an arylmethyloxycarbonyl group in which the aryl moiety is defined as in claims 1 to 6, or a $R_{11}CO-O-(R_{12}CR_{13})$ -O-CO, formyl, acetyl, or trifluoroacetyl group, wherein $R_{11}$ to $R_{13}$ are defined as in claims 1 to 6, and

$Z_5$ denotes a nucleophilic leaving group; or

k) in order to prepare compounds of general formula I wherein F denotes a carbonyl group which is substituted by a $C_{1-4}$-alkoxy group, by an arylalkoxy group (in which the aryl moiety is as defined in claims 1 to 6 and the alkoxy moiety may contain 1 to 4 carbon atoms), or by an $R_{22}O$- or $R_{23}CO\text{-}O\text{-}CHR_{24}O$- group, wherein $R_{22}$ to $R_{24}$ are defined as in claims 1 to 6: reacting a compound of general formula

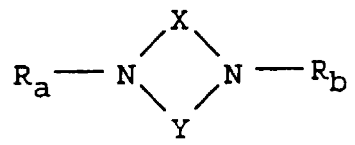

, (XVIII)

wherein

$R_a$, $R_b$, X and Y are defined as in claims 1 to 6, with the proviso that one of the groups $R_a$ to $R_d$ denotes an F'''-E-D- group wherein

E and D are defined as in claims 1 to 6 and

F''' denotes a carboxyl group

with a compound of general formula

$$Z_6 - R_{36} \quad \text{(XIX)}$$

wherein

$R_{36}$ represents a $C_{1-4}$-alkyl group, an arylalkyl group (in which the aryl moiety is defined as in claims 1 to 6 and the alkyl moiety may contain 1 to 4 carbon atoms), or a $R_{22}$- or $R_{23}CO\text{-}O\text{-}CHR_{24}$ group (wherein $R_{22}$ to $R_{24}$ are defined as in claims 1 to 6), and

$Z_6$ denotes a nucleophilic leaving group; or

l) in order to prepare compounds of general formula I wherein X denotes a carbonyl group and Y denotes an ethylene group which is optionally substituted by $R_c$ or $R_d$ or by $R_c$ and $R_d$, and in which a methylene group is replaced by a carbonyl group: cyclising a compound of general formula

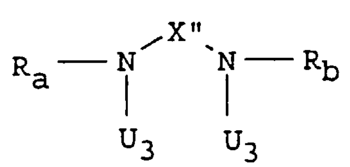

, (XX)

wherein

$R_a$ and $R_b$ are defined as in claims 1 to 6,
X" denotes a carbonyl group,
one of the groups U3 or $U_4$ denotes a hydrogen atom and the other group $U_3$ or $U_4$ denotes an ethylene group which is optionally substituted by $R_c$ or $R_d$ or by $R_c$ and $R_d$, and in which a methylene group is replaced by a $Z_7$-CO group, wherein $Z_7$ denotes a nucleophilic leaving group, optionally formed in the reaction mixture,

and subsequently, if desired, a compound of general formula I thus obtained which contains an unsaturated carbon-carbon bond, is converted by catalytic hydrogenation into a corresponding saturated compound of general formula

I, and/or
if necessary, a protecting group used during the reactions to protect any reactive groups is cleaved and/ or
a compound of general formula I thus obtained is resolved into the stereoisomers thereof and/or
a compound of general formula I thus obtained is converted into the salts thereof, more particularly for pharmaceutical use into the physiologically acceptable salts thereof with an inorganic or organic acid or base.

## Revendications

**1.** Dérivés cycliques de formule générale

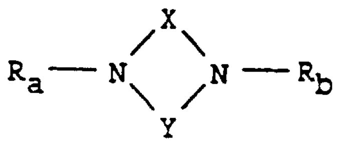

(I)

où

X représente un groupe carbimino substitué sur l'atome d'azote par un groupe cyano, un groupe carbonyle ou sulfonyle,
Y représente un groupe $-CH_2CH_2-$, $-CH_2CH_2CH_2-$, $-CH=CH-$, $-CH_2CO-$ ou $-COCH_2-$ éventuellement substitué par $R_c$ ou $R_c$ et $R_d$,
un groupe -CO-NH-, -NH-CO-, -CH=N- ou -N=CH- éventuellement substitué par $R_c$ ou $R_d$,
l'un des restes $R_a$ à $R_d$ représente un groupe A-B- dans lequel
A représente un groupe de formule

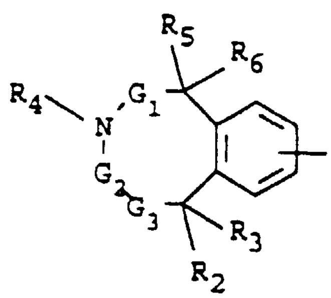

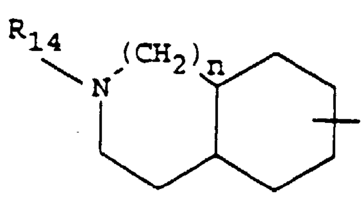

ou

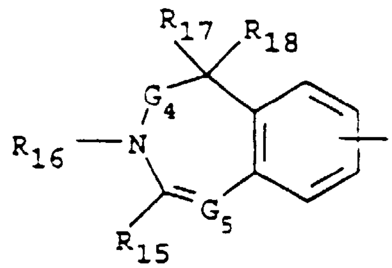

où dans chaque cas la partie benzo des groupes indiqués précédemment peut être substituée par un atome de fluor, de chlore ou de brome, par un groupe alkyle, cyano, trifluorométhyle, hydroxyle ou alcoxy, ou un à trois groupes méthyne peuvent être remplacés chacun par un atome d'azote,

$G_1$ représente une liaison ou un groupe méthylène qui peut être mono- ou disubstitué par un groupe alkyle, les substituants pouvant être identiques ou différents,
$G_2$ représente une liaison ou un groupe méthylène substitué par $R_7$ et $R_8$,
$G_3$ représente un groupe méthylène substitué par $R_9$ et $R_{10}$,
$G_4$ représente une liaison,
$G_5$ représente un atome d'zote ou un groupe méthyne éventuellement substitué par un groupe alkyle,
$R_2$ représente un atome d'hydrogène ou un groupe alkyle,
$R_3$ représente un atome d'hydrogène ou un groupe alkyle,
$R_4$ représente un atome d'hydrogène, un groupe cycloalkyle ou cycloalkylalkyle ayant dans chaque cas 3 à 7 atomes de carbone dans la partie cycloalkyle, un groupe alkyle ayant 1 à 6 atomes de carbone, un groupe alcényle ayant 3 à 6 atomes de carbone, le groupe alcényle ne pouvant pas être lié à l'atome d'azote par la partie vinyle, un groupe hydroxyalkyle, alcoxyalkyle, carboxyalkyle, alcoxycarbonylalkyle, aminocarbonylalkyle, N-alkylaminocarbonylalkyle, N,N-dialkylaminocarbonylalkyle, arylalkyle, alcoxycarbonyle, arylméthyloxycarbonyle, formyle, acétyle, trifluoroacétyle ou $R_{11}CO-O-(R_{12}CR_{13})-O-CO-$ où
$R_{11}$ représente un groupe alkyle,
$R_{12}$ représente un atome d'hydrogène ou un groupe alkyle et
$R_{13}$ représente un atome d'hydrogène,
ou $R_4$ représente avec $R_3$ un groupe alkylène linéaire de 2 ou 3 atomes de carbone,
$R_5$ représente un atome d'hydrogène ou un groupe alkyle ou encore, si $G_5$ représente une liaison, $R_4$ représente avec $R_5$ une liaison supplémentaire,
$R_6$ représente un atome d'hydrogène ou un groupe alkyle ou, si $G_5$ représente une liaison et $R_4$ représente avec $R_5$ une liaison supplémentaire, un groupe amino,
$R_7$ représente un atome d'hydrogène ou un groupe alkyle,
$R_8$ représente un atome d'hydrogène ou un groupe alkyle ou
$R_8$ représente avec $R_4$ un groupe alkylène linéaire de 3 ou 4 atomes de carbone,
$R_9$ représente un atome d'hydrogène ou un groupe alkyle,
$R_{10}$ représente un atome d'hydrogène ou un groupe alkyle ou
$R_{10}$ représente avec $R_4$ un groupe alkyléne linéaire de 3 ou 4 atomes de carbone,
$R_{14}$ représente un atome d'hydrogène ou un groupa alkyle,
$R_{15}$ représente un atome d'hydrogène ou un groupe alkyle,
$R_{16}$ représente un atome d'hydrogène ou un groupe alkyle,
$R_{17}$ représente un atome d'hydrogène ou un groupe alkyle ou
$R_{16}$ représente avec $R_{17}$ une liaison supplémentaire,
$R_{18}$ représente un atome d'hydrogène ou un groupe alkyle ou encore, lorsque $R_{16}$ et $R_{17}$ représentent ensemble une liaison supplémentaire, un atome de chlore ou un groupe amino,
n représente le nombre 1 ou 2, et
B représente une liaison,
un groupe alkylène,
un groupe arylène,
un groupe pyridinyléne, pyrimidinylène, pyrazinylène ou pyridazinylène éventuellement substitué par un ou deux groupes alkyle,
un groupe cyclohexylène éventuellement substitué par un ou deux groupes alkyle,

un groupe pipéridinylène éventuellement substitué par un ou deux groupes alkyle, où un groupe méthylène voisin d'un atome d'azote peut être remplacé par un groupe carbonyle, le second des restes $R_a$ à $R_d$ représente un groupe de formule

F - E - D -

où
D représente un groupe alkylène,
un groupe arylène,
un groupe pyridinylène, pyrimidinyléne, pyrazinylène ou pyridazinylène éventuellement substitué par un ou deux groupes alkyle,
un groupe indanylène, naphtylène, 1,2,3,4-tétrahydronaphtylène ou benzosubéranylène, où dans chaque cas l'un des cycles est lié au reste E et l'autre des cycles est lié au reste cyclique de la formule générale I, un groupe cycloalkylène de 4 à 7 atomes de carbone éventuellement substitué par un ou deux groupes alkyle,
un groupe cycloalkylène de 5 à 7 atomes de carbone éventuellement substitué par un ou deux groupes alkyle, où une unité >CH est remplacée par un atome d'azote, où en outre dans les cycles à 5 à 7 chaînons indiqués précédemment dans chaque cas un groupe méthylène voisin d'un atome d'azote peut être remplacé par un groupe carbonyle,
ou aussi, lorsque E représente un groupe imino cyclique, un groupe alkylènecarbonyle où le groupe carbonyle est lié dans chaque cas à l'atome d'azote du groupe imino cyclique du groupe E,
ou encore, si E ne représente pas une liaison, une liaison, E représente une liaison,
un groupe alkylène qui peut être substitué par un groupe alkyle de 1 à 6 atomes de carbone, par un groupe amino, aryle, alkylamino, dialkylamino, $HNR_{21}$- ou N-alkyl-$NR_{21}$-, où $R_{21}$ représente un groupe aikylcarbonyle ou alkylsulfonyle ayant dans chaque cas 1 à 6 atomes de carbone dans la partie alkyle, un groupe alkyloxycarbonyle ayant au total 2 à 5 atomes de carbone, un groupe cycloalkylcarbonyle ou cycloalkylsulfonyle ayant dans chaque cas 5 à 7 atomes de carbone tans la partie cycloalkyle, un groupe arylalkylcarbonyle, arylalkylsulfonyle, arylalcoxycarbonyle, arylcarbonyle ou arylsulfonyle,
un groupe alcénylène de 2 à 4 atomes de carbone,
un groupe arylène,
un groupe pyridinylène, pyrimidinylène, pyrazinylène ou pyridazinylène éventuellement substitué par un ou deux groupes alkyle,
un groupe cycloalkylène de 5 à 7 atomes de carbone éventuellement substitué par un ou deux groupes alkyle, où une unité >CH peut être remplacée par un atome d'azote qui est lié à un atome de carbone du reste D,
un groupe cycloalkylène de 4 à 7 atomes de carbone dans la partie cycloalkylène éventuellement substitué par un ou deux groupes alkyle,
ou encore, si D ne représente pas une liaison, un groupe alkylène lié au reste D par le reste W, où W représente un atome d'oxygène ou de soufre, un groupe sulfinyle, sulfonyle, -$NR_{20}$-CO- ou -CO-$NR_{20}$- où $R_{20}$ représente un atome d'hydrogène ou un groupe alkyle et le groupe alkylène peut être substitué en outre par un groupe alkyle de 1 à 6 atomes de carbone, par un groupe amino, aryle, alkylamino, dialkylamino, $HNR_{21}$- ou >N-alkyl-$NR_{21}$-, où l'hétéroatome du substituant supplémentaire est séparé d'un hétéroatome du reste W par au moins 2 atomes de carbone et $R_{21}$ est défini comme indiqué précédemment, et
F représente un groupe carbonyle qui est substitué par un groupe hydroxyle, alcoxy, arylalcoxy ou $R_{22}$O-, où
$R_{22}$ représente un groupe cycloalkyle de 5 à 7 atomes de carbone ou un groupe cycloalkylalkyle de 5 à 7 atomes de carbone dans la partie cycloalkyle,
un groupe $R_{23}$CO-O-$CHR_{24}$-O-CO-, phosphono ou O-alkyl-phosphono, où
$R_{23}$ représente un groupe alkyle, alcoxy, cycloalkyle ou cycloalcoxy de 5 à 7 atomes de carbone dans la partie cycloalkyle dans chaque cas, et
$R_{24}$ représente un atome d'hydrogène ou un groupe alkyle, et la plus courte distance entre le reste F et l'atome d'azote du groupe A-B- qui est le plus éloigné du reste F est d'au moins 11 liaisons,
Le troisième des restes $R_a$ à $R_d$ représente un atome d'hydrogène, un groupe alcoxy, le groupe alcoxy ne pouvant pas être lié à un atome d'azote, un groupe alkyle, trifluorométhyle ou aryle et
le quatrième des restes $R_a$ à $R_d$ représente un atome d'hydrogène ou un groupe alkyle,
où, sauf indication contraire,

concernant les parties aryle indiquées lors de la définition des restes précédents on doit entendre un groupe phényle, qui peut être dans chaque cas monosubstitué par $R_{25}$, mono-, di- ou trisubstitué par $R_{26}$ ou monosubstitué par $R_{25}$ et en outre mono- ou disubstitué par $R_{26}$, les substituants pouvant être identiques ou différents, et

$R_{25}$ représente un groupe cyano, aminocarbonyle, alkylaminocarbonyle, dialkylaminocarbonyle, alkylcarbonyle, alkylsulfényle, alkylsulfinyle, alkylsulfonyle ou trifluorométhyle et

$R_{26}$ représente un groupe alkyle, hydroxyle ou alccxy, un atome de fluor, de chlore ou de brome, deux restes $R_{26}$, dans la mesure où ceux-ci sont liés à des atomes de carbone voisins, pouvant aussi représenter un groupe alkylène linéaire de 3 ou 4 atomes de carbone, un groupe 1,3-butadiène-1,4-diylène ou un groupe méthylènedioxy,

concernant les parties aryléne indiquées lors de la définition des restes précédents on doit entendre un groupe phénylène qui peut dans chaque cas être monosubstitué par $R_{25}$, mono- ou disubstitué par $R_{26}$ ou monosubstitué par $R_{25}$ et en outre monosubstitué par $R_{26}$, les substituants pouvant être identiques ou différents et sont définis comme indiqué précédemment,

et, sauf indication contraire, les parties alkyle, alkylène ou alcoxy indiquées précédemment peuvent contenir dans chaque cas 1 à 4 atomes de carbone, et chaque atome de carbone est lié au plus à un hétéroatome dans les parties alkylène et cycloalkylène indiquées précédemment,

leurs tautomères, leurs stéréoisomères et leurs sels.

**2.** Dérivés cycliques de formule générale I selon la revendication 1, où

X représente un groupe carbonyle ou sulfonyle,

Y représente un groupe $-CH_2CH_2-$, $-CH_2CH_2CH_2-$, -CH=CH-, $-CH_2CO-$ ou $-COCH_2-$ éventuellement substitué par un ou deux groupes méthyle,

un groupe -CO-NH-, -CH=N- ou -N=CH- éventuellement substitué par $R_c$,

l'un des restes $R_a$ à $R_c$ représente un groupe A-B- dans lequel

A représente un groupe de formule

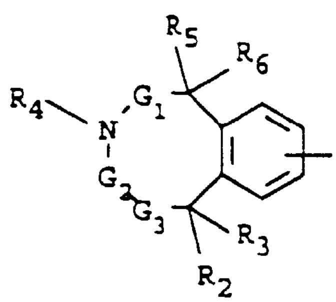

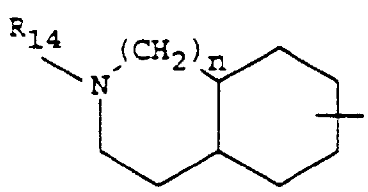

ou

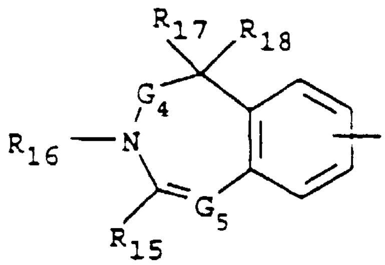

où dans chaque cas dans la partie benzo des groupes indiqués précédemment un ou deux groupes méthyne peuvent être remplacés chacun par un atome d'azote,

$G_1$ représente une liaison ou un groupe méthylène,
$G_2$ représente une liaison,
$G_3$ représente un groupe méthylène,
$G_4$ représente une liaison,
$G_5$ représente un atome d'azote ou un groupe méthyne,
$R_2$ représente un atome d'hydrogène,
$R_3$ représente un atome d'hydrogène,
$R_4$ représente un atome d'hydrogène, un groupe cyclopropyle ou cyclopropylméthyle, un groupe alkyle de 1 à 6 atomes de carbone, un groupe allyle, hydroxyalkyle, carboxyalkyle, alcoxycarbonylalkyle ou benzyle,
$R_5$ représente un atome d'hydrogène,
$R_6$ représente un atome d'hydrogène,
$R_{14}$ représente un atome d'hydrogène ou un groupe alkyle,
$R_{15}$ représente un atome d'hydrogène ou un groupe alkyle,
$R_{16}$ représente un atome d'hydrogène ou un groupe alkyle,
$R_{17}$ représente un atome d'hydrogène ou $R_{16}$ représente avec $R_{17}$ une liaison supplémentaire,
$R_{18}$ représente un atome d'hydrogène ou encore, lorsque $R_{16}$ et $R_{17}$ représentent ensemble une liaison supplémentaire, un groupe amino,
n représente le nombre 1 ou 2, et
B représente une liaison ou un groupe phénylène,
le second des restes $R_a$ à $R_c$ représente un groupe de formule

F - E - D -

où
D représente un groupe alkylène,
un groupe phénylène,
un groupe cyclohexylène ou
un groupe pipéridinylène, l'atome d'azote cyclique étant lié au groupe alkylène linéaire éventuellement substitué du reste E, ou
une liaison,
E représente un groupe alkyléne linéaire qui peut être substitué par un groupe alkyle ou phényle,
un groupe alcénylène de 2 à 4 atomes de carbone,
un groupe phénylène,
ou encore, si D ne représente pas une liaison, un groupe O-alkylène linéaire lié au reste D par l'intermédiaire de l'atome d'oxygène et
F représente un groupe carbonyle qui est substitué par un groupe hydroxyle ou alcoxy
et la plus courte distance entre le reste F et l'atome d'azote du groupe A-B- qui est le plus éloigné du reste F est d'au moins 11 liaisons, et
le troisième des restes $R_a$ à $R_d$ représente un atome d'hydrogène, un groupe alcoxy, le groupe alcoxy ne pouvant pas être lié à un atome d'azote, un groupe alkyle ou phényle,
où, sauf indication contraire, les parties alkyle, alkylène ou alcoxy indiquées précédemment peuvent contenir dans chaque cas 1 à 4 atomes de carbone, et chaque atome de carbone est lié au plus à un hétéroatome dans les parties alkylène et cycloalkylène indiquées précédemment, leurs tautomères, leurs stéréoisomères et leurs sels.

**3.** Dérivés cycliques de formule générale I selon la revendication 1, où

X représente un groupe carbonyle,
Y représente un groupe $-CH_2CH_2-$, $-CH_2CH_2CH_2-$, -CH=CH-, $-CH_2CO-$ ou $-COCH_2-$ ou un groupe -N=CH- éventuellement substitué par un groupe méthyle,
le reste $R_a$ représente un groupe A-B- dans lequel
A représente un groupe de formule

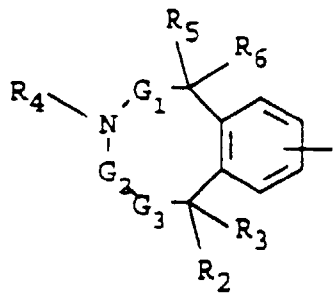

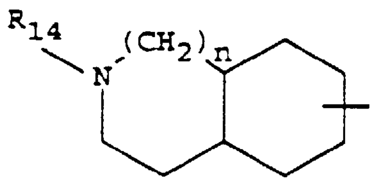

ou

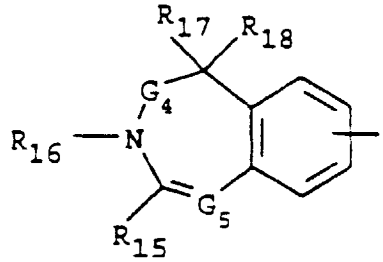

où dans chaque cas dans la partie benzo des groupes indiqués précédemment un ou deux groupes méthyne peuvent être remplacés chacun par un atome d'azote,

$G_1$ représente une liaison ou un groupe méthylène,
$G_2$ représente une liaison,
$G_3$ représente un groupe méthylène,
$G_4$ représente une liaison,
$G_5$ représente un groupe méthyne,
$R_2$ représente un atome d'hydrogène,
$R_3$ représente un atome d'hydrogène,
$R_4$ représente un atome d'hydrogène, un groupe alkyle de 1 à 4 atomes de carbone, un groupe allyle ou benzyle,
$R_5$ représente un atome d'hydrogène,

$R_6$ représente un atome d'hydrogène,
$R_{14}$ représente un atome d'hydrogène ou un groupe méthyle,
$R_{15}$ représente un atome d'hydrogène,
$R_{16}$ représente avec $R_{17}$ une liaison,
$R_{18}$ représente un atome d'hydrogène ou un groupe amino et
n représente le nombre 1,
B représente une liaison,
le reste $R_b$ représente un groupe de formule

F - E - D -

où
D représente un groupe $-CH_2-CH_2-$,
un groupe 1,4-phénylène ou
un groupe 1,4-cyclohexylène,
E représente un groupe $-CH_2-CH_2-$ éventuellement substitué par un groupe méthyle, un groupe -CH=CH-, 1,4-phénylène ou $-O-CH_2-$, l'atome d'oxygène du groupe $-O-CH_2-$ étant lié au reste D, et
F représente un groupe carbonyle qui est substitué par un groupe hydroxyle ou alcoxy de 1 à 4 atomes de carbone, et la plus courte distance entre le reste F et l'atome d'azote du groupe A-B- qui est le plus éloigné du reste F est d'au moins 11 liaisons,
leurs tautomères, leurs stéréoisomères et leurs sels.

**4.** Composés suivants de formule générale I selon la revendication 1 :

(a) 1-[4-[2-(méthoxycarbonyl)éthyl]phényl]-3-(1,2,3,4-tétrahydroisoquinolin-6-yl)-imidazolidin-2-one,

(b) 1-[4-(2-carboxyéthyl)phényl]-3-(1,2,3,4-tétrahydroisoquinolin-6-yl)-imidazolidin-2-one,

(c) 1-[4-(2-carboxyéthyl)phényl]-3-(2-méthyl-1,2,3,4-tétrahydroisoquinolin-6-yl)-imidazolidin-2-one,

(d) 1-[4-[2-(isobutyloxycarbonyl)éthyl]phényl]-3-(1,2,3,4-tétrahydroisoquinolin-6-yl)-imidazolidin-2-one,

(e) 1-[4-(2-carboxyéthyl)phényl]-3-(2,3,4,5-tétrahydro-1H-3-benzazépin-7-yl)-imidazolidin-2-one,

(f) 1-[4-[2-(méthoxycarbonyl)éthyl]phényl]-3-(2,3,4,5-tétrahydro-1H-3-benzazépin-7-yl)-imidazolidin-2-one,

(g) 1-[4-[2-(isopropyloxycarbonyl)éthyl]phényl]-3-(2,3,4,5-tétrahydro-1H-3-benzazépin-7-yl)-imidazolidin-2-one,

(h) 1-[4-(2-carboxyéthyl)phényl]-3-(3-méthyl-2,3,4,5-tétrahydro-1H-3-benzazépin-7-yl)-imidazolidin-2-one,

(i) 4-[4-[2-(carboxyéthyl)phényl]-5-méthyl-2-(2,3,4,5-tétrahydro-1H-3-benzazépin-7-yl)-4H-1,2,4-triazol-3- one,

(j) 1-[trans-4-(2-carboxyéthyl)cyclohexyl]-3-(2,3,4,5-tétrahydro-1H-3-benzazépin-7-yl)-imidazolidin-2-one,

(k) 1-[4-[2-(méthoxycarbonyl)éthyl]phényl]-3-(3-méthyl-2,3,4,5-tétrahydro-1H-3-benzazépin-7-yl)-imidazolidin-2-one,

(l) 1-[4-[2-(éthoxycarbonyl)éthyl]phényl]-3-(3-méthyl-2,3,4, 5-tétrahydro-1H-3-benzazépin-7-yl)-imidazolidin-2-one,

(m) 1-[4-[2-(isopropyloxycarbonyl)éthyl]phényl]-3-(3-méthyl-2,3,4,5-tétrahydro-1H-3-benzazépin-7-yl)-imidazolidin-2-one,

(n) 1-[trans-4-(2-carboxyéthyl)cyclohexyl]-3-(3-méthyl-2,3,4, 5-tétrahydro-1H-3-benzazépin-7-yl)-imidazolidin-2-one,

(o) 1-[trans-4-[2-(isopropyloxycarbonyl) éthyl] cyclohexyl]-3-(3-méthyl-2,3,4,5-tétrahydro-1H-3-benzazépin-7-yi)-imidazolidin-2-one,

(p) 1-[trans-4-[2-(éthoxycarbonyl)éthyl]cyclohexyl]-3-(3-méthyl-2,3,4,5-tétrahydro-1H-3-benzazépin-7-yl)-imidazolidin-2-one,

(q) 1-[trans-4-[2-(méthoxycarbonyl)éthyl]cyclohexyl]-3-(3-méthyl-2,3,4,5-tétrahydro-1H-3-benzazépin-7-yl)-imidazolidin-2-one,

(r) 1-[trans-4-[(carboxyméthyl)oxy]cyclohexyl]-3-(3-méthyl-2,3,4, 5-tétrahydro-1H-3-benzazépin-7-yl)-imidazolidin-2-one,

(s) 3-[trans-4-(2-carboxyéthyl)cyclohexyl]-1-[3-méthyl-2,3,4,5-tétrabydro-1H-3-benzazépin-7-yl)-hydantoïne et

(t) 1-[trans-4-(2-carboxyéthyl)cyclohexyl]-3-(3-éthyl-2,3,4, 5-tétrahydro-1H-3-benzazépin-7-yl)-imidazolidin-2-one,

leurs tautomères et leurs sels.

**5.** 1-[trans-4-(2-carboxyéthyl)cyclohexyl]-3-(3-méthyl-2,3,4,5-tétrahydro-1H-3-benzazépin-7-yl)-imidazolidin-2-one, ses méthyl-, éthyl- et isopropylesters et ses sels.

**6.** 1-[trans-4-(2-carboxyéthyl)cyclohexyl]-3-(3-méthyl-2,3,4,5-tétrahydro-1H-3-benzazépin-7-yl)-imidazolidin-2-one et ses sels.

**7.** Sels physiologiquement acceptables des composés selon au moins l'une des revendications 1 à 6 avec des acides oou des bases inorganiques ou organiques.

**8.** Médicament contenant un composé selon au moins l'une des revendications 1 à 6 ou un sel physiologiquement acceptable selon la revendication 7 et éventuellement un ou plusieurs supports et/ou diluants inertes.

**9.** Utilisation d'un composé selon au moins l'une des revendications 1 à 7 pour la préparation d'un médicament qui convient pour la lutte ou la prévention contre les maladies dans lesquelles il apparaît des agrégats cellulaires de taille relativement petite ou relativement grande ou les interactions cellule-matrice jouent un rôle.

**10.** Procédé de préparation d'un médicament selon la revendication 8 caractérisé en ce que, par voie non chimique, un composé selon au moins l'une des revendications 1 à 7 est incorporé dans un ou plusieurs supports et/ou diluants inertes.

**11.** Procédé de préparation des dérivés cycliques selon les revendications 1 à 7 caractérisé en ce que

a) pour la préparation de composés de formule générale I où F représente un groupe carboxyle, un composé de formule générale

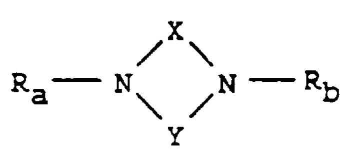

(I)

où

$R_a$, $R_b$, X et Y sont définis comme dans les revendications 1 à 6 avec la condition que l'un des restes $R_a$ à $R_d$ représente un groupe F'-E-D-, où
E et D sont définis comme dans les revendications 1 à 6 et F' représente un groupe convertible en un groupe carboxyle par hydrolyse, traitement avec des acides, thermolyse ou hydrogénolyse,

est converti par hydrolyse, traitement avec des acides, thermolyse ou hydrogénolyse en un composé de formule générale I où F représente un groupe carboxyle ou

b) pour la préparation de composés de formule générale I où $R_{16}$ et $R_{17}$ représentent ensemble une liaison supplémentaire, $G_4$ représente une liaison et $R_{18}$ représente un groupe amino, un composé de formule générale

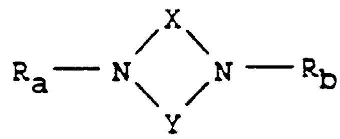

(III)

où

$R_a$, $R_b$, X et Y sont définis comme dans les revendications 1 à 6 avec la condition que l'un des restes $R_a$ à $R_d$ représente un groupe A-B- où
B est défini comme indiqué dans les revendications 1 à 6 et A représente un groupe de formule

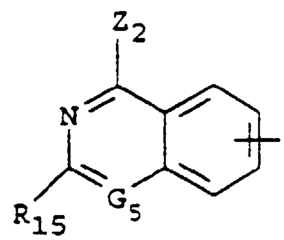

où
la partie benzo, $R_{15}$ et $G_5$ sont définis comme dans les revendications 1 à 6 et
$Z_2$ représente un groupe partant nucléophile, est mis a réagir avec un composé de formule générale

$$H - R_{27} \quad \text{(IV)}$$

où
$R_{27}$ représente un groupe amino, ou

c) pour la préparation de composés de formule générale I où

$R_{16}$ et $R_{17}$ représentent ensemble une liaison supplémentaire,
$G_4$ représente une liaison et $R_{18}$ représente un atome de chlore, un composé de formule générale

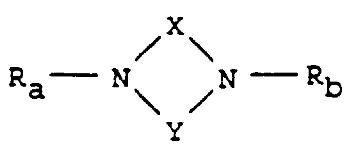

(V)

où
$R_a$, $R_b$, X et Y sont définis comme dans les revendications 1 à 6 avec la condition que l'un des restes $R_a$ à $R_d$ représente un groupe A-B- où
B est défini comme indiqué dans les revendications 1 à 6 et A représente un groupe de formule

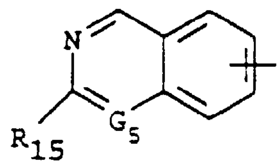

où

la partie benzo, $R_{15}$ et $G_5$ sont définis comme dans les revendications 1 à 6, est mis à réagir en présence d'un halogénure d'acide ou

d) pour la préparation de composés de formule générale I où X représente un groupe carbimino substitué par un groupe cyano, un groupe carbonyle ou sulfonyle et Y représente un groupe alkylène linéaire de 2 ou 3 atomes de carbone éventuellement substitué par $R_c$ ou $R_a$ ou $R_c$ et $R_d$, un composé de formule générale

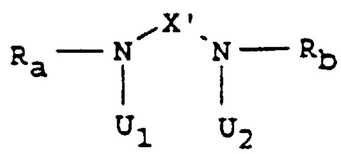

(VI)

où

$R_a$ et $R_b$ sont définis comme dans les revendications 1 à 6,
X' représente un groupe carbimino substitué par un groupe cyano, un groupe carbonyle ou sulfonyle,
l'un des restes $U_1$ et $U_2$ représente un atome d'hydrogène et l'autre des restes $U_1$ et $U_2$ représente un groupe alkylène linéaire de 2 ou 3 atomes de carbone éventuellement substitué par $R_c$ ou $R_d$ ou $R_c$ et $R_d$, auquel est lié en outre en position terminale un groupe partant nucléophile, est cyclisé ou

e) pour la préparation de composés de formule générale I où X représente un groupe carbonyle et Y représente l'un des groupes alkylène linéaires de 2 ou 3 atomes de carbone indiqués dans les revendications 1 à 6, un composé de formule générale

$$R_{28} - NH - T \qquad (VII)$$

est mis à réagir avec un isocyanate de formule générale

$$O = C = N - R_{29} \qquad (VIII)$$

où

l'un des restes $R_{28}$ et $R_{29}$ possède les significations indiquées pour $R_a$ dans les revendications 1 à 6 et l'autre des restes $R_{28}$ et $R_{29}$ possède les significations indiquées pour $R_b$ dans les revendications 1 à 6 et T représente un groupe éventuellement substitué dans la partie alkylidène par $R_c$ ou $R_d$ ou par $R_c$ et $R_d$ de formule

$$-(CH_2)_m - HC(OR_{30})_2$$

où

m représente le nombre 1 ou 2 et
$R_{30}$ représente dans chaque cas un groupe alkyle de 1 à 4 atomes de carbone,
et, éventueilement, un composé ainsi obtenu est ensuite hydrogéné ou

f) pour la préparation de composés de formule générale I où $G_1$ et $G_2$ représentent chacun une liaison, $G_3$ représente un groupe méthylène éventuellement substitué par un groupe alkyle de 1 à 4 atomes de carbone, $R_2$, $R_4$ et $R_5$ représentent chacun un atome d'hydrogène, $R_3$ et $R_6$, qui peuvent être identiques ou différents, représentent chacun un atome d'hydrogène ou un groupe alkyle de 1 à 4 atomes de carbone, un composé de formuie générale

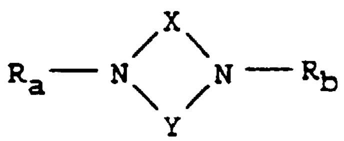

(IX)

où

$R_a$, $R_b$, X et Y sont définis comme dans les revendications 1 à 6 avec la condition que l'un des restes $R_a$ à $R_d$ représente un groupe A-B- où B est défini comme indiqué dans les revendications 1 à 6 et A représente un groupe de formule

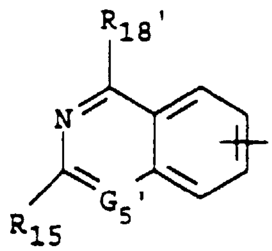

où

la partie benzo et $R_{15}$ sont définis comme dans les revendications 1 à 6,
$G_5$' représente un groupe méthyne éventuellement substitué par un groupe alkyle de 1 à 4 atomes de carbone et
$R_{18}$' représente un atome d'hydrogène ou un groupe alkyle de 1 à 4 atomes de carbone, est hydrogéné ou

g) pour la préparation de composés de formule générale I où F représente un groupe carbonyle substitué par un groupe alcoxy de 1 à 4 atomes de carbone, par un groupe arylalcoxy de 1 à 4 atomes de carbone dans la partie alcoxy, la partie aryle étant définie comme indiqué dans les revendications 1 à 6, ou par un groupe $R_{22}O$-, un composé de formule générale

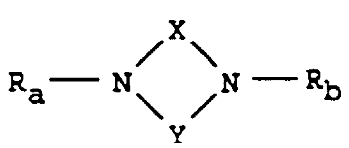

(X)

où

$R_a$, $R_b$, X et Y sont définis comme dans les revendications 1 à 6 avec la condition que l'un des restes $R_a$

à $R_d$ représente un groupe F"-E-D- où
E et D sont définis comme dans les revendications 1 à 6 et F" représente un groupe carboxy- ou alcovycarbonyle, est mis à réagir avec un alcool de formule générale

$$HO - R_{31} \quad (XI)$$

où
$R_{31}$ possède les significations indiquées pour $R_{22}$ dans les revendications 1 à 6 et aussi représente un groupe alkyle de 1 à 4 atomes de carbone ou un groupe arylalkyle où la partie aryle est définie comme indiqué dans les revendications 1 à 6 et la partie alkyle peut contenir 1 à 4 atomes de carbone, ou

h) pour la préparation de composés de formule générale I où $R_4$ représente l'un des restes alkyle éventuellement substitués, des restes alcényle, cycloalkyle, cycloalkylalkyle ou aralkyle indiqués lors de la définition de $R_4$ dans les revendications 1 à 6, un composé de formule générale

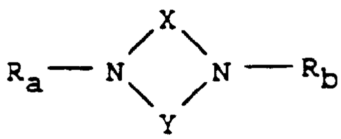

(XI)

où

$R_a$, $R_b$, X et Y sont définis comme dans les revendications 1 à 6 avec la condition que l'un des restes $R_a$ à $R_d$ représente un groupe A-B- où
A et B sont définis comme dans les revendications 1 à 6 avec la condition que $R_4$ représente un atome d'hydrogène, est mis à réagir avec un composé de formule générale

$$Z_3 - R_{32} \quad (XIII)$$

où
$R_{32}$ représente un groupe alkyle de 1 à 6 atomes de carbone, un groupe cycloalkyle ou cycloalkylalkyle où la partie cycloalkyle peut contenir dans chaque cas 3 à 7 atomes de carbone et la partie alkyle peut contenir 1 à 4 atomes de carbone, un groupe alcényle de 3 à 6 atomes de carbone, un groupe arylalkyle, hydroxyalkyle, alcoxyalkyle, carboxyalkyle, alcoxycarbonylalkyle, aminocarbonylalkyle, N-alkylaminocarbonylalkyle ou N,N-dialkylaminocarbonyle, où la partie aryle et les parties alkyle sont définies comme dans les revendications 1 à 6, et
$Z_3$ représente un groupe partant nucléophile ou
$Z_3$ représente avec un atome d'hydrogène voisin du reste $R_{32}$ un atome d'oxygène, éventuellement en présence d'un réducteur ou

i) pour la préparation de composés de formule générale I où X représente un groupe carbimino substitué sur l'atome d'azote par un groupe cyano, un groupe carbonyle ou sulfonyle, un composé de formule générale

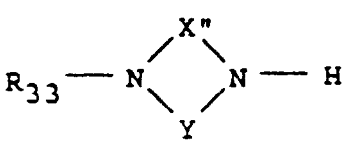

(XIV)

est mis à réagir avec un composé de formule générale

$$Z_4 - R_{34} \quad \text{(XV)}$$

où

Y est défini comme dans les revendications 1 à 6,
X" représente un groupe carbimino substitué sur l'atome d'azote par un groupe cyano, un groupe carbonyle ou sulfonyle,
l'un des restes $R_{33}$ et $R_{34}$ possède les significations indiquées pour $R_a$ dans les revendications 1 à 6 et l'autre des restes $R_{33}$ et $R_{34}$ possède les significations indiquées pour $R_b$ dans les revendications 1 à 6 et $Z_4$ représente un groupe partant nucléophile ou

j) pour la préparation de composés de formule générale I où $R_4$ représente un groupe alcoxycarbonyle, arylméthyloxycarbonyle, formyle, acétyle, trifluoroacétyle ou $R_{11}CO-O-(R_{12}CR_{13})-O-CO-$ où $R_{11}$ à $R_{13}$ et la partie aryle sont définis comme dans les revendications 1 à 6 et la partie alcoxy peut contenir 1 à 4 atomes de carbone, un composé de formule générale

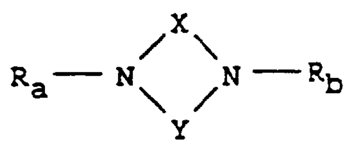

(XVI)

où

$R_a$, $R_b$, X et Y sont définis comme dans les revendications 1 à 6 avec la condition que $R_4$ représente un atome d'hydrogène, est mis à réagir avec un composé de formule générale

$$Z_5 - R_{35} \quad \text{(XVII)}$$

où
$R_{36}$ représente un groupe alcoxycarbonyle ayant au total 2 à 5 atomes de carbone, un groupe arylméthyloxycarbonyle où la partie aryle est définie comme dans les revendications 1 à 6, un groupe $R_{11}CO-O-(R_{12}CR_{13})-O-CO-$, formyle, acétyle ou trifluoroacétyle, où $R_{11}$ à $R_{13}$ sont définis comme dans les revendications 1 à 6, et
$Z_5$ représente un groupe partant nucléophile, ou

k) pour la préparation de composés de formule générale I où F représente un groupe carbonyle qui est substitué par un groupe alcoxy de 1 à 4 atomes de carbone, par un groupe arylalcoxy où la partie aryle est définie comme indiqué dans les revendications 1 à 6 et la partie alcoxy peut contenir 1 à 4 atomes de carbone, par un groupe $R_{22}O-$ ou $R_{23}CO-O-CHR_{24}-O-$ où $R_{22}$ à $R_{24}$ sont définis comme indiqué dans les revendications 1 à 6, un composé de formule générale

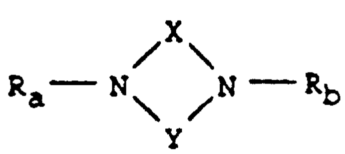

(XVIII)

où

$R_a$, $R_b$, X et Y sont définis comme dans les revendications 1 à 6 avec la condition que l'un des restes $R_a$ à $R_d$ représente un groupe F'''-E-D- où

E et D sont définis comme dans les revendications 1 à 6 et F''' représente un groupe carboxyle, est mis à réagir avec un composé de formule générale

$$Z_6 - R_{36} \qquad (XIX)$$

ou

$R_{36}$ représente un groupe alkyle de 1 à 4 atomes de carbone, un groupe arylalkyle où la partie aryle est définie comme indiqué dans les revendications 1 à 6 et la partie alkyle peut contenir 1 à 4 atomes de carbone, un groupe $R_{22}$- ou $R_{23}$CO-O-CH$R_{24}$- où $R_{22}$ à $R_{24}$ sont définis comme indiqué dans les revendications 1 à 6 et

$Z_6$ représente un groupe partant nucléophile, ou

l) pour la préparation de composés de formule générale I où X représente un groupe carbonyle et Y représente un groupe éthylène éventuellement substitué par $R_c$ ou $R_d$ ou $R_c$ et $R_d$ où un groupe méthylène est remplacé par un groupe carbonyle, un composé éventuellement formé dans le mélange réactionnel de formule générale

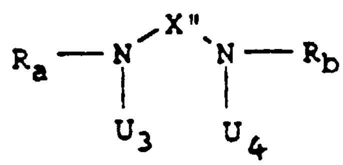

(XX)

où

$R_a$ et $R_b$ sont définis comme dans les revendications 1 à 6, X'' représente un groupe carbonyle, l'un des restes $U_3$ et $U_4$ représente un atome d'hydrogène et l'autre des restes $U_3$ et $U_4$ représente un groupe éthylène éventuellement substitué par $R_c$ ou $R_d$ ou $R_c$ et $R_d$ où un groupe méthylène est remplacé par un groupe $Z_7$-CO- où $Z_7$ représente un groupe partant nucléophile, est cyclisé et, si on le souhaite, un composé de formule générale I ainsi obtenu, qui contient une liaison carbone-carbone insaturée, est converti par hydrogénation catalytique en un composé saturé correspondant de formule générale I et/ou si nécessaire, un reste protecteur utilisé pendant les réactions pour la protection de groupes réactifs est clivé et/ou

un composé de formule générale I ainsi obtenu est résolu en ses stéréoisomères et/ou

un composé de formule générale I ainsi obtenu est converti en ses sels, en particulier pour l'utilisation pharmaceutique en ses sels physiologiquement acceptables avec un acides ou une base inorganique ou organique.